# EUROPEAN PATENT APPLICATION

(11) **EP 4 059 949 A1**
(43) Date of publication of application: **21.09.2022**
(21) Application number: 21305336.6
(22) Date of filing: 18.03.2021
(51) Int. Cl.: C07K 14/195, C12N 15/10, C07K 14/47

(54) **ANTI-FACTOR C3 SAC7D VARIANTS AND THEIR MEDICAL USE FOR TREATING COMPLEMENT-MEDIATED DISORDERS**

(71) Applicant: AFFILOGIC, 44200 Nantes (FR)
(72) Inventor: The designation of the inventor has not yet been filed
(74) Representative: Flesselles, Bruno F.G.

(57) **Abstract**

The invention relates to a polypeptide comprising a variant of a protein of the Sac7d family that specifically binds to the complement component 3 (C3) and/or the component C3b obtained after hydrolysis of C3, and inhibits the complement cascade.

## Description

### Introduction

Disruption of the complement balance associated with an increased production and/or activation of complement molecules may lead to dysregulation of the immune system and appearance or worsening of autoimmune, inflammatory, degenerative, hematological, or ischemic disorders.

### SUMMARY OF THE INVENTION

In a first aspect, the invention relates to a polypeptide comprising a variant of a member of the Sac7d family binding to C3 and/or C3b, wherein the variant comprises from 4 to 20 mutated residues in the interface of binding of the member of the Sac7d family to its natural ligand, and wherein said variant comprises W24Y and R42W mutations with the numbering corresponding to Sac7d (SEQ ID NO: 1) residues. In particular, the variant comprises from 5 to 14 or from 5 to 13 mutated residues in the interface of binding of the member of the Sac7d family to its natural ligand.

In particular, the polypeptide further comprises K9T, S31L and A44Y mutations, with the numbering corresponding to Sac7d (SEQ ID NO: 1) residues.

In some embodiments, the polypeptide further comprises at least one mutation selected from D16E, N37Q and M56L, with the numbering corresponding to Sac7d (SEQ ID NO: 1) residues.

In particular, the mutated residues in the interface of binding of the member of the Sac7d family to its natural ligand are selected from the group consisting of V2, K3, K5, K7, Y8, K9, G10, E14, T17, K21, K22, W24, V26, G27, K28, M29, S31, T33, D36, N37, G38, K39, T40, A44, S46, E47, K48, D49, A50 and P51 of Sac7d.

In particular the member of the Sac7d family is selected from the group consisting of Sac7d from *Sulfolobus acidocaldarius,* Sac7e from *Sulfolobus acidocaldarius,* SSo7d from *Sulfolobus solfataricus,* Ssh7b from *Sulfolobus shibatae,* Ssh7a from *Sulfolobus shibatae,* DBP7 from *Sulfolobus tokodaii,* Sis7a from *Sulfolobus islandicus,* Mse7 from *Metallosphaera sedula,* Mcu7 from *Metallosphaera cuprina,* Aho7a from *Acidianus hospitalis,* Aho7b from *Acidianus hospitalis,* Aho7c from *Acidianus hospitalis* and Sto7 from *Sulfurisphaera tokodaii.*

In some embodiments, the polypeptide comprises SEQ ID NO: 59, SEQ ID NO: 45 SEQ ID NO: 22, SEQ ID NO: 27, SEQ ID NO: 17, SEQ ID NO: 64, SEQ ID NO: 69, SEQ ID NO: 74, SEQ ID NO: 79, SEQ ID NO: 84, SEQ ID NO: 89, SEQ ID NO: 94 or SEQ ID NO: 99.

In some embodiments, the polypeptide comprises SEQ ID NO: 87, SEQ ID NO: 88, SEQ ID NO: 82, SEQ ID NO: 83, SEQ ID NO: 77, SEQ ID NO: 78, SEQ ID NO: 102, SEQ ID NO: 103, SEQ ID NO: 97, SEQ ID NO: 98, SEQ ID NO: 92, SEQ ID NO: 93 or amino acids 1-54 of these sequences.

In some embodiments, the polypeptide consists in the variant of a member of the Sac7d family binding to C3 and/or C3b.

In some embodiments, the variant of a member of the Sac7d family binding to C3 and/or C3b is conjugated to an organic molecule.

In some embodiments, the variant of a member of the Sac7d family binding to C3 and/or C3b is conjugated to another polypeptide, in particular another variant of a protein of the Sac7d family.

The invention also relates to a nucleic acid molecule coding for the polypeptide as described, an expression vector comprising such nucleic acid molecule (and including elements allowing transcription in a host cell), and to a host cell comprising the nucleic acid molecule or the expression vector.

The invention also relates to a pharmaceutical composition comprising the polypeptide as disclosed, the nucleic acid as disclosed, the expression vector as disclosed, or the host cell as disclosed, and a pharmaceutically acceptable carrier.

The invention also relates to a method for producing the polypeptide as disclosed, comprising
a. Culturing a cell culture wherein the cells have been transformed by the expression vector as disclosed,
   And
b. Recovering the polypeptide.

The invention also relates to the polypeptide or the nucleic acid as disclosed as a medicament.

The invention also relates to the polypeptide, the nucleic acid, the expression vector, or the cell as disclosed for use for treatment of a complement-mediated disorder.

In particular, the complement-mediated disorder is characterized by complement-mediated damage to red blood cells, in particular paroxysmal nocturnal hemoglobinuria or atypical hemolytic uremic syndrome.

In some embodiments, the complement-mediated disorder is an autoimmune disease, optionally wherein the disorder is multiple sclerosis.

In some embodiments, the complement-mediated disorder involves the kidney, optionally wherein the disorder is membranoproliferative glomerulonephritis, lupus nephritis, IgA nephropathy (IgAN), primary membranous nephropathy (primary MN), C3 glomerulopathy (C3G), or acute kidney injury.

In some embodiments, the complement-mediated disorder involves the central or peripheral nervous system or neuromuscular junction, optionally wherein the disorder is neuromyelitis optica, Guillain-Barré syndrome, multifocal motor neuropathy, or myasthenia gravis.

In some embodiments, the complement-mediated disorder involves the respiratory system, optionally wherein the disorder is characterized by pulmonary fibrosis.

In some embodiments, the complement-mediated disorder involves the vascular system, optionally wherein the disorder is characterized by vasculitis.

The invention also relates to a method of treating a subject having or at risk of a complement-mediated disorder, the method comprising administering to the subject a composition comprising an effective amount of the polypeptide, the nucleic acid, the expression vector, the host cell, or the pharmaceutical composition as disclosed.

### DETAILED DESCRIPTION OF THE INVENTION

In particular, the invention relates to a polypeptide comprising a variant of the Sac7d protein or of a protein of the Sac7d family that specifically binds to the complement component 3 (C3) and/or the component C3b obtained after hydrolysis of C3. In particular, the variant binds to both C3 and C3b. It also preferably inhibits the complement cascade, and, in particular can compete with compstatin. Such variant is useful for treatment of a complement-mediated disorder (e.g., in a subject having or at risk of a complement-mediated disorder) and/or for modulating complement. In particular, in certain embodiments the variant binds to the C3b fragment of the C3 protein, either after C3 hydrolysis (when the C3b fragment has been released) or before hydrolysis (when the C3b fragment is still within the C3 protein).

In preferred embodiments, such variant comprises SEQ ID NO: 17, SEQ ID NO: 18, SEQ ID NO: 19, SEQ ID NO: 20, SEQ ID NO: 21, SEQ ID NO: 31, SEQ ID NO: 32, SEQ ID NO: 33, SEQ ID NO: 34, SEQ ID NO: 35, SEQ ID NO: 45, SEQ ID NO: 46, SEQ ID NO: 47, SEQ ID NO: 48 or SEQ ID NO: 49. These sequences are based on the sequence consensus for the Sac7d family and have been obtained following the teachings of WO 2012/150314 which shows that it is possible to perform portability of the mutations from one protein of the Sac7d family to another one, these proteins presenting a similar structure and binding site as well as similar amino acid sequences.

In particular, in certain embodiments said variant is a variant of the Sac7d protein, and comprises SEQ ID NO: 22, SEQ ID NO: 23 SEQ, ID NO: 24, SEQ ID NO: 25, SEQ ID NO: 26, SEQ ID NO: 36, SEQ ID NO: 37, SEQ ID NO: 38, SEQ ID NO: 39, SEQ ID NO: 40, SEQ ID NO: 50, SEQ ID NO: 51, SEQ ID NO: 52, SEQ ID NO: 53, or SEQ ID NO: 54. In certain embodiments, said variant is a variant of the Sso7d protein, and comprises SEQ ID NO: 27, SEQ ID NO: 28, SEQ ID NO: 29, SEQ ID NO: 30, SEQ ID NO: 41, SEQ ID NO: 42, SEQ ID NO: 43, SEQ ID NO: 44, SEQ ID NO: 55, SEQ ID NO: 56, SEQ ID NO: 57 or SEQ ID NO: 58.

It is to be noted that the sequences indicated above comprise a methionine at its N-terminal, but that it is also possible to perform the invention when such methionine listed at the N-terminal end of each sequence is not included. Similarly, the amino acids located at the end-terminus of the proteins may be omitted. In particular the amino acids located after residue L58 of Sac7d (residues located after L60 of SEQ ID NO: 16 or L59 of Sso7d) may be omitted. Consequently, the variant may comprise amino acids 2-58 of SEQ ID NO: 22, SEQ ID NO: 23 SEQ, ID NO: 24, SEQ ID NO: 25, SEQ ID NO: 26, SEQ ID NO: 36, SEQ ID NO: 37, SEQ ID NO: 38, SEQ ID NO: 39, SEQ ID NO: 40, SEQ ID NO: 50, SEQ ID NO: 51, SEQ ID NO: 52, SEQ ID NO: 53, or SEQ ID NO: 54, or amino acids 2-59, 2-60, 2-61, 2-62 or 2-63 of SEQ ID NO: 22, SEQ ID NO: 23 SEQ, ID NO: 24, SEQ ID NO: 25, SEQ ID NO: 26, SEQ ID NO: 36, SEQ ID NO: 37, SEQ ID NO: 38, SEQ ID NO: 39, SEQ ID NO: 40, SEQ ID NO: 50, SEQ ID NO: 51, SEQ ID NO: 52, SEQ ID NO: 53, or SEQ ID NO: 54. When based on the Sso7d scaffold, the variant may comprise amino acids 2-59 of SEQ ID NO: 27, SEQ ID NO: 28 SEQ ID NO: 29, SEQ ID NO: 30, SEQ ID NO: 41, SEQ ID NO: 42, SEQ ID NO: 43, SEQ ID NO: 44, SEQ ID NO: 55, SEQ ID NO: 56, SEQ ID NO: 57 or SEQ ID NO: 58, or amino acids 2-60 of SEQ ID NO: 27, SEQ ID NO: 28 SEQ ID NO: 29, SEQ ID NO: 30, SEQ ID NO: 41, SEQ ID NO: 42, SEQ ID NO: 43, SEQ ID NO: 44, SEQ ID NO: 55, SEQ ID NO: 56, SEQ ID NO: 57 or SEQ ID NO: 58.

When based on Sac7d, the polypeptide may comprise any of SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, or SEQ ID NO: 88, or amino acids 1-57, 1-58, 1-59, 1-60, 1-61, 1-62, 1-63 of any of these sequences.

When based on Aho7c, the polypeptide may comprise any of SEQ ID NO: 89, SEQ ID NO: 90, SEQ ID NO: 91, SEQ ID NO: 92, SEQ ID NO: 93, SEQ ID NO: 94, SEQ ID NO: 95, SEQ ID NO: 96 SEQ ID NO: 97, SEQ ID NO: 98, SEQ ID NO: 99, SEQ ID NO: 100, SEQ ID NO: 101, SEQ ID NO: 102, or SEQ ID NO: 103, or amino acids 1-56 or 1-57 of any of these sequences.

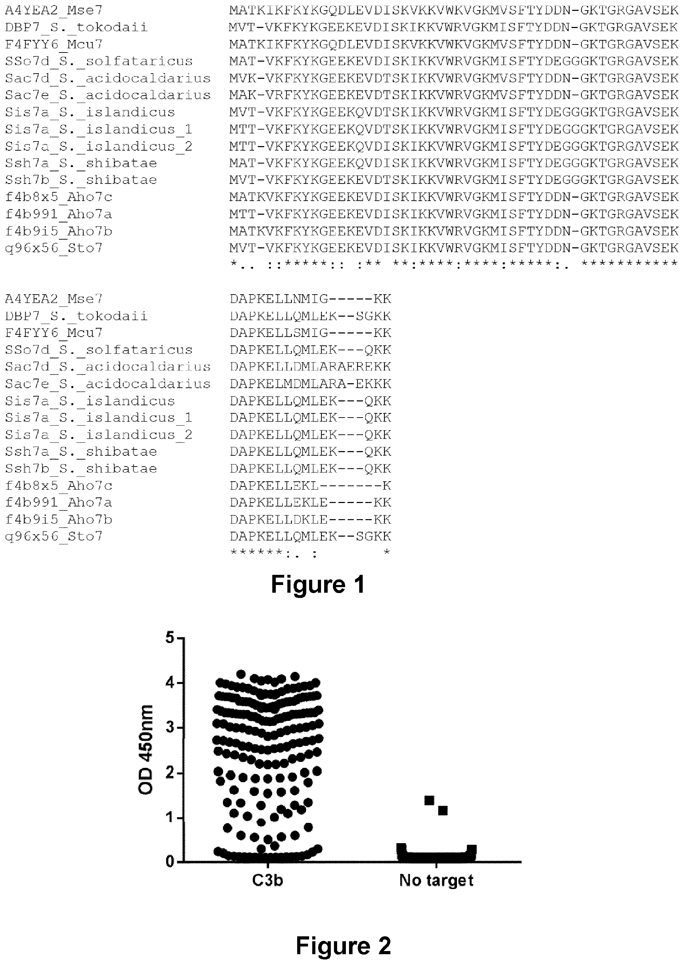

All these sequences describe specific variants based on proteins of the Sac7d family. As explained below, it is possible, using the alignment of Figure 1, to design variants of other proteins of the family.

A sequence that is consensus for proteins Sac7d and Aho7c is SEQ ID NO: 104. Variants binding to C3 and/or C3b can also comprise SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66 SEQ ID NO: 67, SEQ ID NO: 68, SEQ ID NO: 69, SEQ ID NO: 70, SEQ ID NO: 71, SEQ ID NO: 72, or SEQ ID NO: 73, which are based on this consensus sequence.

In case of discrepancy between the specification and the sequence listing, the sequences mentioned in the specification are preponderant.

As indicated above, the specification specifically discloses variants of Sac7d (SEQ ID NO: 1) Aho7c (SEQ ID NO: 14), Sso7d (SEQ ID NO: 2), but the teachings are applicable to the other proteins of the Sac7d family, in particular Sto7 (SEQ ID NO: 15) which is very similar to Sac7d and Aho7c. The teachings are also applicable to other OB-fold domains as disclosed in WO2007139397. The invention is also applicable to SH3 domains, a small protein domain of about 60 amino acid residues, initially, described as a conserved sequence in the viral adaptor protein v-Crk and described under PF00018 in the PFAM database. The SH3 domain has a characteristic beta-barrel fold that consists of five or six β-strands arranged as two tightly packed anti-parallel β sheets. The linker regions may contain short helices. It is to be noted that OB-fold and SH3 domains share homology and that, in view of the knowledge of the sequence and structure of these domains, it is possible to determine, in any of OB-fold or SH3 domain, to which amino acids correspond the amino acids as disclosed below for Sac7d.

In a specific embodiment, the polypeptide consists in the variant of a protein of the Sac7d family binding to C3 and/or C3b.

In a specific embodiment, the variant of a protein of the Sac7d family binding to C3 and/or C3b is linked or fused to another protein or polypeptide. In particular, the other protein or polypeptide may be the same or another variant of a protein of the Sac7d family, binding to C3 and/or C3b or to another target. In this embodiment, it is preferred when the other variant of the Sac7d family binds to albumin.

In specific embodiments, the variant is present in a polypeptide and is thus covalently linked by amine bonds to other proteins or polypeptides presenting a biological interest.

In an embodiment, the polypeptide is conjugated to an organic molecule that presents some functionality, in particular a kinase inhibitor that is used as a VEGF inhibitor.

The invention also pertains to a genetic construct comprising a DNA sequence coding for the polypeptide described herein, to a vector comprising such genetic construct, and to a host cell comprising the genetic construct in its genome.

The invention also pertains to a method for producing the polypeptide herein disclosed, comprising the steps consisting of
a. Culturing a cell culture wherein the cells have been transformed by a genetic construct as disclosed,
   and
b. Recovering the polypeptide.

The invention also relates to a polypeptide herein disclosed as a medicament.

The invention also relates to a polypeptide herein disclosed for use thereof for the treatment of a complement-associated or -mediated disease, alone or in combination with another adapted treatment.

The invention also relates to a method for treating a subject in need thereof comprising administering a therapeutic amount of a polypeptide herein disclosed to the subject, in particular when the subject has a complement-mediated disease.

The invention also relates to a composition containing a polypeptide herein disclosed and another agent for simultaneous, separate or sequential (spread out over time) use in the treatment of a complement associated or mediated disease. Such other agent is selected among agents already known for treating the complement associated or mediated disease.

It is particularly adapted when the disease is as disclosed below.

The invention also relates to these variants in therapeutic, diagnostic or purification uses. The invention also relates to compositions, in particular oral or topical (dermal), containing the polypeptide or the variants.

It is reminded that the sequence of Sac7d is:

MVKVKFKYKGEEKEVDTSKIKKVWRVGKMVSFTYDDNGKTGRGAVSEKDAPKEL
LDMLARAEREKK (SEQ ID NO: 1).

The sequence of Sso7d is

MATVKFKYKGEEKEVDISKIKKVWRVGKMISFTYDEGGGKTGRGAVSEKDAP
KELLQMLEKQKK (SEQ ID NO: 2).

The variants binding to C3 and/or C3b herein disclosed contain mutations at positions corresponding to the positions 7, 8, 9, 21, 22, 24, 26, 29, 31, 33, 40, 42, 44 and/or 46 of the Sac7d sequence. It is however to be noted that the threonine at position 33 may be maintained in the variants, as well as the threonine at position 40, the alanine at position 44, the valine at position 26 or the serine at position 46. However, the variants binding to C3 and/or C3b herein disclosed contain at least 4, more preferably at least 5, more preferably at least 6, more preferably at least 7, more preferably at least 8, more preferably at least 9, more preferably at least 10, more preferably at least 11, more preferably at least 12 mutated amino acids as compared to SEQ ID NO: 1 (i.e. amino acids that are different than the corresponding ones of SEQ ID NO: 1, or to the sequence of the protein of the Sac7d family from which they are issued). The generally present at most 20, more preferably at most 18, more preferably at most 16, 15, 14 or 13 mutated amino acids as compared to SEQ ID NO: 1 (or to the sequence of the protein of the Sac7d family from which they are issued).

In a preferred embodiment, the polypeptide comprises the sequence SEQ ID NO: 45 or amino acids 2-60 of SEQ ID NO: 45.

MXXXVXFXXXGEEKXVXXSKIXXVYRXGKXXXFXYDXXXGKXGWGXVXEKDAPK
ELXXXLXXXXXXXX (SEQ ID NO: 45).

In the context of the present application, and unless indicated otherwise, X can be any naturally occurring amino acid, in particular any of the standard amino acids (i.e., the 20 amino acids encoded in the standard genetic code).

In preferred embodiments, the polypeptide comprises the sequence MXXXVXFXXXGEEKXVDXSKIXXVYRXGKXXXFXYDXXXGKXGWGXVXEKDAPK ELXXXLXXXXXXXX (SEQ ID NO: 17), or amino acids 2-60 of SEQ ID NO: 17. The amino acid D17 may be modified in particular as D17E (SEQ ID NO: 31). Amino acids 2-60 of SEQ ID NO: 31 may also be used.
These sequences are based on SEQ ID NO: 16 which is the sequence that is consensus to proteins of the Sac7d family. SEQ ID NO: 17 and SEQ ID NO: 31 differ from SEQ ID NO: 16 by the presence of the mutation W25Y (which corresponds to the mutation W24Y in Sac7d) and R44W (which corresponds to the mutation R42W in Sac7d). Indeed, the inventors have shown that absence of these two amino acids alters the binding capability of the variant to C3 and/or C3b. SEQ ID NO: 31 further differs from SEQ ID NO: 16 by the D17E mutation.
It is further indicated that the sequences provided herein contain a Methionine as the first amino acid. However, this methionine can be omitted in the polypeptide herein disclosed. Furthermore, the C-terminus of the proteins herein disclosed are not necessary for obtaining the biological effect of the polypeptides and can be omitted. This is further indicated below.

In these sequences SEQ ID NO: 45, SEQ ID NO: 17 and SEQ ID NO: 31, the amino acids designated as X at positions 8-10, 22-23, 27, 30, 32, 34, 42, 46 and 48 are as indicated in Table 2. The other amino acids designated as X are indicated in Table 1 or Table 10 (where "-" designates no amino acid).

**Table 1. description of amino acids in SEQ ID NO: 17-SEQ ID NO: 21 and SEQ ID NO: 31-SEQ ID NO: 35 and SEQ ID NO: 45-SEQ ID NO: 49.**

| **Position** | **Amino acid** |
|---|---|
| 2 | X is V, A or T |
| 3 | X is T or K |
| 4 | X is - or K |
| 6 | X is R or K |
| 15 | X is E or Q |
| 18 | X is T or I |
| 31 | X is V or I |
| 37 to 39 | XXX is EGG or DN- |
| 57 | X is M or L |
| 58 | X is Q, D or E |
| 59 | X is M or K |
| 61 to 67 | -------K, EKS--GKK, EK---QKK, ARAEREKK, ARAERE-K, ARAERE--, ARA-EKKK, E-----KK, EKS--GKK, ACAEREKK or ACA-EKKK |

**Table 2. description of amino acids in SEQ ID NO: 17 and SEQ ID NO: 31 and SEQ ID NO: 45.**

| **Position** | **Amino acid** | **Preferred amino acids** |
|---|---|---|
| 8 | X can be any amino acid | D, E A, V, or P |
| 9 | X can be any amino acid | A, L, I, V, T or H |
| 10 | X can be any amino acid | T, I |
| 22 | X is A, S, T, V, I, L, Y, or F | F, Y, A, L, or S |
| 23 | X can be any naturally occurring amino acid | H, N, A, K, Q |
| 27 | X is S, T, V, I, L, Y, or F | I, L, Y, V or T |
| 30 | X can be any amino acid | D, E, S, or A |
| 32 | X is L, I, V, Y, F, M, or H | L, Y, I or M |
| 34 | X is S, T or A | S, T or A |
| 42 | X is T or I | T or I |
| 46 | X is F, Y, I, V, L, M or H | Y, F |
| 48 | X is S, T, H or R | S |

In another embodiment, the polypeptide comprises the sequence SEQ ID NO: 46 or amino acids 2-60 of SEQ ID NO: 46.

MXXXVXFXXTGEEKXVXXSKIXXVYRXGKXXLFXYDXXXGKXGWGYVXEKDAPK
ELXXXLXXXXXXXX (SEQ ID NO: 46)

In another embodiment, the polypeptide comprises the sequence MXXXVXFXXTGEEKXVDXSKIXXVYRXGKXXLFXYDXXXGKXGWGYVXEKDAPK ELXXXLXXXXXXXX (SEQ ID NO: 18) or amino acids 2-60 of SEQ ID NO: 18 or:
MXXXVXFXXTGEEKXVEXSKIXXVYRXGKXXLFXYDXXXGKXGWGYVXEKDAPK ELXXXLXXXXXXXX (SEQ ID NO: 32), or amino acids 2-60 of SEQ ID NO: 32.
The nature of the amino acids X is indicated in Tables 1, 3 and 10.

**Table 3. description of amino acids in SEQ ID NO: 18 and SEQ ID NO: 32 and SEQ ID NO: 46**

| **Position** | **Amino acid** | **Preferred amino acids** |
|---|---|---|
| 8 | X can be any amino acid | D, E A, V, or P |
| 9 | X can be any amino acid | A, L, I, V, T or H |
| 22 | X is A, S, T, V, I, L, Y, or F | F, Y, A, L, or S |
| 23 | X can be any naturally occurring amino acid | H, N, A, K, Q |
| 27 | X is S, T, V, I, L, Y, or F | I, L, Y, V or T |
| 30 | X can be any amino acid | D, E, S, or A |
| 34 | X is S, T or A | S, T or A |
| 42 | X is T or I | T or I |
| 48 | X is S, T, H or R | S |

In a preferred embodiment, the polypeptide comprises the sequence SEQ ID NO: 47 or amino acids 2-60 of SEQ ID NO: 47.

MXXXVXFXATGEEKXVXXSKIXXVYRXGKDXLFSYDXXXGKIGWGYVSEKDAPKE
LXXXLXXXXXXXX (SEQ ID NO: 47)

In another embodiment, the polypeptide comprises the sequence MXXXVXFXATGEEKXVDXSKIXXVYRXGKDXLFSYDXXXGKIGWGYVSEKDAPK ELXXXLXXXXXXXX (SEQ ID NO: 19) or amino acids 2-60 of SEQ ID NO: 19 or:
MXXXVXFXXTGEEKXVEXSKIXXVYRXGKXXLFXYDXXXGKXGWGYVXEKDAPK ELXXXLXXXXXXXX (SEQ ID NO: 33), or amino acids 2-60 of SEQ ID NO: 33.
The nature of the amino acids X is indicated in Tables 1, 4 and 10.

**Table 4. description of amino acids in SEQ ID NO: 19 and SEQ ID NO: 33 and SEQ ID NO: 47**

| **Position** | **Amino acid** | **Preferred amino acids** |
|---|---|---|
| 8 | X can be any amino acid | D, E A, V, or P |
| 22 | X is A, S, T, V, I, L, Y, or F | F, Y, A, L, or S |
| 23 | X can be any naturally occurring amino acid | H, N, A, K, Q |
| 27 | X is S, T, V, I, L, Y, or F | I, L, Y, V or T |

In a preferred embodiment, the polypeptide comprises the sequence SEQ ID NO: 48 or amino acids 2-60 of SEQ ID NO: 48.
MXXXVXFDATGEEKXVXXSKISAVYRTGKDXLFSYDXXXGKIGWGYVSEKDAPKE LXXXLXXXXXXXX (SEQ ID NO: 48)
In another embodiment, the polypeptide comprises the sequence MXXXVXFDATGEEKXVDXSKISAVYRTGKDXLFSYDXXXGKIGWGYVSEKDAPK ELXXXLXXXXXXXX (SEQ ID NO: 20) or amino acids 2-60 of SEQ ID NO: 20 or
MXXXVXFDATGEEKXVEXSKISAVYRTGKDXLFSYDXXXGKIGWGYVSEKDAPKE LXXXLXXXXXXXX (SEQ ID NO: 34), or amino acids 2-60 of SEQ ID NO: 34.
The nature of the amino acids X is indicated in Table 1.

In a preferred embodiment, the polypeptide comprises the sequence SEQ ID NO: 49 or amino acids 2-60 of SEQ ID NO: 49.
MXXXVXFAATGEEKXVXXSKIANVYRVGKDXLFSYDXXXGKIGWGYVSEKDAPK ELXXXLXXXXXXXX (SEQ ID NO: 49)
In a further embodiment, the polypeptide comprises the sequence
MXXXVXFDATGEEKXVDXSKISAVYRTGKDXLFSYDXXXGKIGWGYVSEKDAPK ELXXXLXXXXXXXX (SEQ ID NO: 21) or amino acids 2-60 of SEQ ID NO: 21 or
MXXXVXFAATGEEKXVDXSKIANVYRVGKDXLFSYDXXXGKIGWGYVSEKDAPK ELXXXLXXXXXXXX (SEQ ID NO: 35), or amino acids 2-60 of SEQ ID NO: 35. The nature of the amino acids X is indicated in Table 1.

**Table 10. description of amino acids in SEQ ID NO: 45 to SEQ ID NO: 49**

| **Position** | **Amino acid** |
|---|---|
| 17 | X is E or D |

In a further embodiment, the polypeptide comprises the sequence MVKVKFXXXGEEKEVXTSKIXXVYRXGKXVXFXYDDXGKXGWGXVXEKDAPKEL LDLLARAERE (SEQ ID NO: 50) or amino acids 2-58 of SEQ ID NO: 50. In a further embodiment, the polypeptide comprises the sequence MVKVKFXXXGEEKEVDTSKIXXVYRXGKXVXFXYDDNGKXGWGXVXEKDAPKEL LDMLARAERE (SEQ ID NO: 22) or amino acids 2-58 of SEQ ID NO: 22. or
MVKVKFXXXGEEKEVETSKIXXVYRXGKXVXFXYDDQGKXGWGXVXEKDAPKEL LDMLARAERE (SEQ ID NO: 36), or amino acids 2-58 of SEQ ID NO: 36.
The nature of the amino acids X is indicated in Tables 5 and 11.

**Table 5. description of amino acids in SEQ ID NO: 22 and SEQ ID NO: 36 and SEQ I D NO: 50**

| **Position** | **Amino acid** | **Preferred amino acid** |
|---|---|---|
| 7 | X can be any amino acid | D, E A, V, or P |
| 8 | X can be any amino acid | A, L, I, V, T or H |
| 9 | X can be any amino acid | T, I |
| 21 | X is A, S, T, V, I, L, Y, or F | F, Y, A, L, or S |
| 22 | X can be any naturally occurring amino acid | H, N, A, K, Q |
| 26 | X is S, T, V, I, L, Y, or F | I, L, Y, V or T |
| 29 | X can be any amino acid | D, E, S, or A |
| 31 | X is L, I, V, Y, F, M, or H | L, Y, I or M |
| 33 | X is S, T or A | S, T or A |
| 40 | X is T or I | T or I |
| 44 | X is F, Y, I, V, L, M or H | Y, F |
| 46 | X is S, T, H or R | S |

In a further embodiment, the polypeptide comprises the sequence MVKVKFXXTGEEKEVXTSKIXXVYRXGKXVLFXYDDXGKXGWGYVXEKDAPKEL LDLLARAERE (SEQ ID NO: 51) or amino acids 2-58 of SEQ ID NO: 51. In a further embodiment, the polypeptide comprises the sequence MVKVKFXXTGEEKEVDTSKIXXVYRXGKXVLFXYDDNGKXGWGYVXEKDAPKEL LDMLARAERE (SEQ ID NO: 23) or amino acids 2-58 of SEQ ID NO: 23. or
MVKVKFXXTGEEKEVETSKIXXVYRXGKXVLFXYDDQGKXGWGYVXEKDAPKEL LDMLARAERE (SEQ ID NO: 37), or amino acids 2-58 of SEQ ID NO: 37.
The nature of the amino acids X is indicated in Tables 6 and 11.

**Table 6. description of amino acids in SEQ ID NO: 23 and SEQ ID NO: 37 and SEQ ID NO: 51**

| **Position** | **Amino acid** | **Preferred amino acid** |
|---|---|---|
| 7 | X can be any amino acid | D, E A, V, or P |
| 8 | X can be any amino acid | A, L, I, V, T or H |
| 21 | X is A, S, T, V, I, L, Y, or F | F, Y, A, L, or S |
| 22 | X can be any naturally occurring amino acid | H, N, A, K, Q |
| 26 | X is S, T, V, I, L, Y, or F | I, L, Y, V or T |
| 29 | X can be any amino acid | D, E, S, or A |
| 33 | X is S, T or A | S, T or A |
| 40 | X is T or I | T or I |
| 46 | X is S, T, H or R | S |

In a further embodiment, the polypeptide comprises the sequence MVKVKFXATGEEKEVXTSKIXXVYRXGKDVLFSYDDXGKIGWGYVSEKDAPKELL DLLARAERE (SEQ ID NO: 52) or amino acids 2-58 of SEQ ID NO: 52. In a further embodiment, the polypeptide comprises the sequence MVKVKFXATGEEKEVDTSKIXXVYRXGKDVLFSYDDNGKIGWGYVSEKDAPKELL DMLARAERE (SEQ ID NO: 24) or amino acids 2-58 of SEQ ID NO: 24. or
MVKVKFXATGEEKEVETSKIXXVYRXGKDVLFSYDDQGKIGWGYVSEKDAPKELL DMLARAERE (SEQ ID NO: 38), or amino acids 2-58 of SEQ ID NO: 38.
The nature of the amino acids X is indicated in Tables 7 and 11.

**Table 7. description of amino acids in SEQ ID NO: 24 and SEQ ID NO: 38 and SEQ ID NO: 52.**

| **Position** | **Amino acid** | **Preferred amino acid** |
|---|---|---|
| 7 | X can be any amino acid | D, E A, V, or P |
| 21 | X is A, S, T, V, I, L, Y, or F | F, Y, A, L, or S |
| 22 | X can be any naturally occurring amino acid | H, N, A, K, Q |
| 26 | X is S, T, V, I, L, Y, or F | I, L, Y, V or T |

In a further embodiment, the polypeptide comprises the sequence MVKVKFDATGEEKEVXTSKISAVYRTGKDVLFSYDDXGKIGWGYVSEKDAPKELL DLLARAERE (SEQ ID NO: 53) or amino acids 2-58 of SEQ ID NO: 53 (see also table 11)
In a further embodiment, the polypeptide comprises the sequence MVKVKFDATGEEKEVDTSKISAVYRTGKDVLFSYDDNGKIGWGYVSEKDAPKELL DLLARAERE (SEQ ID NO: 25) or amino acids 2-58 of SEQ ID NO: 25. or
MVKVKFDATGEEKEVETSKISAVYRTGKDVLFSYDDQGKIGWGYVSEKDAPKELL DLLARAERE (SEQ ID NO: 39), or amino acids 2-58 of SEQ ID NO: 39.

In a further embodiment, the polypeptide comprises the sequence MVKVKFAATGEEKEVXTSKIANVYRVGKDVLFSYDDXGKIGWGYVSEKDAPKELL DLLARAERE (SEQ ID NO: 54) or amino acids 2-58 of SEQ ID NO: 54 (see also table 11)
In a further embodiment, the polypeptide comprises the sequence MVKVKFAATGEEKEVDTSKIANVYRVGKDVLFSYDDNGKIGWGYVSEKDAPKELL DLLARAERE (SEQ ID NO: 26) or amino acids 2-58 of SEQ ID NO: 26. or
MVKVKFAATGEEKEVETSKIANVYRVGKDVLFSYDDQGKIGWGYVSEKDAPKELL DLLARAERE (SEQ ID NO: 40), or amino acids 2-58 of SEQ ID NO: 40.

**Table 11. description of amino acids in SEQ ID NO: 50 to SEQ ID NO: 54**

| **Position** | **Amino acid** |
|---|---|
| 16 | X is E or D |
| 37 | X is Q or N |

For all the sequences above, such polypeptide may be such that they further comprise a K (lysine) at the C-terminus after the "ERE" pattern.

In a further embodiment, the polypeptide comprises the sequence
MATVKFXXXGEEKEVXISKIXXVYRXGKXIXFXYDEGGGKXGWGXVXEKDAPKEL LQLLEKQ (SEQ ID NO: 55) or amino acids 2-59 of SEQ ID NO: 55 (see also table 12).
In a further embodiment, the polypeptide comprises the sequence MATVKFXXXGEEKEVDISKIXXVYRXGKXIXFXYDEGGGKXGWGXVXEKDAPKEL LQLLEKQ (SEQ ID NO: 27) or amino acids 2-59 of SEQ ID NO: 27. or
MATVKFXXXGEEKEVEISKIXXVYRXGKXIXFXYDEGGGKXGWGXVXEKDAPKEL LQLLEKQ (SEQ ID NO: 41), or amino acids 2-59 of SEQ ID NO: 41.
The nature of the amino acids X is indicated in Table 8.

**Table 8. description of amino acids in SEQ ID NO: 27 and SEQ ID NO: 41 and SEQ ID NO: 55**

| **Position** | **Amino acid** | **Preferred amino acid** |
|---|---|---|
| 7 | X can be any amino acid | D, E A, V, or P |
| 8 | X can be any amino acid | A, L, I, V, T or H |
| 9 | X can be any amino acid | T, I |
| 21 | X is A, S, T, V, I, L, Y, or F | F, Y, A, L, or S |
| 22 | X can be any naturally occurring amino acid | H, N, A, K, Q |
| 26 | X is S, T, V, I, L, Y, or F | I, L, Y, V or T |
| 29 | X can be any amino acid | D, E, S, or A |
| 31 | X is L, I, V, Y, F, M, or H | L, Y, I or M |
| 33 | X is S, T or A | S, T or A |
| 41 | X is T or I | T or I |
| 45 | X is F, Y, I, V, L, M or H | Y, F |
| 47 | X is S, T, H or R | S |

In a further embodiment, the polypeptide comprises the sequence MATVKFXATGEEKEVXISKIXXVYRXGKDILFSYDEGGGKIGWGYVSEKDAPKELL QLLEKQ (SEQ ID NO: 56) or amino acids 2-59 of SEQ ID NO: 56 (see also table 12).
In a further embodiment, the polypeptide comprises the sequence MATVKFXATGEEKEVDISKIXXVYRXGKDILFSYDEGGGKIGWGYVSEKDAPKELL QLLEKQ (SEQ ID NO: 28) or amino acids 2-59 of SEQ ID NO: 28. or
MATVKFXATGEEKEVEISKIXXVYRXGKDILFSYDEGGGKIGWGYVSEKDAPKELL QLLEKQ (SEQ ID NO: 42), or amino acids 2-59 of SEQ ID NO: 42.
The nature of the amino acids X is indicated in Table 9.

**Table 9. description of amino acids in SEQ ID NO: 28 and SEQ ID NO: 42 and SEQ ID NO: 56**

| **Position** | **Amino acid** | **Preferred amino acid** |
|---|---|---|
| 7 | X can be any amino acid | D, E A, V, or P |
| 21 | X is A, S, T, V, I, L, Y, or F | F, Y, A, L, or S |
| 22 | X can be any naturally occurring amino acid | H, N, A, K, Q |
| 26 | X is S, T, V, I, L, Y, or F | I, L, Y, V or T |

In a further embodiment, the polypeptide comprises the sequence MATVKFDATGEEKEVXISKISAVYRTGKDILFSYDEGGGKIGWGYVSEKDAPKELL QLLEKQ (SEQ ID NO: 57) or amino acids 2-59 of SEQ ID NO: 57 (see also table 12).
In a further embodiment, the polypeptide comprises the sequence MATVKFDATGEEKEVDISKISAVYRTGKDILFSYDEGGGKIGWGYVSEKDAPKELL QLLEKQ (SEQ ID NO: 29) or amino acids 2-59 of SEQ ID NO: 29.
or
MATVKFDATGEEKEVEISKISAVYRTGKDILFSYDEGGGKIGWGYVSEKDAPKELL QLLEKQ (SEQ ID NO: 43), or amino acids 2-59 of SEQ ID NO: 43.

In a further embodiment, the polypeptide comprises the sequence MATVKFAATGEEKEVXISKIANVYRVGKDILFSYDEGGGKIGWGYVSEKDAPKELL QLLEKQ (SEQ ID NO: 58) or amino acids 2-59 of SEQ ID NO: 58 (see also table 12).
In a further embodiment, the polypeptide comprises the sequence MATVKFAATGEEKEVDISKIANVYRVGKDILFSYDEGGGKIGWGYVSEKDAPKELL QLLEKQ (SEQ ID NO: 30) or amino acids 2-59 of SEQ ID NO: 30.
or
MATVKFAATGEEKEVEISKIANVYRVGKDILFSYDEGGGKIGWGYVSEKDAPKELL QLLEKQ (SEQ ID NO: 44), or amino acids 2-59 of SEQ ID NO: 44.

**Table 12. description of amino acids in SEQ ID NO: 55 to SEQ ID NO: 58**

| **Position** | **Amino acid** |
|---|---|
| 16 | X is E or D |

When based on the Sac7d scaffold, preferred sequences are SEQ ID NO: 25, SEQ ID NO: 26, SEQ ID NO: 39 or SEQ ID NO: 40, or amino acids 2-58 of these sequences.

Most preferred sequences are SEQ ID NO: 39 or SEQ ID NO: 40, or amino acids 2-58 of these sequences.

When based on the Sso7d scaffold, preferred sequences are SEQ ID NO: 29, SEQ ID NO: 30, SEQ ID NO: 43 or SEQ ID NO: 44 or amino acids 2-59 of these sequences.

The variants binding to C3 and/or C3b herein disclosed contain between 4 to 20 mutations as compared to the wild type corresponding protein. These mutations are preferably at positions corresponding to the positions 7, 8, 9, 21, 22, 26, 29, 31, 33, 40, 44 and 46 of the Sac7 sequence. Among the mutations, it is to be noted that the variants contain a Tyrosine (Y) at position corresponding to 24 of Sac7d and a Tryptophan (W) at position corresponding to 42 of Sac7d. It has indeed been shown that absence of these two amino acids alters C3/C3b binding.

In some embodiments, the variants contain also a Glutamic Acid (E) at position corresponding to 16 of Sac7d and/or a Glutamine (Q) at position corresponding to 37 of Sac7d. These mutations are not essential for binding to C3/C3b, but improve pharmacological properties of the variant.

As seen in the above sequences, the Valine 26 and/or Serine 46 can be maintained when the polypeptide is based on the Sac7d scaffold, as well as Threonine 33 and/or Threonine 40, and/or Alanine 44.

### The Sac7d protein family

The Sac7d family is defined as relating to the Sac7d protein and corresponds to a family of 7 kDa DNA-binding proteins isolated from extremophilic bacteria. It is herein disclosed as a representative species of OB-fold domains, that is preferably used in the context of the invention. Since SH3 domains share homology with OB-fold domains, the teachings pertaining to Sac7d are also applicable to SH3 scaffolds.

These proteins and this family are in particular described in WO 2008/068637. Thus, within the context of the present invention, a protein belongs to the Sac7d family when it has one of the sequences SEQ ID NO: 1 to SEQ ID NO: 15, or when it has a sequence corresponding to the sequence SEQ ID NO: 16, which is a consensus sequence (obtained from SEQ ID NO: 1 to SEQ ID NO: 9 and SEQ ID NO: 12 to SEQ ID NO: 15. In this consensus sequence, a dash - indicates no amino acid, the proteins not having all the same size). This Sac7d family comprises in particular the Sac7d or Sac7e proteins derived from *Sulfolobus acidocaldarius,* the Sso7d protein derived from *Sulfolobus solfataricus,* the DBP 7 also called Sto7 protein derived from *Sulfolobus tokodaii,* the Ssh7b protein derived from *Sulfolobus shibatae,* the Ssh7a protein derived from *Sulfolobus shibatae,* Mse7 derived from *Metallosphaera sedula,* Mcu7 derived from *Metallosphaera cuprina,* Aho7a or Aho7b or Aho7c derived from *Acidianus hospitalis,* Sis7a or Sis7b derived from *Sulfolobus islandicus* and the p7ss protein derived from *Sulfolobus solfataricus.* In view of the broad similarity of the sequences of the proteins of the Sac7d family, it is directly possible and easy to identify the amino acids of another protein than Sac7d that correspond to given amino acids of Sac7d. In particular, one can also use the teachings of WO 2008/068637 that shows (in the figures) and explains (in the specification) that such proteins are superimposable. The contents of this document are incorporated herein by reference, in particular with regards to the description of methods for aligning and superimposing various OB-fold proteins. As indicated above, it is useful to use the numeration of the amino acids from the Sac7d protein for designating a specific amino acid, and the equivalent amino acids can be obtained from Figure 1.

WO 2012/150314 shows that the mutations from one protein of the Sac7d family can be carried to another protein of the same family. This portability amounts to creating a mutant of another protein of the Sac7d family, starting from a mutant of one protein of said family. The first mutant may have been obtained in particular by carrying out the process of WO 2008/068637. Consequently, it is possible, using in particular the teachings of WO 2012/150314 and of figure 1, to obtain a mutant of any protein of the Sac7d family, starting from a mutant of any other protein of such family. As an illustration, for a mutant of Sac7d, one can introduce the mutated amino acids of the Sac7d mutant within the scaffold of another protein, using the sequence alignment of figure 1. As an illustration, variants of Sso7d, obtained from the Sac7d variants herein disclosed, are described as SEQ ID NO: 27, SEQ ID NO: 28 SEQ ID NO: 29, SEQ ID NO: 30, SEQ ID NO: 41, SEQ ID NO: 42, SEQ ID NO: 43, SEQ ID NO: 44, SEQ ID NO: 55, SEQ ID NO: 56, SEQ ID NO: 57 and SEQ ID NO: 58.

Using the consensus sequence, variants are depicted by SEQ ID NO: 17, SEQ ID NO: 18, SEQ ID NO: 19, SEQ ID NO: 20, SEQ ID NO: 21, SEQ ID NO: 31, SEQ ID NO: 32, SEQ ID NO: 33, SEQ ID NO: 34, SEQ ID NO: 35, SEQ ID NO: 45, SEQ ID NO: 46, SEQ ID NO: 47, SEQ ID NO: 48 or SEQ ID NO: 49.

The number of mutated residues introduced within a wild-type protein sequence to obtain a variant is preferably between 4 and 25, or more specifically between 4 and 22 or between 4 and 20. It is thus possible to obtain variants preferably having at least 4, more preferably at least 5, more preferably at least 6, more preferably at least 7 or 8, even more preferably at least 10, but generally less than 25, more preferably less than 22, even more preferably less than 20 or less than 15 or 14 substituted amino acids compared with the wild-type OB-fold protein (or domain). It is to be noted that all and any ranges are considered herein, (such as 5-20 or 7-25 and so forth). Particularly preferred ranges are 4-20, 4-17 and 6-17, 4-14 and 6-14.

It is preferred when 7, 8, 9, 10, 11, 12, 13 or 14 amino acids are mutated in the binding site of the OB-fold domain, relative to the wild-type OB-fold domain. These mutations are thus preferably introduced in the amino acids corresponding to V2, K3, K5, K7, Y8, K9, G10, E11, K13, E14, T17, K21, K22, W24, V26, G27, K28, M29, S31, T33, Y34, D36, N37, G38, K39, T40, R42, A44, S46, E47, K48, D49, A50 and P51 of Sac7d (SEQ ID NO: 1)

In particular, it is interesting when the number of mutated amino acids is between 7 and 14 (limits included). In particular, the variants can also comprise amino acid insertions as indicated in WO 2008/068637.

As indicated, proteins of the Sac7d family are Sac7d or Sac7e derived from *Sulfolobus acidocaldarius,* Sso7d derived from *Sulfolobus solfataricus,* DBP 7 also called Sto7 derived from *Sulfolobus tokodaii,* Ssh7b derived from *Sulfolobus shibatae,* Ssh7a derived from *Sulfolobus shibatae,* Mse7 derived from *Metallosphaera sedula,* Mcu7 derived from *Metallosphaera cuprina,* Aho7a or Aho7b or Aho7c derived from *Acidianus hospitalis,* Sis7a or Sis7b derived from *Sulfolobus islandicus* and p7ss derived from *Sulfolobus solfataricus.* The various sequences of the Sac7d, Sso7d, Sac7e, Ssh7b, Ssh7a, DBP7, Sis7a (3 alleles), Mse7, Mcu7, Aho7a, Aho7b, Aho7c and Sto7 proteins are represented by SEQ ID NO: 1 to SEQ ID NO: 15 respectively.

A variant of a protein of this Sac7d family may be called a nanofitin. The invention is thus preferentially implemented on variants of the proteins represented by SEQ ID NO: 1 to SEQ ID NO: 15, or a protein having a sequence that reads on SEQ ID NO: 16 (consensus sequence), in particular on variants of Sac7d.

### Link of Sac7d protein with OB-fold proteins and SH3 domains

The OB-fold proteins are known in the art. They are in particular described in the documents cited above, and also in Arcus (Curr Opin Struct Biol. 2002 Dec; 12(6):794-801). OB-fold is in the form of a cylinder having five beta (β) sheets. Most OB-fold proteins use the same binding interface of their natural ligand, which may be an oligosaccharide, an oligonucleotide, a protein, a metal ion or a catalytic substrate. This binding interface comprises mainly the residues located in the beta sheets. Certain residues located in the loops may also be involved in the binding of an OB-fold protein with its natural ligand. Thus, applications WO 2007/139397 and WO 2008/068637 and the Arcus document (2002, op. cit.) describe the OB-fold-protein domains for binding with their natural ligand.

In particular, document WO 2008/068637 describes precisely how to identify the binding domain of an OB-fold protein. By superimposing several sequences and 3D structures of proteins having OB-fold domains, using WU-Blast2 (Lopez et al., 2003, Nucleic Acids Res 31, 3795-3798), T-COFFEE ((Notredame et al., 2000, J Mol Biol 302, 205-217) or DALI lite (Holm and Park, 2000, Bioinformatics 16, 566-567), it is possible to identify the positions of the binding domains and in particular the amino acids which can be modified. Taking the sequence of Sac7d (SEQ ID NO: 1) as a reference, these are the residues V2, K3, K5, K7, Y8, K9, G10, E11, K13, E14, T17, K21, K22, W24, V26, G27, K28, M29, S31, T33, Y34, D35, D36, N37, G38, K39, T40, G41, R42, A44, S46, E47, K48, D49, A50 and P51.

WO 2008/068637 describes that it is possible to perform a superimposition of 3D structures of OB-fold proteins or domains (10 domains were used in this application, including Sac7d), using the DALI website (http://www.ebi.ac.uk/dali/interactive.html)(Holm and Sander, 1998, Nucleic Acids Res 26, 316-319). Thus, it is easy to identify, for any OB-fold protein (or any OB-fold domain), the amino acids involved in the binding site and corresponding to the Sac7d amino acids mentioned above. Consequently, providing the amino acids that can be mutated in one of these proteins makes it possible to identify the corresponding amino acids for any other OB-fold domain.

It is also to be noted that OB-fold domains resemble SH3 domains and that it is also possible to identify equivalents of amino acids of Sac7d in SH3 domains.

### Example of OB-fold or SH3 domain

Non-limitative examples of OB-fold proteins which can be used according to the invention are Sac7d, Sso7d, the N-terminal domain of SEB (Papageorgiou et al., 1998), the chain A of the Shiga-like toxin IIe (PDB 2bosa), the human Neutrophil Activatin Peptide-2 (NAP-2, PDB 1tvxA), the Molybdenum Binding Protein (modg) of Azotobacter vinelandii (PDB 1h9j), the N-terminal domain of SPE-C (Roussel et al., 1997), the B5 subunit of E. coli Shiga-like toxin (Kitov et al., 2000), Cdc13 (Mitton-Fry et al., 2002), the cold-shock DNA-binding domain of the human Y-box protein YB-1 (Kloks et al., 2002), the E. coli inorganic pyrophosphatase EPPase (Samygina et al., 2001), or any of the proteins listed in Table 3 of the article by (Arcus, 2002), such as 1krs (Lysyl-tRNA synthetase LysS, E.coli), 1c0aA (Asp- tRNA synthetase, E.coli), 1b8aA (Asp- tRNA synthetase, P. kodakaraensis), 1lylA (Lysyl-tRNA synthetase LysU, E.coli), 1quqA (Replication protein A, 32kDa subunit, Human), 1quqB (Replication protein A, 14kDa subunit, Human), 1jmcA (Replication protein A, 70kDa subunit (RPA70) fragment, Human), 1otc (Telomere-end-binding protein, O. nova), 3ullA (Mitochondrial ssDNA-binding protein, Human), 1prtF (Pertussis toxin S5 subunit, B. pertussis), 1bcpD (Pertussis toxin S5 subunit (ATP bound), B. pertussis), 3chbD (Cholera Toxin, V. cholerae), 1tiiD (Heat-labile toxin, E. coli), 2bosA (Verotoxin-1/Shiga toxin, B-pentamer, E. coli), 1br9 (TIMP-2, Human), 1an8 (Superantigen SPE-C, S. pyogenes), 3seb (Superantigen SPE, S. aureus), 1aw7A (Toxic shock syndrome toxin, S. aureus), 1jmc (Major cold-shock protein, E.coli), 1bkb (Initiation translation factor 5a, P. aerophylum), 1sro (S1 RNA-binding domain of PNPase, E.coli), 1d7qA (Initiation translation factor 1, elF1a, Human), 1ah9 (Initiation translation factor 1, IF1, E.coli), 1b9mA (Mo-dependent transcriptional regulator ModE, E.coli), 1ckmA (RNA guanylyltransferase, Chlorella virus, PBCV-1), 1a0i (ATP-dependent DNA ligase, Bacteriophage T7), 1snc (Staphylococcal nuclease, S. aureus), 1hjp (DNA helicase RuvA subunit, N-terminal domain, E.coli), 1pfsA (Gene V protein, Pseudomonas bacteriophage pf3), 1gvp (Gene V protein, Filamentous bacteriophage (f1, M13)), 1gpc (Gene 32 protein (gp32) core, Bacteriophage T4), 1wgjA (Inorganic pyrophosphatase, S. cerevisiae), and 2prd (Inorganic pyrophosphatase, T. thermophilus).

Non-exhaustive examples of proteins with SH3 domaines are Signal transducing adaptor proteins, CDC24, Cdc25, PI3 kinase, Phospholipase, Ras GTPase-activating protein, Vav proto-oncogene, GRB2, p54 S6 kinase 2 (S6K2), SH3D21, C10orf76 (potentially), STAC3, Some myosins, SHANK1,2,3, ARHGAP12, C8orf46, TANGO1, Integrase, Focal Adhesion Kinase (FAK, PTK2), Proline-rich tyrosine kinase (Pyk2, CADTK, PTK2beta), or TRIP10 (cip4).

### Description of C3 and/or C3b-binding variants based on Sac7d and Aho7c

Sac7d and Aho7c are proteins that have large similarity. One can also note that Sto (SEQ ID NO: 15) also present similarity with these proteins.

SEQ ID NO: 104 corresponds to the consensus sequence of after alignment of amino acids 2-66 of Sac7d (SEQ ID NO: 1) and 3-60 of Aho7c (SEQ ID NO: 14). The starting amino acids have been omitted as they are not essential in the structure of the proteins.

XKVKFKYKGEEKEVDXSKIKKVWRVGKMXSFTYDDNGKTGRGAVSEKDAPK ELLXXXXXXXXXX (SEQ ID NO: 104)

In this consensus sequences, X (or Xaa) are as in Table 13.

**Table 13. Description of some amino acids represented as Xaa in SEQ ID NO: 59-73.**

| Position | Amino-acid | Preferred 1 Sac7d | Preferred 2 Aho7c |
|---|---|---|---|
| 1 | V or T | V | T |
| 16 | T or I | T | I |
| 29 | V or I | V | I |
| 55-64 | DMLARAEREK, DLLARAEREK or EKLK------ | DMLARAEREK or DLLARAEREK | EKLK |

Consensus sequence for variants binding to C3 and/or C3b are represented by

**Table 14: sequences of the variants based on the consensus sequence of Sac7d-Aho7c. Amino acids represented by X are further described in Tables 13, 15, and 16.**

| SEQ ID NO: | Sequence |
|---|---|
| 59 | XKVKFXXXGEEKEVXXSKIXXVYRXGKXXXFXYDDXGKXGWGX VXEKDAPKELLXXXXXXXXX |
| 60 | XKVKFXXTGEEKEVXXSKIXXVYRXGKXXLFXYDDXGKXGWGY VXEKDAPKELLXXXXXXXXXX |
| 61 | XKVKFXATGEEKEVXXSKIXXVYRXGKDXLFSYDDXGKIGWGY VSEKDAPKELLXXXXXXXXXX |
| 62 | XKVKFDATGEEKEVXXSKISAVYRTGKDXLFSYDDXGKIGWGY VSEKDAPKELLXXXXXXXXXX |
| 63 | XKVKFAATGEEKEVXXSKIANVYRVGKDXLFSYDDXGKIGWGY VSEKDAPKELLXXXXXXXXXX |
| 64 | XKVKFXXXGEEKEVDXSKIXXVYRXGKXXXFXYDDNGKXGWGX VXEKDAPKELLXXXXXXXXXX |
| 65 | XKVKFXXTGEEKEVDXSKIXXVYRXGKXXLFXYDDNGKXGWGY VXEKDAPKELLXXXXXXXXXX |
| 66 | XKVKFXATGEEKEVDXSKIXXVYRXGKDXLFSYDDNGKIGWGY VSEKDAPKELLXXXXXXXXXX |
| 67 | XKVKFDATGEEKEVDXSKISAVYRTGKDXLFSYDDNGKIGWGY VSEKDAPKELLXXXXXXXXXX |
| 68 | XKVKFAATGEEKEVDXSKIANVYRVGKDXLFSYDDNGKIGWGY VSEKDAPKELLXXXXXXXXXX |
| 69 | XKVKFXXXGEEKEVEXSKIXXVYRXGKXXXFXYDDQGKXGWGX VXEKDAPKELLXXXXXXXXXX |
| 70 | XKVKFXXTGEEKEVEXSKIXXVYRXGKXXLFXYDDQGKXGWGY VXEKDAPKELLXXXXXXXXXX |
| 71 | XKVKFXATGEEKEVEXSKIXXVYRXGKDXLFSYDDQGKIGWGY VSEKDAPKELLXXXXXXXXXX |
| 72 | XKVKFDATGEEKEVEXSKISAVYRTGKDXLFSYDDQGKIGWGY VSEKDAPKELLXXXXXXXXXX |
| 73 | XKVKFAATGEEKEVEXSKIANVYRVGKDXLFSYDDQGKIGWGY VSEKDAPKELLXXXXXXXXXX |

**Table 15. description of amino acids indicated as Xaa in SEQ ID NO: 59-103. It is to be noted that this table is to be used for the sequences mentioned above, for the Xaa that they bear. For some sequences, an amino acid is specified at some of the positions of Table 15 and the line of the table is thus not applicable. The listing sequence depicts the Xaa of the second column of this table.**

| Position | Type of Amino-acid | Preferred amino acid | Preferred Property | Preferred 1 (B10-3) | Preferred 2 (H03-3) |
|---|---|---|---|---|---|
| 6 | any amino acid | D, E A, V, or P | tolerant | D | A |
| 7 | any amino acid | A, L, I, V, T or H | tolerant | A | A |
| 8 | any amino acid | T, I | tolerant | T | T |
| 20 | A, S, T, V, I, L, Y, or F | F, Y, A, L, or S | hydrophobic | S | A |
| 21 | any amino acid | H, N, A, K, Q | tolerant | A | N |
| 23 | Y | Y | Y | Y | Y |
| 25 | S, T, V, I, L, Y, F | I, L, Y, V or T | - | T | V |
| 28 | any amino acid | D, E, S, or A | tolerant | D | D |
| 30 | L, I, V, Y, F, M, or H | L, Y, I or M | hydrophobic | L | L |
| 32 | S, T or A | S, T or A | | S | S |
| 39 | T or I | T or I | - | I | I |
| 41 | W | W | W | W | W |
| 43 | F, Y, I, V, or L, M or H | Y, F | hydrophobic | Y | Y |
| 45 | S, T, H or R | S | - | S | S |

In these sequences, some amino acids that are not involved in binding can also be modified on the variants, without modifying the binding and biological properties of the proteins. They are represented in Table 16.

**Table 16. Amino acids represented as Xaa in SEQ ID NO: 59-103. Xaa at position 56 is only present in SEQ ID NO: 74-88.**

| Position | Amino-acid |
|---|---|
| 15 | Xaa is D or E |
| 36 | Xaa is N or Q |
| 56 | Xaa is M or L |

### Description of C3 and/or C3b-binding variants based on Sac7d (SEQ ID NO: 1)

Specific variants based on Sac7d, binding to C3 and/or C3b are depicted by SEQ ID NO: 74 to SEQ ID NO: 88.

In these sequences, the starting methionine (M) of the Sac7d protein has been omitted. It has indeed been shown by the Applicant that the activity of the proteins is not modified when this amino acid is deleted. Likewise, it is possible to omit the last 7 amino acids from the variants and retain activity.

Consequently, proteins depicted by amino acids 1-57 of any of SEQ ID NO: 74 to SEQ ID NO: 88 are also variants according to the invention. One can also cite proteins depicted by amino acids 1-58, 1-59, 1-60, 1-61, 1-62, 1-63 of any of SEQ ID NO: 74 to SEQ ID NO: 88, which are also variants according to the invention.

Consequently, the polypeptide comprise any of SEQ ID NO: 71 to SEQ ID NO: 88, or any truncated protein based on these sequences, and as disclosed above.

Such variants are represented by

**Table 17: sequences of the variants based on Sac7d. Amino acids represented by X are further described in Tables 15 and 16.**

| SEQ ID NO: | Sequence |
|---|---|
| 74 | VKVKFXXXGEEKEVXTSKIXXVYRXGKXVXFXYDDXGKXGWGX VXEKDAPKELLDXLARAEREK |
| 75 | VKVKFXXTGEEKEVXTSKIXXVYRXGKXVLFXYDDXGKXGWGY VXEKDAPKELLDXLARAEREK |
| 76 | VKVKFXATGEEKEVXTSKIXXVYRXGKDVLFSYDDXGKIGWGYV SEKDAPKELLDXLARAEREK |
| 77 | VKVKFDATGEEKEVXTSKISAVYRTGKDVLFSYDDXGKIGWGYV SEKDAPKELLDXLARAEREK |
| 78 | VKVKFAATGEEKEVXTSKIANVYRVGKDVLFSYDDXGKIGWGY VSEKDAPKELLDXLARAEREK |
| 79 | VKVKFXXXGEEKEVDTSKIXXVYRXGKXVXFXYDDNGKXGWGX VXEKDAPKELLDMLARAEREK |
| 80 | VKVKFXXTGEEKEVDTSKIXXVYRXGKXVLFXYDDNGKXGWGY VXEKDAPKELLDMLARAEREK |
| 81 | VKVKFXATGEEKEVDTSKIXXVYRXGKDVLFSYDDNGKIGWGY VSEKDAPKELLDMLARAEREK |
| 82 | VKVKFDATGEEKEVDTSKISAVYRTGKDVLFSYDDNGKIGWGY VSEKDAPKELLDMLARAEREK |
| 83 | VKVKFAATGEEKEVDTSKIANVYRVGKDVLFSYDDNGKIGWGY VSEKDAPKELLDMLARAEREK |
| 84 | VKVKFXXXGEEKEVETSKIXXVYRXGKXVXFXYDDQGKXGWGX VXEKDAPKELLDLLARAEREK |
| 85 | VKVKFXXTGEEKEVETSKIXXVYRXGKXVLFXYDDQGKXGWGY VXEKDAPKELLDLLARAEREK |
| 86 | VKVKFXATGEEKEVETSKIXXVYRXGKDVLFSYDDQGKIGWGY VSEKDAPKELLDLLARAEREK |
| 87 | VKVKFDATGEEKEVETSKISAVYRTGKDVLFSYDDQGKIGWGY VSEKDAPKELLDLLARAEREK |
| 88 | VKVKFAATGEEKEVETSKIANVYRVGKDVLFSYDDQGKIGWGY VSEKDAPKELLDLLARAEREK |

As indicated above, it is possible to modify D16 of Sac7d (which is located at position 15 in SEQ ID NO: 74 to SEQ ID NO: 88, as the M1 present in Sac7d has been omitted), N37 of Sac7d (herein located at position 36) or M57 of Sac7d (herein located at position 56) as disclosed in Table 16.

As for the consensus sequence of Sac7d / Aho7c, any combination of substitution is foreseen (numbering is with respect to SEQ ID NO: 74 to SEQ ID NO: 88), even though the sequence listing doesn't all depict them:

| | | | |
|---|---|---|---|
| D15 | N36 | M56 | SEQ ID NO: 79-83 |
| D15 | N36 | L56 | |
| D15 | Q36 | M56 | |
| D15 | Q36 | L56 | |
| E15 | N36 | M56 | |
| E15 | N36 | L56 | |
| E15 | Q36 | M56 | |
| E15 | Q36 | L56 | SEQ ID NO: 84-88 |

As indicated above, the sequences all contain the mutated amino acids Y23 and W41 (corresponding respectively to position 24, and 42 of SEQ ID NO: 1, which contain the N-terminus methionine), which differ from the amino acids that are naturally present in Sac7d.

The Applicant has indeed found that these amino acids are present in various variants binding to C3 and/or C3b, and that modification of such leads to decrease of binding (loss of affinity or loss of binding). The other amino acids can be variable, under conditions mentioned in Table 15.

It is preferred when the variants contain the T8 (corresponding to position 9 of SEQ ID NO: 1), L30 (corresponding to position 31 of SEQ ID NO: 1) and Y43 (corresponding to position 44 of SEQ ID NO: 1).

Very interesting variants are depicted by SEQ ID NO: 76, SEQ ID NO: 77, SEQ ID NO: 78, SEQ ID NO: 81, SEQ ID NO: 82, SEQ ID NO: 83, SEQ ID NO: 86, SEQ ID NO: 87, SEQ ID NO: 88. These variants all further contain A7, D28, S32, I39 and S45 (corresponding respectively to positions 8, 29, 33, 40 and 46 of SEQ ID NO: 1).

SEQ ID NO: 87 (named B10-3) is of particular interest, as well as SEQ ID NO: 82.

SEQ ID NO: 88 (named H03-3) is also of particular interest, as well as SEQ ID NO: 83.

### Description of proteins based on Aho7c (SEQ ID NO: 14)

Variants of Aho7c, binding to C3 and/or C3b are depicted by SEQ ID NO: 89 to SEQ ID NO: 103. As indicated above, such protein is very similar to Sac7d. It has also been shown and reminded herein that mutations of Sac7d can be carried from Sac7d to another protein (WO 2012/150314).

In these sequences, the starting methionine and alanine (MA) of the Aho7c protein (SEQ ID NO: 14) have been omitted. It has indeed been shown by the Applicant that the activity of the proteins is not modified when these amino acids are deleted. Likewise, it is possible to omit the last 4 amino acids from the variants and retain activity.

Consequently, proteins depicted by amino acids 1-55 of any of SEQ ID NO: 89 to SEQ ID NO: 103 are also variants according to the invention. One can also cite proteins depicted by amino acids 1-56, 1-57, 1-58 of any of SEQ ID NO: 89 to SEQ ID NO: 103, which are also variants according to the invention.

Consequently, the polypeptide comprise any of SEQ ID NO: 89 to SEQ ID NO: 103, or any truncated protein based on these sequences, and as disclosed above.

Such variants are represented by

**Table 18: sequences of the variants based on Ao7c. Amino acids represented by X are further described in Tables 15 and 16.**

| SEQ ID NO: | Sequence |
|---|---|
| 89 | TKVKFXXXGEEKEVXISKIXXVYRXGKXIXFXYDDXGKXGWGXV XEKDAPKELLEKLK |
| 90 | TKVKFXXTGEEKEVXISKIXXVYRXGKXILFXYDDXGKXGWGYV XEKDAPKELLEKLK |
| 91 | TKVKFXATGEEKEVXISKIXXVYRXGKDILFSYDDXGKIGWGYVS EKDAPKELLEKLK |
| 92 | TKVKFDATGEEKEVXISKISAVYRTGKDILFSYDDXGKIGWGYVS EKDAPKELLEKLK |
| 93 | TKVKFAATGEEKEVXISKIANVYRVGKDILFSYDDXGKIGWGYVS EKDAPKELLEKLK |
| 94 | TKVKFXXXGEEKEVDISKIXXVYRXGKXIXFXYDDNGKXGWGXV XEKDAPKELLEKLK |
| 95 | TKVKFXXTGEEKEVDISKIXXVYRXGKXILFXYDDNGKXGWGYV XEKDAPKELLEKLK |
| 96 | TKVKFXATGEEKEVDISKIXXVYRXGKDILFSYDDNGKIGWGYVS EKDAPKELLEKLK |
| 97 | TKVKFDATGEEKEVDISKISAVYRTGKDILFSYDDNGKIGWGYVS EKDAPKELLEKLK |
| 98 | TKVKFAATGEEKEVDISKIANVYRVGKDILFSYDDNGKIGWGYV SEKDAPKELLEKLK |
| 99 | TKVKFXXXGEEKEVEISKIXXVYRXGKXIXFXYDDQGKXGWGXV XEKDAPKELLEKLK |
| 100 | TKVKFXXTGEEKEVEISKIXXVYRXGKXILFXYDDQGKXGWGYV XEKDAPKELLEKLK |
| 101 | TKVKFXATGEEKEVEISKIXXVYRXGKDILFSYDDQGKIGWGYVS EKDAPKELLEKLK |
| 102 | TKVKFDATGEEKEVEISKISAVYRTGKDILFSYDDQGKIGWGYVS EKDAPKELLEKLK |
| 103 | TKVKFAATGEEKEVEISKIANVYRVGKDILFSYDDQGKIGWGYV SEKDAPKELLEKLK |

As indicated above, it is possible to modify D17 of Aho7c (which is located at position 15 in SEQ ID NO: 89 to SEQ ID NO: 103, as the M1A2 present in Aho7c have been omitted), or N38 of Aho7c (herein located at position 36) as disclosed in Table 16.

As for the consensus sequence of Sac7d / Aho7c, any combination of substitution is foreseen (numbering is with respect to SEQ ID NO: 89 to SEQ ID NO: 103), even though the sequence listing doesn't all depict them:

| | | |
|---|---|---|
| D15 | N36 | SEQ ID NO: 94-98 |
| D15 | Q36 | |
| E15 | N36 | |
| E15 | Q36 | SEQ ID NO: 99-103 |

As indicated above, the sequences all contain the mutated amino acids Y23 and W41 (corresponding respectively to position 25, and 43 of SEQ ID NO: 14, which contain the N-terminus methionine and alanine), which differ from the amino acids that are naturally present in Aho7c.

The Applicant has indeed found that these amino acids are present in various variants binding to C3 and/or C3b, and that modification of such leads to decrease of binding (loss of affinity or loss of binding). The other amino acids can be variable, under conditions mentioned in Table 15.

It is preferred when the variants contain the T8 (corresponding to position 10 of SEQ ID NO: 1), L30 (corresponding to position 32 of SEQ ID NO: 1) and Y43 (corresponding to position 45 of SEQ ID NO: 14).

Very interesting variants are depicted by SEQ ID NO: 91, SEQ ID NO: 92, SEQ ID NO: 93, SEQ ID NO: 96, SEQ ID NO: 97, SEQ ID NO: 98, SEQ ID NO: 101, SEQ ID NO: 102, SEQ ID NO: 103. These variants all further contain A7, D28, S32, I39 and S45 (corresponding respectively to positions 9, 30, 34, 41 and 47 of SEQ ID NO: 14).

SEQ ID NO: 102 (based on B10-3) is of particular interest, as well as SEQ ID NO: 97.

SEQ ID NO: 103 (based on H03-3) is also of particular interest, as well as SEQ ID NO: 98.

### Production of the identified variants

The sequence of the identified variant can be cloned in any appropriate vector by any molecular genetic methods known in the art.

These recombinant DNA constructs comprising a nucleotide sequence, coding for a polypeptide comprising a variant as described above, are used in connection with a vector, such as a plasmid, phagemid, phage or viral vector.

These recombinant acid molecules can be produced by techniques described in Sambrook et al., 1989 (Sambrook J, Fritsch EF and Maniatis T (1989) Molecular cloning: a laboratory manual, Cold Spring Harbor Laboratory Press, New York). Alternatively, the DNA sequences may be chemically synthesized using, for example, synthesizers.

Recombinant constructs of the invention comprise the expression vectors that are capable of expressing the RNA and thus lead to production of proteins from the above genetic sequences. The vector may thus further comprise regulatory sequences, including a suitable promoter operably linked to the open reading frame (ORF) of the genetic sequences herein disclosed. The vector may further comprise a selectable marker sequence such as an antibiotic resistance gene. Specific initiation and bacterial secretory signals also may be required for efficient translation of the coding sequences when bacteria as used as the expression host.

### Production of the molecules

Cells are transfected or transformed with vectors containing the sequences coding for the polypeptides comprising the variant as disclosed above.

The cells are the cultured in such conditions as to have the protein expressed and favorably secreted. The conditions of culture of the cells are the conditions generally used for recombinant antibody production and are known in the art. Such conditions that are known in the art can also be optimized by the person skilled in the art if needed. Kunert and Reinhart (Appl Microbiol Biotechnol. 2016; 100: 3451-3461) review such methods and provide ample references thereto.

One can use bacterial, phage (Shukra et al, Eur J Microbiol Immunol (Bp). 2014; 4(2): 91-98) or eukaryotic systems of production.

One shall prefer to use eukaryotic cells in order to obtain proper post-translational modifications such as glycosylation.

In particular, one can use CHO (Chinese Hamster Ovary) cells, PER.C6 cells (human cell line, Pau et al, Vaccine. 2001 21;19(17-19):2716-21), HEK 293b cells (Human embryonic kidney cells 293), NS0 cells (cell line derived from the non-secreting murine myeloma) or EB66 cells (a duck cell line Valneva, Lyons, France).

Also provided by the present disclosure are host cells containing at least one of the DNAs constructs coding for a polypeptide comprising the variant as disclosed herein. The host cell can be any cell for which expression vectors are available. As indicated above, it may be a higher eukaryotic host cell, such as a mammalian cell, a lower eukaryotic host cell, such as a yeast cell, or a prokaryotic cell, such as a bacterial cell.

Introduction of the recombinant construct into the host cell is performed by any method known in the art (such as calcium phosphate transfection, lipofection, DEAE, dextran mediated transfection, electroporation or phage infection). The vectors can be inserted within the genome of the host cell, or be maintained as an extragenomic vector (such as a Bacterial Artificial Chromosome or a Yeast Artificial Chromosome). When introduced within the cell genome, such introduction may be random or targeted using methods known in the art (homologous recombination or the like).

### Bacterial hosts and expression

Useful expression vectors for bacterial use are constructed by inserting the recombinant DNA sequence together with suitable translation initiation and termination signals in operable reading phase with a functional promoter. The vector will comprise one or more phenotypic selectable markers and an origin of replication to ensure maintenance of the vector and, if desirable, to provide amplification within the host.

Suitable prokaryotic hosts for transformation include *E. coli, Bacillus subtilis, Salmonella typhimurium* and various species within the genera *Pseudomonas, Streptomyces,* and *Staphylococcus.*

### Eukaryotic hosts and expression

Examples of the eukaryotic host cells include vertebrate cells, insect cells, and yeast cells. In particular, one can use the cells mentioned above.

The transformed or transfected cells are cultured according to methods known in the art and the polypeptide are recovered from intracellular or extracellular fractions (depending on whether it is secreted or not).

### Isolation of the molecules

The recombinant protein produced can be separated and purified by any of various known separation methods utilizing the physical or chemical property of the protein, from the intracellular or extracellular fraction.

In particular, one can use methods such as precipitation, ultrafiltration, various types of liquid chromatography such as molecular sieve chromatography (gel filtration), adsorption chromatography, ion exchange chromatography, and affinity chromatography, dialysis, and a combination thereof.

In general, any method known and used to purify recombinant polypeptides is adapted for the purification of the molecules herein disclosed.

If a tag has been introduced within the recombinant sequence (such as a poly-Histidine tag), one can purify the molecules using this tag. However, in certain embodiments, it is preferred to use affinity to purify the molecules.

One can, in particular, use the fact that the molecule herein produced binds to a specific target and use any affinity method (affinity column, FACS, beads) to isolate such molecules.

One particular advantage of the molecules herein disclosed is that they don't need to be glycosylated to be active and can thus be produced in any type of cells, and not necessarily eukaryotic cells. They are particularly well produced in bacterial cells.

### Modification of the variants

The variants described above, and having the ability to bind C3 and/or C3b can be modified by any method known in the art.

### Preparing a polypeptide comprising the variant

It is possible to prepare a DNA sequence containing two coding sequences, in frame, one for the variant herein disclosed and another for a protein or peptide of interest. The resulting expressed protein would thus be a polypeptide that contains both proteins. The vector may be built in such a way that it would contain a sequence coding for a linker that would be located between the two proteins in the expressed polypeptide.

Consequently, the invention also encompasses a polypeptide comprising the variant of a protein of the OB-fold protein (preferably of the Sac7d family) binding to C3 and/or C3b which is fused or linked (preferably through amine bond as disclosed above) to another protein or polypeptide.

In a specific embodiment, the other protein or polypeptide comprises another variant of a protein of the Sac7d family, in particular as herein disclosed.

Are of particular interest
- A polypeptide comprising a variant of an OB-fold protein of the Sac7d family binding to C3 and/or C3b, fused with a variant of an OB-fold protein of the Sac7d family binding to albumin or PD-L1.

In another embodiment, the other protein or polypeptide is an antibody. In this embodiment, the variant of the OB-fold domain is fused to at least one of the heavy or light chain of an immunoglobulin monomer, preferably at the N- or C-terminus of the light or heavy chain. In another embodiment, the variant may be fused to both heavy or light chains.

In order to obtain such compounds, one can use a genetic construct comprising a DNA sequence selected from the group consisting of
a. the sequence coding for the heavy chain of an antibody fused, at its 3'end with the sequence coding for the variant of an OB-fold protein (potentially with an sequence coding for a linker)
b. the sequence coding for the heavy chain of an antibody fused, at its 5' end with the sequence coding for the variant of an OB-fold protein (potentially with an sequence coding for a linker)
c. the sequence coding for the light chain of an antibody fused, at its 3' end with the sequence coding for the variant of an OB-fold protein (potentially with an sequence coding for a linker)
d. the sequence coding for the light chain of an antibody fused, at its 5' end with the sequence coding for the variant of an OB-fold protein (potentially with an sequence coding for a linker).

This fusion can be made at the N-terminus and/or the C-terminus of the antibody chain (heavy and/or light chain). It is to be noted that, especially when using a small OB-fold domain (about 70 amino acids) such as a protein from the Sac7d family, it is possible to obtain a molecule that will have the structure of the antibody (two light chains paired to two heavy chains, and such dimers paired together), with the antibody regions, and further binding regions consisting of the modified OB-fold domain.

In a specific embodiment, the antibody part of the protein herein disclosed is a IgG molecule.

In another embodiment, the antibody part of the protein herein disclosed is a IgA molecule.

In another embodiment, the antibody part of the protein herein disclosed is a IgM molecule.

In another embodiment, the antibody part of the protein herein disclosed is a IgD molecule.

In another embodiment, the antibody part of the protein herein disclosed is a IgE molecule.

The antibody may be a human antibody, a rodent antibody (such as a mouse antibody or a rat antibody), a cat antibody, a dog antibody, a chicken antibody, a goat antibody, a camelid antibody (such as a camel antibody, a llama antibody, an alpaca antibody or a nanobody), a shark antibody, or an antibody from any other species. It may be a chimeric or humanized antibody. As reminded in Wikipedia, humanized antibodies are antibodies from non-human species whose protein sequences have been modified to increase their similarity to antibody variants produced naturally in humans. A chimeric antibody contains sequences from different species.

It is preferred when the antibody that is part of the molecule herein disclosed is an antibody that comprises two identical heavy chains (of about 400 to 500 amino acids, generally around 450 amino acids) and two identical light chains. Consequently, the antibody comprises the same Fab variable regions. This antibody is therefore a monospecific antibody where both parts (combination of light and heavy chains) of the antibody bind to the same epitope of an antigen.

However, the antibody may present different heavy and/or light chains. In particular, in some embodiments, the antibody is a bi-specific antibody. The term "antibody" thus encompasses both "classical antibodies" as disclosed above having identical heavy and light chains, but also engineered antibodies that have more than one specificity.

In a specific embodiment, the antibody presents one heavy and light chain from one antibody and another heavy and light chain from another antibody.

The antibody may bind to a target selected in the group consisting of:
Cell surface receptors: Insulin receptor, Low density lipoprotein receptor-related protein 1, Transferrin receptor, Epidermal growth factor receptor, Epidermal growth factor receptor variant III, Her2, Her3, Her4, PMSA, IGF-1R, GITR, RAGE, CD28.

Cell surface proteins: Mesothelin, EpCam, CD19, CD20, CD38, CD3, TIM-3, CEA, cMet, ICAM1, ICAM3, MadCam, a4b7, CD7, CD4, CD138.

Angiogenesis factors and Growth factors: Angiopoietin 2, HGF, PDGF, EGF, GM-CSF, HB-EGF, TGF

Immune checkpoint inhibitors or activators: PD-1, PD-L1, CTLA4, CD28, B7-1, B7-2, ICOS, ICOSL, B7-H3, B7-H4, LAG3, KIR, 4-1BB, OX40, CD27, CD40L, TIM3, A2aR

Circulating proteins: TNFa, IL23, IL12, IL33, IL4, IL13, IL5, IL6, IL4, IFNg, IL17, RANKL, Bace1, alpha Synuclein, Tau, amyloid.

Alternatively, the polypeptide may contain a variant as disclosed above, and a biologically active molecule, such as an erythropoietin, an interferon, or etanercept.

In another embodiment, the variant of the OB-fold domain (in particular the variant of a protein of the Sac7d family) is conjugated to an organic molecule. This may be done by any method known in the art. In particular, one can chemically link the molecule to the protein. As a molecule, one can cite antiproliferative (cytotoxic and cytostatic agents) include cytotoxic compounds (e.g., broad spectrum), angiogenesis inhibitors cell cycle progression inhibitors, PBK/m-TOR/AKT pathway inhibitors, MAPK signaling pathway inhibitors, kinase inhibitors, protein chaperones inhibitors, HDAC inhibitors, PARP inhibitors, Wnt/Hedgehog signaling pathway inhibitors, RNA polymerase inhibitors and proteasome inhibitors. One can also use anti-inflammatory molecules.

One can cite in particular DNA-binding or alkylating drugs such as anthracyclines (doxorubicin, epirubicin, idarubicin, daunorubicin) and its analogs, alkylating agents, such as calicheamicins, dactinomycines, mitromycines, pyrrolobenzodiazepines, and the like. One can also cite cell cycle progression inhibitors such as CDK inhibitors, Rho-kinase inhibitors, checkpoint kinase inhibitors, aurora kinase inhibitors, PLK inhibitors, and KSP inhibitors. One can also cite thalidomide and its derivatives lenalidomide and pomalidomide. In order for treating inflammation disorders, one can also use cyclooxygenase-2 inhibitors, 5-lipoxygenase inhibitors, quercetin and/or resveratrol as molecules conjugated to the polypeptide comprising the variant.

Interesting and preferred molecules are also disclosed above.

### Use of the variants

The variants can be used in particular in therapeutic methods, especially for treating a complement mediated or complement associated disease.

The invention thus relates to a method of treatment of a complement mediated or complement associated disease, comprising the administration of a therapeutic amount of a polypeptide as disclosed or of a variant of an OB-fold herein disclosed (in particular a variant of a protein of the Sac7d family) to a subject in need thereof. The invention also relates at a method for inhibiting complement cascade in a subject, comprising the administration of an effective amount of a polypeptide as disclosed or of a variant of an OB-fold herein disclosed (in particular a variant of a protein of the Sac7d family) to the subject.

The term "therapeutic amount" or "effective amount", as used herein, is the amount sufficient to effect beneficial or desired results, such as clinical results, and an "effective amount" depends upon the context in which it is being applied. An effective amount is an amount that provides therapeutic improvement while minimizing side or adverse effect. Therapeutic improvement may be regression of the complement mediated or complement associated disease, improvement in life quality of the subject, improvement of the efficacy of a combined treatment. The effective amount may also be an amount that leads to inhibition of the complement cascade, as clinically observed.

One can administer the variant by any method of the art.

In particular, one can inject the variant. In another embodiment, one can apply the variant topically (either on the skin or on the eye of the patient) as disclosed in WO 2014/173899. In another embodiment, one can administer the variant orally, as disclosed in WO 2016/062874.

Other routes of administration are also contemplated. In some embodiments a variant may be administered intravenously or subcutaneously. In some embodiments a variant may be administered intraocularly (e.g., intravitreally) for treating an eye disorder. In some embodiments a variant may be administered by the intrathecal route (e.g., for treating disorders affecting the central nervous system).

The variants or polypeptides containing the variants can also be used in diagnostic methods. In particular, such variants or polypeptide may be linked to any marker known in the art and used in imagery methods.

The invention thus also relates to a method for detecting the presence of, or for quantifying, C3 and/or C3b in a sample, comprising the step of
a. Exposing the sample to a variant as disclosed that binds to C3 and/or C3b, in such conditions that such binding is possible
b. Recovering the variant and/or detecting, or measuring the amount of, the C3 and/or C3b bound to the variant.

The recovery of b) can be performed by various washes or methods common in the art. The detection or quantification can be performed by any method such as ELISA, chromatography, fluorescence or other methods in the art.

### Complement associated or mediated disease

A complement associated or complement mediated disease or condition relates to any disease or condition where increase of the complement system is observed and can lead to complement-mediated damage to an organ, tissue, or cells of the subject.

The polypeptide herein disclosed can be administered to the subject to inhibit the complement, alone or in combination with one or other product(s) that also inhibit the complement system or target other targets associated with the disease, or have an efficacy in the treatment or relief of the disease or condition.

One can cite
- protection of red blood cells (RBCs) in particular in paroxysmal nocturnal hemoglobinuria or atypical hemolytic uremic syndrome;
- protection of transplanted organs, tissues, and/or cells (generally in combination with other immunosuppressant drugs), in particular reduction of ischemia/reperfusion (I/R) injury

Paroxysmal nocturnal hemoglobinuria (PNH) is a rare disorder characterized by complement-mediated intravascular hemolysis, hemoglobinuria, bone marrow failure, and thrombophilia. Atypical hemolytic syndrome (aHUS) is a chronic disorder characterized by microangiopathic hemolytic anemia, thrombocytopenia, and acute renal failure and is caused by inappropriate complement activation, often due to mutations in genes encoding complement regulatory proteins. Complement-mediated hemolysis may also occur in a diverse group of other conditions including autoimmune hemolytic anemias, such as primary chronic cold agglutinin disease and certain reactions to drugs and other foreign substances.

Transplantation (graft) relates to replacement of organs and tissues, and blood transfusions are herein considered is considered a "graft". Ischemia-reperfusion (I/R) injury occurs when the organ has been subjected to lack of oxygenation between the harvest from the donor and the grafting in the host. The polypeptides herein disclosed have some utility to prevent I/R injury and rejection of the transplant. They can be used in the transplant medium, used during carriage of the transplanted organ from the donor to the host, or they can be administered to the host after transplantation.

Other Complement-Mediated or associated Disorders include eye disorders such as age-related macular degeneration (AMD), diabetic retinopathy, glaucoma, or uveitis, autoimmune diseases in particular when mediated at least in part by antibodies against one or more self antigens. One can cite rheumatoid arthritis, myasthenia gravis, neuromyelitis optica (NMO, Devic's disease), some glomerulopathies of the kidney.

Other diseases that can be treated by the polypeptides herein disclosed include intracerebral hemorrhages, neurodegenerative diseases, anaphylaxis or infusion reaction, asthma, rhinosinusitis, nasal polyposis, chronic obstructive pulmonary disease (COPD) or idiopathic pulmonary fibrosis. In some embodiments the disease is Alzheimer's disease, Parkinson's disease, Huntington's disease, amyotrophic lateral sclerosis progressive supranuclear palsy, Lewy body dementia (i.e., dementia with Lewy bodies or Parkinson's disease dementia), frontotemporal dementia, traumatic brain injury, traumatic spinal cord injury, multisystem atrophy, chronic traumatic encephalopathy, chronic inflammatory demyelinating polyneuropathy, Guillain-Barré syndrome, multifocal motor neuropathy, Creutzfeldt-Jakob disease, chronic pain (e.g., neuropathic pain) or leptomeningeal metastasis. Other diseases that can be treated by the polypeptides herein disclosed include chronic inflammatory diseases, which may be auto-immune diseases, such as psoriasis, atopic dermatitis; systemic scleroderma and sclerosis; inflammatory bowel disease (IBD) (such as Crohn's disease and ulcerative colitis); Behcet's Disease; dermatomyositis; polymyositis; multiple sclerosis (MS); dermatitis; meningitis; encephalitis; uveitis; osteoarthritis; lupus nephritis; rheumatoid arthritis (RA), Sjogren's syndrome, vasculitis; central nervous system (CNS) inflammatory disorders, chronic hepatitis; chronic pancreatitis, glomerulonephritis; sarcoidosis; thyroiditis, pathologic immune responses to tissue/organ transplantation (e.g., transplant rejection); COPD, asthma, bronchiolitis, hypersensitivity pneumonitis, idiopathic pulmonary fibrosis (IPF), periodontitis, and gingivitis.

One can also cite disorders that affect the musculoskeletal system, such as rheumatoid arthritis or psoriatic arthritis, juvenile chronic arthritis, spondyloarthropathies Reiter's syndrome, gout), or the circulatory system (vasculitis or other disorder associated with vessel inflammation, e.g., blood vessel and/or lymph vessel inflammation, polyarteritis nodosa, Wegener's granulomatosis, giant cell arteritis, Churg-Strauss syndrome, microscopic polyangiitis, Henoch-Schonlein purpura, Takayasu's arteritis, Kawasaki disease, Horton disease, or Behcet's disease).

In some embodiments the disorder is cancer.

### DESCRIPTION OF THE FIGURES

Figure 1: alignment of proteins of the Sac7d family.
Figure 2: verification of binding to C3b in selected variants binding to C3.
Figure 3: cluster repartition of binders to C3 and C3b, based on sequence similarity.
Figure 4: competition assay with compstatin for C3b binding of Nanofitins from different cluster.
Figure 5: EC50 in ELISA for C3b binding of various Nanofitins.
Figure 6: inhibition of complement cascade of various Nanofitins.

### Example 1. Identification of variants that bind to C3 and C3b

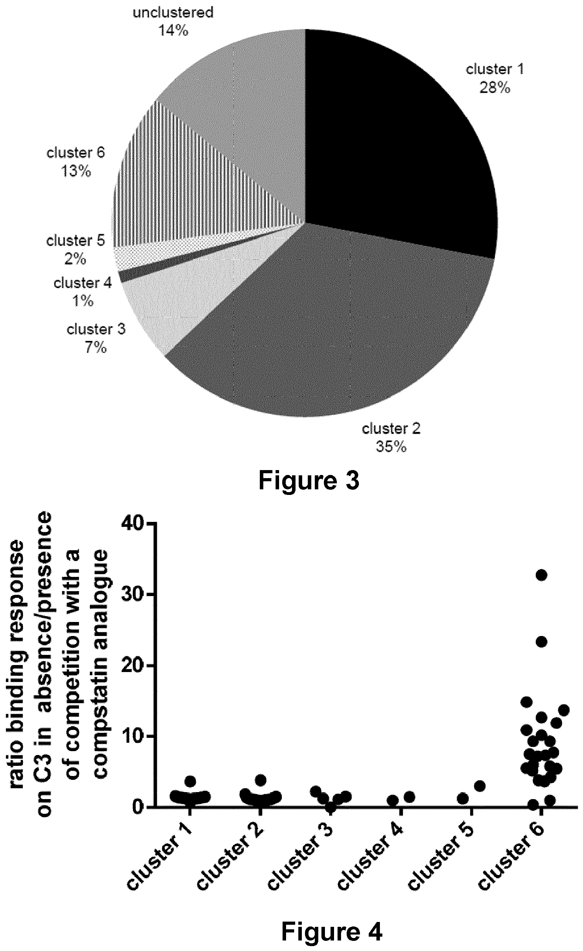

The conversion of C3 into C3b comes with significant structural changes. 190 variants binding to C3 and C3b were screened by ELISA as crude bacterial supernatant using immobilized C3b as a target, which made t possible to verify a high proportion of positive binders (Figure 2). Subsequent sequencing showed a strong sequence diversity, with very few repeated sequences. Furthermore, segregation of the Nanofitins into several clusters based on the homology of their binding site highlighted an enrichment for specific patterns with more than 50 % of the hits found in clusters 1 and 2 (Figure 3). Such variants, based on the Sac7d sequence, are called nanofitins.

### Example 2. Identification of Nanofitins competing with Compstatin

Compstatin is a 13-residue synthetic peptide that binds C3 at the macroglobulin (MG) domains 4 and 5. This binding site is part of the MG-ring formed by domains MG1-6, which is structurally conserved by C3 and its truncated variants C3b and C3c. Interestingly, this domain is far away from any other known binding site on C3 suggesting that Compstatin inhibitory activity involves a mechanism of action based on steric hindrance; hence blocking complement activation and amplification by restricting access of the substrate C3 to the convertase complexes.
Candidates targeting an epitope overlapping that of compstatin were identified *via* a competition assay, by biolayer interferometry, using Nanofitins found specific to C3b and representative of each sequence cluster (clusters 1 to 6). The ratio of the binding response on C3b to the one on C3b complexed by a compstatin analogue was used as a measure of the competing potential of the different Nanofitins. It was found that the binding response of the Nanofitins from the cluster 1 to 5 was not affected by the presence of the compstatin analogue (ratio ∼ 1), which suggests that they bind C3b on a different epitope than the compstatin.
Nanofitins from the cluster 6 showed a reduced binding response in presence of the compstatin analogue (ratio > 1), suggesting that the compstatin is masking their epitope (Figure 4).

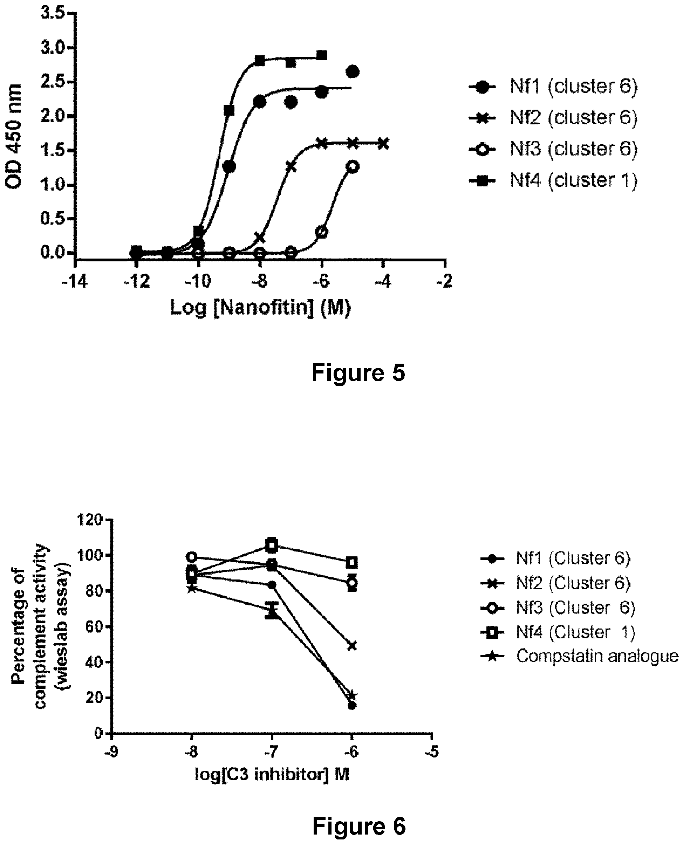

Further characterization included an evaluation of the dose response by ELISA and a screen of the complement cascade inhibition activity at four concentrations (1, 0.1 and 0.01 µM) in a Wieslab assay based on the classical pathway.
The three clones from the cluster 6 were found capable of inhibiting the complement cascade, with a neutralization potential that is correlated with their EC50 in ELISA. It was also noted that binding to C3b appears to be not sufficient to trigger an efficient inhibition of the complement cascade as Nf4 from the cluster 1 appears to be a weak inhibitor despite a low EC50 in ELISA. Taken together these data indicate that the clones from the cluster 6 show a functional compstatin-like mechanism of action by both targeting a similar epitope and providing similar neutralization activity. This is depicted in Figures 5 and 6.
One clone, named Nf1, was further investigated and characterized. The sequence of this clone falls under SEQ ID NO: 22.

### Example 3. Characterization /improvement of clone Nf1

Important residues involved in the specificity for C3 were identified.
The other residues were further modified to adjust the dissociation rate.
Mutants were generated, by substituting all the residues initially randomized in the naïve Nanofitin library by an alanine (except at position 8 which appears to be already an alanine in Nf1).
The binding capacity of these different variants was then evaluated in ELISA at 10 and 100 nM, respectively the EC100 and 10 fold the EC100 of Nf1.
It was found that the Y24 and W42 were the most important residues to maintain C3b binding with good affinity.
It was also found that positions 9, 31, and 44 were also important (to a lesser degree) to maintain good affinity C3b. Position 46 was found to be more permissive.
In comparison, the other alanine variants retained a binding capacity on C3b in ELISA at the two concentrations investigated, with various levels of impact on their binding response depending on the position involved. This result is suggesting that the primary residues of Nf1 contributing to the specificity for C3 are located at the positions 24 and 42, with some possible variations at positions 9, 31, and 44 and, also at position 46.
The other residues are quite tolerant to a substitution into an alanine suggesting a less important, or secondary, contribution to the specificity.

It was noted that the primary residues are located at the core of the binding site and the secondary ones at the periphery.

Optimization of the binders was performed, by determination of residues at positions 7, 21, 22, 26, 29, 33 and 40 that would improve affinity.
Two of the clones showing the highest ELISA signal (0D 450 nm > 4) were purified and further characterized for affinity measurement and neutralization potency in weislab assays based on the alternative and classical pathways. As a result of the randomization and subsequent off-rate selection, these 2 variants of Nf1 (SEQ ID NO: 25 and SEQ ID NO: 26) were found to exhibit a slower off rate and an overall higher affinity compared to Nf1 itself.
Binding kinetic parameters of the anti-C3 Nanofitins were measured by interferometry on Octet RED96 system (ForteBio). Streptavidin biosensors were first functionalized with biotinylated anti-GFP Nanofitins (10 µg/mL in TBS containing 0.002% Tween 20 and 0.01% BSA) at 2 nm and then loaded with anti-C3 GFP tagged Nanofitins (10 µg/mL in TBS containing 0.002% Tween 20 and 0.01% BSA) at 1.5 nm. Biosensors were allowed to equilibrate for 60 s and binding kinetic was then evaluated by exposing simultaneously biosensors to various concentrations (150, 50, 16.6, 5.55 and 1.85 and 0 nM) of C3b in TBS containing 0.002% Tween 20 and 0.01%. Association and dissociation steps were measured for 180 s each. Biosensors were regenerated using three cycles of alternating wash for 10 s in Glycine 10 mM pH 2.5 and in TBS. All the steps were run at 30°C with a continuous shake speed of 1000 RPM. The biosensor exposed to the 0 nM concentration was used as a background reference. Sensorgrams were processed using a single reference subtraction and analyzed using the Octet Data Analysis software 11.1 (ForteBio). Fitting was performed with a 1:1 binding fit model.
This gain in affinity (Table 19) translated in a higher neutralization potency compared to Nf1, as demonstrated in a weislab assay.

**Table 19. measure of affinity of C3-binders after optimization.**

| | KD (M) | ka (1/Ms) | kdis (1/s) | Full R^2 |
|---|---|---|---|---|
| Nf1 | 4.36E-08 | 1.04E+05 | 4.55E-03 | 0.9992 |
| B10 (SEQ ID NO: 25) | 7.31 E-09 | 5.92E+04 | 4.33E-04 | 0.9998 |
| H03 (SEQ ID NO: 26) | 2.89E-09 | 6.52E+04 | 1.88E-04 | 0.9996 |

### Example 4. The variants are tolerant to fusion at both N- and C-terminus ends

The paratope of Nanofitins and their N- and C-terminus extremities are located on opposite faces. As a consequence, Nanofitins can be engaged in fusion constructions without altering their target engagement, which could include the genetic fusion or conjugation to a peptide or a protein. This was demonstrated for the anti-C3 Nanofitin B10. B10 Nanofitin is retaining its functionality regardless of the presence of a fusion at its N- and C-terminus ends. This was demonstrated with different peptide composition and length as well as proteins (Table XXX). It is to be noted here that a similar property has been observed with the fusion to full length antibodies (brevet NF-Ab) or another Nanofitin (brevet multispe). Furthermore, Nanofitins can be expressed either recombinantly in a variety of expression hosts (prokaryotic or eukaryotic) with examples given for E. coli and CHO, or by full chemical synthesis. In all the cases explored with the B10, a fully functional Nanofitin is recovered as demonstrated by biolayer interferometry experiments.

**Table 20. measure of affinity of C3-binders with various tags in N- or C-terminus.**

| Nter tag | CterTag | Expression system | KD (nM) | Full R^2 |
|---|---|---|---|---|
| none | none | Recombinant, E. coli | 18.5 | 0.9976 |
| none | none | Full chemical synthesis | 18 | 0.9967 |
| peptide (10-mers) | none | Recombinant, E. coli | 8.2 | 0.9993 |
| peptide (10-mers) | 30 kDa protein | Recombinant, E. coli | 7.3 | 0.9998 |
| peptide (10-mers) | peptide (15-mers) | Recombinant, E. coli | 2.5 | 0.9991 |
| peptide (10-mers) | Nanofitin | Recombinant, E. coli | 11.5 | 0.9990 |
| 48 kDa protein | None | Recombinant, CHO | 6.7 | 0.9948 |

## Claims

1. A polypeptide comprising a variant of a member of the Sac7d family binding to C3 and/or to C3b, wherein the variant comprises from 4 to 20 mutated residues in the interface of binding of the member of the Sac7d family to its natural ligand, and wherein said variant comprises W24Y and R42W mutations with the numbering corresponding to the numbering of Sac7d residues as depicted in SEQ ID NO: 1.

2. The polypeptide of claim 1, further comprising K9T, S31L and A44Y mutations, with the numbering corresponding to the numbering of Sac7d residues as depicted in SEQ ID NO: 1.

3. The polypeptide of claim 1 or 2, further comprising at least one mutation selected from D16E, N37Q and M56L, with the numbering corresponding to the numbering of Sac7d residues as depicted in SEQ ID NO: 1.

4. The polypeptide of any one of claims 1 to 3, wherein the mutated residues in the interface of binding of the member of the Sac7d family to its natural ligand are selected from the group consisting of V2, K3, K5, K7, Y8, K9, G10, E14, T17, K21, K22, W24, V26, G27, K28, M29, S31, T33, D36, N37, G38, K39, T40, A44, S46, E47, K48, D49, A50 and P51 of Sac7d as depicted in SEQ ID NO: 1.

5. The polypeptide of any one of claim 1 to 4, wherein the member of the Sac7d family is selected from the group consisting of Sac7d from *Sulfolobus acidocaldarius,* Sac7e from *Sulfolobus acidocaldarius,* SSo7d from *Sulfolobus solfataricus,* Ssh7b from *Sulfolobus shibatae,* Ssh7a from *Sulfolobus shibatae,* DBP7 from *Sulfolobus tokodaii,* Sis7a from *Sulfolobus islandicus,* Mse7 from *Metallosphaera sedula,* Mcu7 from *Metallosphaera cuprina,* Aho7a from *Acidianus hospitalis,* Aho7b from *Acidianus hospitalis,* Aho7c from *Acidianus hospitalis* and Sto7 from *Sulfurisphaera tokodaii.*

6. The polypeptide of any one of claims 1 to 5, which comprises SEQ ID NO: 59, SEQ ID NO: 45 SEQ ID NO: 22, SEQ ID NO: 27, SEQ ID NO: 17, SEQ ID NO: 64, SEQ ID NO: 69, SEQ ID NO: 74, SEQ ID NO: 79, SEQ ID NO: 84, SEQ ID NO: 89, SEQ ID NO: 94 or SEQ ID NO: 99.

7. The polypeptide of any one of claims 1 to 6, which comprises SEQ ID NO: 87, SEQ ID NO: 88, SEQ ID NO: 82, SEQ ID NO: 83, SEQ ID NO: 77, SEQ ID NO: 78, SEQ ID NO: 102, SEQ ID NO: 103, SEQ ID NO: 97, SEQ ID NO: 98, SEQ ID NO: 92, SEQ ID NO: 93 or amino acids 1-54 of these sequences.

8. The polypeptide of any one of claims 1 to 7, which consists in the variant of a member of the Sac7d family binding to C3 and/or C3b.

9. The polypeptide of any one of claims 1 to 7, wherein the variant of a member of the Sac7d family binding to C3 and/or C3b which is conjugated to an organic molecule.

10. The polypeptide of any one of claims 1 to 7, wherein the variant of a member of the Sac7d family binding to C3 and/or C3b which is conjugated to another polypeptide, in particular another variant of a protein of the Sac7d family.

11. A nucleic acid molecule coding for the polypeptide of any one of claims 1 to 8, and 10.

12. A pharmaceutical composition comprising the polypeptide of any one of claims 1-10, or the nucleic acid of claim 11, and a pharmaceutically acceptable carrier.

13. The polypeptide of any one of claims 1 to 13 or the nucleic acid of claim 14 as a medicament.

14. The polypeptide of any one of claims 1 to 13, or the nucleic acid of claim 14, for use for treatment of a complement-mediated disorder.

15. The polypeptide for use of claim 14, wherein the complement-mediated disorder is selected from a disease **characterized by** complement-mediated damage to red blood cells, in particular paroxysmal nocturnal hemoglobinuria or atypical hemolytic uremic syndrome, an autoimmune disease, in particular multiple sclerosis, a disorder involving the kidney, in particular membranoproliferative glomerulonephritis, lupus nephritis, IgA nephropathy (IgAN), primary membranous nephropathy (primary MN), C3 glomerulopathy (C3G), or acute kidney injury, a disorder involving the central or peripheral nervous system or neuromuscular junction, in particular neuromyelitis optica, Guillain-Barré syndrome, multifocal motor neuropathy, or myasthenia gravis, a disorder involving the respiratory system, in particular pulmonary fibrosis, and a disorder involving the vascular system, in particular **characterized by** vasculitis.


SEQUENCE LISTING

<110>  AFFILOGIC

<120>  POLYPEPTIDE FOR COMPLEMENT INHIBITION

<130>  BRV 194 - EP PRIO

<160>  104

<170>  PatentIn version 3.5

<210>  1
<211>  66
<212>  PRT
<213>  Sulfolobus acidocaldarius

<400>  1

Met Val Lys Val Lys Phe Lys Tyr Lys Gly Glu Glu Lys Glu Val Asp
1               5                   10                  15

Thr Ser Lys Ile Lys Lys Val Trp Arg Val Gly Lys Met Val Ser Phe
            20                  25                  30

Thr Tyr Asp Asp Asn Gly Lys Thr Gly Arg Gly Ala Val Ser Glu Lys
        35                  40                  45

Asp Ala Pro Lys Glu Leu Leu Asp Met Leu Ala Arg Ala Glu Arg Glu
    50                  55                  60

Lys Lys
65

<210>  2
<211>  64
<212>  PRT
<213>  Sulfolobus solfataricus

<400>  2

Met Ala Thr Val Lys Phe Lys Tyr Lys Gly Glu Glu Lys Glu Val Asp
1               5                   10                  15

Ile Ser Lys Ile Lys Lys Val Trp Arg Val Gly Lys Met Ile Ser Phe
            20                  25                  30

Thr Tyr Asp Glu Gly Gly Gly Lys Thr Gly Arg Gly Ala Val Ser Glu
        35                  40                  45

Lys Asp Ala Pro Lys Glu Leu Leu Gln Met Leu Glu Lys Gln Lys Lys
    50                  55                  60

<210>  3

<211>  65
<212>  PRT
<213>  Sulfolobus acidocaldarius

<400>  3

Met Ala Lys Val Arg Phe Lys Tyr Lys Gly Glu Glu Lys Glu Val Asp
1               5                   10                  15

Thr Ser Lys Ile Lys Lys Val Trp Arg Val Gly Lys Met Val Ser Phe
            20                  25                  30

Thr Tyr Asp Asp Asn Gly Lys Thr Gly Arg Gly Ala Val Ser Glu Lys
        35                  40                  45

Asp Ala Pro Lys Glu Leu Met Asp Met Leu Ala Arg Ala Glu Lys Lys
    50                  55                  60

Lys
65

<210>  4
<211>  64
<212>  PRT
<213>  Sulfolobus shibatae

<400>  4

Met Val Thr Val Lys Phe Lys Tyr Lys Gly Glu Glu Lys Glu Val Asp
1               5                   10                  15

Thr Ser Lys Ile Lys Lys Val Trp Arg Val Gly Lys Met Ile Ser Phe
            20                  25                  30

Thr Tyr Asp Glu Gly Gly Gly Lys Thr Gly Arg Gly Ala Val Ser Glu
        35                  40                  45

Lys Asp Ala Pro Lys Glu Leu Leu Gln Met Leu Glu Lys Gln Lys Lys
    50                  55                  60

<210>  5
<211>  64
<212>  PRT
<213>  Sulfolobus shibatae

<400>  5

Met Ala Thr Val Lys Phe Lys Tyr Lys Gly Glu Glu Lys Gln Val Asp
1               5                   10                  15

Ile Ser Lys Ile Lys Lys Val Trp Arg Val Gly Lys Met Ile Ser Phe
            20                  25                  30

Thr Tyr Asp Glu Gly Gly Gly Lys Thr Gly Arg Gly Ala Val Ser Glu
        35                  40                  45

Lys Asp Ala Pro Lys Glu Leu Leu Gln Met Leu Glu Lys Gln Lys Lys
    50                  55                  60

<210>  6
<211>  64
<212>  PRT
<213>  Sulfolobus tokodaii

<400>  6

Met Val Thr Val Lys Phe Lys Tyr Lys Gly Glu Glu Lys Glu Val Asp
1               5                   10                  15

Ile Ser Lys Ile Lys Lys Val Trp Arg Val Gly Lys Met Ile Ser Phe
            20                  25                  30

Thr Tyr Asp Asp Asn Gly Lys Thr Gly Arg Gly Ala Val Ser Glu Lys
        35                  40                  45

Asp Ala Pro Lys Glu Leu Leu Gln Met Leu Glu Lys Ser Gly Lys Lys
    50                  55                  60

<210>  7
<211>  64
<212>  PRT
<213>  Sulfolobus islandicus

<400>  7

Met Val Thr Val Lys Phe Lys Tyr Lys Gly Glu Glu Lys Gln Val Asp
1               5                   10                  15

Thr Ser Lys Ile Lys Lys Val Trp Arg Val Gly Lys Met Ile Ser Phe
            20                  25                  30

Thr Tyr Asp Glu Gly Gly Gly Lys Thr Gly Arg Gly Ala Val Ser Glu
        35                  40                  45

Lys Asp Ala Pro Lys Glu Leu Leu Gln Met Leu Glu Lys Gln Lys Lys
    50                  55                  60

<210>  8
<211>  64
<212>  PRT
<213>  Sulfolobus islandicus

<400>  8

Met Thr Thr Val Lys Phe Lys Tyr Lys Gly Glu Glu Lys Gln Val Asp

1               5                   10                  15

Thr Ser Lys Ile Lys Lys Val Trp Arg Val Gly Lys Met Ile Ser Phe
            20                  25                  30

Thr Tyr Asp Glu Gly Gly Gly Lys Thr Gly Arg Gly Ala Val Ser Glu
        35                  40                  45

Lys Asp Ala Pro Lys Glu Leu Leu Gln Met Leu Glu Lys Gln Lys Lys
    50                  55                  60

<210>  9
<211>  64
<212>  PRT
<213>  Sulfolobus islandicus

<400>  9

Met Thr Thr Val Lys Phe Lys Tyr Lys Gly Glu Glu Lys Gln Val Asp
1               5                   10                  15

Ile Ser Lys Ile Lys Lys Val Trp Arg Val Gly Lys Met Ile Ser Phe
            20                  25                  30

Thr Tyr Asp Glu Gly Gly Gly Lys Thr Gly Arg Gly Ala Val Ser Glu
        35                  40                  45

Lys Asp Ala Pro Lys Glu Leu Leu Gln Met Leu Glu Lys Gln Lys Lys
    50                  55                  60

<210>  10
<211>  62
<212>  PRT
<213>  Metallosphaera sedula.

<400>  10

Met Ala Thr Lys Ile Lys Phe Lys Tyr Lys Gly Gln Asp Leu Glu Val
1               5                   10                  15

Asp Ile Ser Lys Val Lys Lys Val Trp Lys Val Gly Lys Met Val Ser
            20                  25                  30

Phe Thr Tyr Asp Asp Asn Gly Lys Thr Gly Arg Gly Ala Val Ser Glu
        35                  40                  45

Lys Asp Ala Pro Lys Glu Leu Leu Asn Met Ile Gly Lys Lys
    50                  55                  60

<210>  11
<211>  62

<212>  PRT
<213>  Metallosphaera cuprina

<400>  11

Met Ala Thr Lys Ile Lys Phe Lys Tyr Lys Gly Gln Asp Leu Glu Val
1               5                   10                  15

Asp Ile Ser Lys Val Lys Lys Val Trp Lys Val Gly Lys Met Val Ser
            20                  25                  30

Phe Thr Tyr Asp Asp Asn Gly Lys Thr Gly Arg Gly Ala Val Ser Glu
        35                  40                  45

Lys Asp Ala Pro Lys Glu Leu Leu Ser Met Ile Gly Lys Lys
    50                  55                  60

<210>  12
<211>  61
<212>  PRT
<213>  Acidianus hospitalis

<400>  12

Met Thr Thr Val Lys Phe Lys Tyr Lys Gly Glu Glu Lys Glu Val Asp
1               5                   10                  15

Ile Ser Lys Ile Lys Lys Val Trp Arg Val Gly Lys Met Ile Ser Phe
            20                  25                  30

Thr Tyr Asp Asp Asn Gly Lys Thr Gly Arg Gly Ala Val Ser Glu Lys
        35                  40                  45

Asp Ala Pro Lys Glu Leu Leu Glu Lys Leu Glu Lys Lys
    50                  55                  60

<210>  13
<211>  62
<212>  PRT
<213>  Acidianus hospitalis

<400>  13

Met Ala Thr Lys Val Lys Phe Lys Tyr Lys Gly Glu Glu Lys Glu Val
1               5                   10                  15

Asp Ile Ser Lys Ile Lys Lys Val Trp Arg Val Gly Lys Met Ile Ser
            20                  25                  30

Phe Thr Tyr Asp Asp Asn Gly Lys Thr Gly Arg Gly Ala Val Ser Glu
        35                  40                  45

Lys Asp Ala Pro Lys Glu Leu Leu Asp Lys Leu Glu Lys Lys
    50                  55                  60

<210>  14
<211>  60
<212>  PRT
<213>  Acidianus hospitalis

<400>  14

Met Ala Thr Lys Val Lys Phe Lys Tyr Lys Gly Glu Glu Lys Glu Val
1               5                   10                  15

Asp Ile Ser Lys Ile Lys Lys Val Trp Arg Val Gly Lys Met Ile Ser
            20                  25                  30

Phe Thr Tyr Asp Asp Asn Gly Lys Thr Gly Arg Gly Ala Val Ser Glu
        35                  40                  45

Lys Asp Ala Pro Lys Glu Leu Leu Glu Lys Leu Lys
    50                  55                  60

<210>  15
<211>  64
<212>  PRT
<213>  Sulfurisphaera tokodaii

<400>  15

Met Val Thr Val Lys Phe Lys Tyr Lys Gly Glu Glu Lys Glu Val Asp
1               5                   10                  15

Ile Ser Lys Ile Lys Lys Val Trp Arg Val Gly Lys Met Ile Ser Phe
            20                  25                  30

Thr Tyr Asp Asp Asn Gly Lys Thr Gly Arg Gly Ala Val Ser Glu Lys
        35                  40                  45

Asp Ala Pro Lys Glu Leu Leu Gln Met Leu Glu Lys Ser Gly Lys Lys
    50                  55                  60

<210>  16
<211>  68
<212>  PRT
<213>  Artificial Sequence

<220>
<223>  Consensus sequence

<220>
<221>  VARIANT
<222>  (2)..(2)
<223>  Xaa is V, A or T

<220>
<221>  VARIANT
<222>  (3)..(3)
<223>  Xaa is T or K

<220>
<221>  VARIANT
<222>  (4)..(4)
<223>  Xaa is - or K

<220>
<221>  VARIANT
<222>  (6)..(6)
<223>  Xaa is R or K

<220>
<221>  VARIANT
<222>  (15)..(15)
<223>  Xaa is E or Q

<220>
<221>  VARIANT
<222>  (18)..(18)
<223>  Xaa is T or I

<220>
<221>  VARIANT
<222>  (31)..(31)
<223>  Xaa is V or I

<220>
<221>  VARIANT
<222>  (37)..(39)
<223>  Xaa Xaa Xaa is EGG or DN-

<220>
<221>  VARIANT
<222>  (57)..(57)
<223>  Xaa is M or L

<220>
<221>  VARIANT
<222>  (58)..(58)
<223>  Xaa is Q, D or E

<220>
<221>  VARIANT
<222>  (59)..(59)
<223>  Xaa is M or K

<220>
<221>  VARIANT
<222>  (61)..(68)
<223>  XaaXaaXaaXaaXaaXaaXaaXaa is -------K, EKS--GKK, EK---QKK,
      ARAEREKK, ARAERE-K, ARAERE--, ARA-EKKK, E-----KK, EKS--GKK,
      ACAEREKK or ACA-EKKK

<400>  16

Met Xaa Xaa Xaa Val Xaa Phe Lys Tyr Lys Gly Glu Glu Lys Xaa Val
1               5                   10                  15

Asp Xaa Ser Lys Ile Lys Lys Val Trp Arg Val Gly Lys Met Xaa Ser
            20                  25                  30

Phe Thr Tyr Asp Xaa Xaa Xaa Gly Lys Thr Gly Arg Gly Ala Val Ser
        35                  40                  45

Glu Lys Asp Ala Pro Lys Glu Leu Xaa Xaa Xaa Leu Xaa Xaa Xaa Xaa
    50                  55                  60

Xaa Xaa Xaa Xaa
65

<210>  17
<211>  68
<212>  PRT
<213>  Artificial Sequence

<220>
<223>  Consensus sequence

<220>
<221>  VARIANT
<222>  (2)..(2)
<223>  Xaa is V, A or T

<220>
<221>  VARIANT
<222>  (3)..(3)
<223>  Xaa is T or K

<220>
<221>  VARIANT
<222>  (4)..(4)
<223>  Xaa is - or K

<220>
<221>  VARIANT
<222>  (6)..(6)
<223>  Xaa is R or K

<220>
<221>  VARIANT
<222>  (8)..(10)
<223>  Xaa is any amino acid

<220>
<221>  VARIANT
<222>  (15)..(15)
<223>  Xaa is E or Q

<220>
<221>  VARIANT
<222>  (18)..(18)
<223>  Xaa is T or I

<220>
<221>  VARIANT

<222>  (22)..(22)
<223>  Xaa is A, S, T, V, I, L, Y, or F

<220>
<221>  VARIANT
<222>  (23)..(23)
<223>  Xaa is any amino acid

<220>
<221>  VARIANT
<222>  (27)..(27)
<223>  Xaa is S, T, V, I, L, Y, or F

<220>
<221>  VARIANT
<222>  (30)..(30)
<223>  Xaa is any amino acid

<220>
<221>  VARIANT
<222>  (31)..(31)
<223>  Xaa is V or I

<220>
<221>  VARIANT
<222>  (32)..(32)
<223>  Xaa is L, I, V, Y, F, M or H

<220>
<221>  VARIANT
<222>  (34)..(34)
<223>  Xaa is S, T or A

<220>
<221>  VARIANT
<222>  (37)..(39)
<223>  Xaa Xaa Xaa is EGG or DN-

<220>
<221>  VARIANT
<222>  (42)..(42)
<223>  Xaa is T or I

<220>
<221>  VARIANT
<222>  (46)..(46)
<223>  Xaa is F, Y, I, V, L, M or H

<220>
<221>  VARIANT
<222>  (48)..(48)
<223>  Xaa is S, T, H or R

<220>
<221>  VARIANT
<222>  (57)..(57)
<223>  Xaa is M or L

<220>
<221>  VARIANT
<222>  (58)..(58)
<223>  Xaa is Q, D or E

<220>
<221>  VARIANT
<222>  (59)..(59)
<223>  Xaa is M or K

<220>
<221>  VARIANT
<222>  (61)..(68)
<223>  XaaXaaXaaXaaXaaXaaXaaXaa is -------K, EKS--GKK, EK---QKK,
      ARAEREKK, ARAERE-K, ARAERE--, ARA-EKKK, E-----KK, EKS--GKK,
      ACAEREKK or ACA-EKKK

<400>  17

Met Xaa Xaa Xaa Val Xaa Phe Xaa Xaa Xaa Gly Glu Glu Lys Xaa Val
1               5                   10                  15

Asp Xaa Ser Lys Ile Xaa Xaa Val Tyr Arg Xaa Gly Lys Xaa Xaa Xaa
            20                  25                  30

Phe Xaa Tyr Asp Xaa Xaa Xaa Gly Lys Xaa Gly Trp Gly Xaa Val Xaa
        35                  40                  45

Glu Lys Asp Ala Pro Lys Glu Leu Xaa Xaa Xaa Leu Xaa Xaa Xaa Xaa
    50                  55                  60

Xaa Xaa Xaa Xaa
65

<210>  18
<211>  68
<212>  PRT
<213>  Artificial Sequence

<220>
<223>  Consensus sequence

<220>
<221>  VARIANT
<222>  (2)..(2)
<223>  Xaa is V, A or T

<220>
<221>  VARIANT
<222>  (3)..(3)
<223>  Xaa is T or K

<220>
<221>  VARIANT
<222>  (4)..(4)
<223>  Xaa is - or K

<220>
<221>  VARIANT
<222>  (6)..(6)
<223>  Xaa is R or K

<220>
<221>  VARIANT
<222>  (8)..(9)
<223>  Xaa is any amino acid

<220>
<221>  VARIANT
<222>  (15)..(15)
<223>  Xaa is E or Q

<220>
<221>  VARIANT
<222>  (18)..(18)
<223>  Xaa is T or I

<220>
<221>  VARIANT
<222>  (22)..(22)
<223>  Xaa is A, S, T, V, I, L, Y, or F

<220>
<221>  VARIANT
<222>  (23)..(23)
<223>  Xaa is any amino acid

<220>
<221>  VARIANT
<222>  (27)..(27)
<223>  Xaa is S, T, V, I, L, Y, or F

<220>
<221>  VARIANT
<222>  (30)..(30)
<223>  Xaa is any amino acid

<220>
<221>  VARIANT
<222>  (31)..(31)
<223>  Xaa is V or I

<220>
<221>  VARIANT
<222>  (34)..(34)
<223>  Xaa is S, T or A

<220>
<221>  VARIANT
<222>  (37)..(39)
<223>  Xaa Xaa Xaa is EGG or DN-

<220>
<221>  VARIANT
<222>  (42)..(42)
<223>  Xaa is T or I

<220>
<221>  VARIANT
<222>  (48)..(48)
<223>  Xaa is S, T, H or R

<220>
<221>  VARIANT
<222>  (57)..(57)

<223>  Xaa is M or L

<220>
<221>  VARIANT
<222>  (58)..(58)
<223>  Xaa is Q, D or E

<220>
<221>  VARIANT
<222>  (59)..(59)
<223>  Xaa is M or K

<220>
<221>  VARIANT
<222>  (61)..(68)
<223>  XaaXaaXaaXaaXaaXaaXaaXaa is -------K, EKS--GKK, EK---QKK,
      ARAEREKK, ARAERE-K, ARAERE--, ARA-EKKK, E-----KK, EKS--GKK,
      ACAEREKK or ACA-EKKK

<400>  18

Met Xaa Xaa Xaa Val Xaa Phe Xaa Xaa Thr Gly Glu Glu Lys Xaa Val
1               5                   10                  15

Asp Xaa Ser Lys Ile Xaa Xaa Val Tyr Arg Xaa Gly Lys Xaa Xaa Leu
            20                  25                  30

Phe Xaa Tyr Asp Xaa Xaa Xaa Gly Lys Xaa Gly Trp Gly Tyr Val Xaa
        35                  40                  45

Glu Lys Asp Ala Pro Lys Glu Leu Xaa Xaa Xaa Leu Xaa Xaa Xaa Xaa
    50                  55                  60

Xaa Xaa Xaa Xaa
65

<210>  19
<211>  68
<212>  PRT
<213>  Artificial Sequence

<220>
<223>  Consensus sequence

<220>
<221>  VARIANT
<222>  (2)..(2)
<223>  Xaa is V, A or T

<220>
<221>  VARIANT
<222>  (3)..(3)
<223>  Xaa is T or K

<220>
<221>  VARIANT
<222>  (4)..(4)

<223>  Xaa is - or K

<220>
<221>  VARIANT
<222>  (6)..(6)
<223>  Xaa is R or K

<220>
<221>  VARIANT
<222>  (8)..(8)
<223>  Xaa is any amino acid

<220>
<221>  VARIANT
<222>  (15)..(15)
<223>  Xaa is E or Q

<220>
<221>  VARIANT
<222>  (18)..(18)
<223>  Xaa is T or I

<220>
<221>  VARIANT
<222>  (22)..(22)
<223>  Xaa is A, S, T, V, I, L, Y, or F

<220>
<221>  VARIANT
<222>  (23)..(23)
<223>  Xaa is any amino acid

<220>
<221>  VARIANT
<222>  (27)..(27)
<223>  Xaa is S, T, V, I, L, Y, or F

<220>
<221>  VARIANT
<222>  (31)..(31)
<223>  Xaa is V or I

<220>
<221>  VARIANT
<222>  (37)..(39)
<223>  Xaa Xaa Xaa is EGG or DN-

<220>
<221>  VARIANT
<222>  (57)..(57)
<223>  Xaa is M or L

<220>
<221>  VARIANT
<222>  (58)..(58)
<223>  Xaa is Q, D or E

<220>
<221>  VARIANT
<222>  (59)..(59)
<223>  Xaa is M or K

<220>

<221>  VARIANT
<222>  (61)..(68)
<223>  XaaXaaXaaXaaXaaXaaXaaXaa is -------K, EKS--GKK, EK---QKK,
       ARAEREKK, ARAERE-K, ARAERE--, ARA-EKKK, E-----KK, EKS--GKK,
       ACAEREKK or ACA-EKKK

<400>  19

Met Xaa Xaa Xaa Val Xaa Phe Xaa Ala Thr Gly Glu Glu Lys Xaa Val
1               5                   10                  15

Asp Xaa Ser Lys Ile Xaa Xaa Val Tyr Arg Xaa Gly Lys Asp Xaa Leu
            20                  25                  30

Phe Ser Tyr Asp Xaa Xaa Xaa Gly Lys Ile Gly Trp Gly Tyr Val Ser
        35                  40                  45

Glu Lys Asp Ala Pro Lys Glu Leu Xaa Xaa Xaa Leu Xaa Xaa Xaa Xaa
    50                  55                  60

Xaa Xaa Xaa Xaa
65

<210>  20
<211>  68
<212>  PRT
<213>  Artificial Sequence

<220>
<223>  Consensus sequence

<220>
<221>  VARIANT
<222>  (2)..(2)
<223>  Xaa is V, A or T

<220>
<221>  VARIANT
<222>  (3)..(3)
<223>  Xaa is T or K

<220>
<221>  VARIANT
<222>  (4)..(4)
<223>  Xaa is - or K

<220>
<221>  VARIANT
<222>  (6)..(6)
<223>  Xaa is R or K

<220>
<221>  VARIANT
<222>  (15)..(15)
<223>  Xaa is E or Q

<220>

<221>  VARIANT
<222>  (18)..(18)
<223>  Xaa is T or I

<220>
<221>  VARIANT
<222>  (31)..(31)
<223>  Xaa is V or I

<220>
<221>  VARIANT
<222>  (37)..(39)
<223>  Xaa Xaa Xaa is EGG or DN-

<220>
<221>  VARIANT
<222>  (57)..(57)
<223>  Xaa is M or L

<220>
<221>  VARIANT
<222>  (58)..(58)
<223>  Xaa is Q, D or E

<220>
<221>  VARIANT
<222>  (59)..(59)
<223>  Xaa is M or K

<220>
<221>  VARIANT
<222>  (61)..(68)
<223>  XaaXaaXaaXaaXaaXaaXaaXaa is -------K, EKS--GKK, EK---QKK,
      ARAEREKK, ARAERE-K, ARAERE--, ARA-EKKK, E-----KK, EKS--GKK,
      ACAEREKK or ACA-EKKK

<400>  20

Met Xaa Xaa Xaa Val Xaa Phe Asp Ala Thr Gly Glu Glu Lys Xaa Val
1               5                   10                  15

Asp Xaa Ser Lys Ile Ser Ala Val Tyr Arg Thr Gly Lys Asp Xaa Leu
            20                  25                  30

Phe Ser Tyr Asp Xaa Xaa Xaa Gly Lys Ile Gly Trp Gly Tyr Val Ser
        35                  40                  45

Glu Lys Asp Ala Pro Lys Glu Leu Xaa Xaa Xaa Leu Xaa Xaa Xaa Xaa
    50                  55                  60

Xaa Xaa Xaa Xaa
65

<210>  21
<211>  68
<212>  PRT
<213>  Artificial Sequence

<220>
<223>  Consensus sequence

<220>
<221>  VARIANT
<222>  (2)..(2)
<223>  Xaa is V, A or T

<220>
<221>  VARIANT
<222>  (3)..(3)
<223>  Xaa is T or K

<220>
<221>  VARIANT
<222>  (4)..(4)
<223>  Xaa is - or K

<220>
<221>  VARIANT
<222>  (6)..(6)
<223>  Xaa is R or K

<220>
<221>  VARIANT
<222>  (15)..(15)
<223>  Xaa is E or Q

<220>
<221>  VARIANT
<222>  (18)..(18)
<223>  Xaa is T or I

<220>
<221>  VARIANT
<222>  (31)..(31)
<223>  Xaa is V or I

<220>
<221>  VARIANT
<222>  (37)..(39)
<223>  Xaa Xaa Xaa is EGG or DN-

<220>
<221>  VARIANT
<222>  (57)..(57)
<223>  Xaa is M or L

<220>
<221>  VARIANT
<222>  (58)..(58)
<223>  Xaa is Q, D or E

<220>
<221>  VARIANT
<222>  (59)..(59)
<223>  Xaa is M or K

<220>
<221>  VARIANT
<222>  (61)..(68)
<223>  XaaXaaXaaXaaXaaXaaXaaXaa is -------K, EKS--GKK, EK---QKK,

ARAEREKK, ARAERE-K, ARAERE--, ARA-EKKK, E-----KK, EKS--GKK,
ACAEREKK or ACA-EKKK

<400> 21

Met Xaa Xaa Xaa Val Xaa Phe Ala Ala Thr Gly Glu Glu Lys Xaa Val
1               5                   10                  15

Asp Xaa Ser Lys Ile Ala Asn Val Tyr Arg Val Gly Lys Asp Xaa Leu
            20                  25                  30

Phe Ser Tyr Asp Xaa Xaa Xaa Gly Lys Ile Gly Trp Gly Tyr Val Ser
        35                  40                  45

Glu Lys Asp Ala Pro Lys Glu Leu Xaa Xaa Xaa Leu Xaa Xaa Xaa Xaa
    50                  55                  60

Xaa Xaa Xaa Xaa
65

<210> 22
<211> 64
<212> PRT
<213> Artificial Sequence

<220>
<223> Complement-binding Variant of Sac7d

<220>
<221> VARIANT
<222> (7)..(9)
<223> Xaa is any amino acid

<220>
<221> VARIANT
<222> (21)..(21)
<223> Xaa is A, S, T, V, I, L, Y, or F

<220>
<221> VARIANT
<222> (22)..(22)
<223> Xaa is any amino acid

<220>
<221> VARIANT
<222> (26)..(26)
<223> Xaa is S, T, V, I, L, Y, or F

<220>
<221> VARIANT
<222> (29)..(29)
<223> Xaa is any amino acid

<220>
<221> VARIANT
<222> (31)..(31)
<223> Xaa is L, I, V, Y, F, M or H

<220>
<221> VARIANT
<222> (33)..(33)
<223> Xaa is S, T or A

<220>
<221> VARIANT
<222> (40)..(40)
<223> Xaa is T or I

<220>
<221> VARIANT
<222> (44)..(44)
<223> Xaa is F, Y, I, V, L, M or H

<220>
<221> VARIANT
<222> (46)..(46)
<223> Xaa is S, T, H or R

<400> 22

Met Val Lys Val Lys Phe Xaa Xaa Xaa Gly Glu Glu Lys Glu Val Asp
1               5                   10                  15

Thr Ser Lys Ile Xaa Xaa Val Tyr Arg Xaa Gly Lys Xaa Val Xaa Phe
            20                  25                  30

Xaa Tyr Asp Asp Asn Gly Lys Xaa Gly Trp Gly Xaa Val Xaa Glu Lys
        35                  40                  45

Asp Ala Pro Lys Glu Leu Leu Asp Met Leu Ala Arg Ala Glu Arg Glu
    50                  55                  60

<210> 23
<211> 64
<212> PRT
<213> Artificial Sequence

<220>
<223> Complement-binding Variant of Sac7d

<220>
<221> VARIANT
<222> (7)..(8)
<223> Xaa is any amino acid

<220>
<221> VARIANT
<222> (21)..(21)
<223> Xaa is A, S, T, V, I, L, Y, or F

<220>
<221> VARIANT
<222> (22)..(22)
<223> Xaa is any amino acid

<220>
<221>  VARIANT
<222>  (26)..(26)
<223>  Xaa is S, T, V, I, L, Y, or F

<220>
<221>  VARIANT
<222>  (29)..(29)
<223>  Xaa is any amino acid

<220>
<221>  VARIANT
<222>  (33)..(33)
<223>  Xaa is S, T or A

<220>
<221>  VARIANT
<222>  (40)..(40)
<223>  Xaa is T or I

<220>
<221>  VARIANT
<222>  (46)..(46)
<223>  Xaa is S, T, H or R

<400>  23

Met Val Lys Val Lys Phe Xaa Xaa Thr Gly Glu Glu Lys Glu Val Asp
1               5                   10                  15

Thr Ser Lys Ile Xaa Xaa Val Tyr Arg Xaa Gly Lys Xaa Val Leu Phe
            20                  25                  30

Xaa Tyr Asp Asp Asn Gly Lys Xaa Gly Trp Gly Tyr Val Xaa Glu Lys
        35                  40                  45

Asp Ala Pro Lys Glu Leu Leu Asp Met Leu Ala Arg Ala Glu Arg Glu
    50                  55                  60

<210>  24
<211>  64
<212>  PRT
<213>  Artificial Sequence

<220>
<223>  Complement-binding Variant of Sac7d

<220>
<221>  VARIANT
<222>  (7)..(7)
<223>  Xaa is any amino acid

<220>
<221>  VARIANT
<222>  (21)..(21)
<223>  Xaa is A, S, T, V, I, L, Y, or F

<220>

<221>  VARIANT
<222>  (22)..(22)
<223>  Xaa is any amino acid

<220>
<221>  VARIANT
<222>  (26)..(26)
<223>  Xaa is S, T, V, I, L, Y, or F

<400>  24

Met Val Lys Val Lys Phe Xaa Ala Thr Gly Glu Glu Lys Glu Val Asp
1               5                   10                  15

Thr Ser Lys Ile Xaa Xaa Val Tyr Arg Xaa Gly Lys Asp Val Leu Phe
            20                  25                  30

Ser Tyr Asp Asp Asn Gly Lys Ile Gly Trp Gly Tyr Val Ser Glu Lys
        35                  40                  45

Asp Ala Pro Lys Glu Leu Leu Asp Met Leu Ala Arg Ala Glu Arg Glu
    50                  55                  60

<210>  25
<211>  64
<212>  PRT
<213>  Artificial Sequence

<220>
<223>  Complement-binding variant of Sac7d

<400>  25

Met Val Lys Val Lys Phe Asp Ala Thr Gly Glu Glu Lys Glu Val Asp
1               5                   10                  15

Thr Ser Lys Ile Ser Ala Val Tyr Arg Thr Gly Lys Asp Val Leu Phe
            20                  25                  30

Ser Tyr Asp Asp Asn Gly Lys Ile Gly Trp Gly Tyr Val Ser Glu Lys
        35                  40                  45

Asp Ala Pro Lys Glu Leu Leu Asp Leu Leu Ala Arg Ala Glu Arg Glu
    50                  55                  60

<210>  26
<211>  64
<212>  PRT
<213>  Artificial Sequence

<220>
<223>  Complement-binding variant of Sac7d

<400>  26

Met Val Lys Val Lys Phe Ala Ala Thr Gly Glu Glu Lys Glu Val Asp
1               5                   10                  15

Thr Ser Lys Ile Ala Asn Val Tyr Arg Val Gly Lys Asp Val Leu Phe
            20                  25                  30

Ser Tyr Asp Asp Asn Gly Lys Ile Gly Trp Gly Tyr Val Ser Glu Lys
        35                  40                  45

Asp Ala Pro Lys Glu Leu Leu Asp Leu Leu Ala Arg Ala Glu Arg Glu
    50                  55                  60

<210>  27
<211>  64
<212>  PRT
<213>  Artificial Sequence

<220>
<223>  Complement-binding Variant of Sso7d

<220>
<221>  VARIANT
<222>  (7)..(9)
<223>  Xaa is any amino acid

<220>
<221>  VARIANT
<222>  (21)..(21)
<223>  Xaa is A, S, T, V, I, L, Y, or F

<220>
<221>  VARIANT
<222>  (22)..(22)
<223>  Xaa is any amino acid

<220>
<221>  VARIANT
<222>  (26)..(26)
<223>  Xaa is S, T, V, I, L, Y, or F

<220>
<221>  VARIANT
<222>  (29)..(29)
<223>  Xaa is any amino acid

<220>
<221>  VARIANT
<222>  (31)..(31)
<223>  Xaa is L, I, V, Y, F, M or H

<220>
<221>  VARIANT
<222>  (33)..(33)
<223>  Xaa is S, T or A

<220>
<221>  VARIANT
<222>  (41)..(41)

<223>  Xaa is T or I

<220>
<221>  VARIANT
<222>  (45)..(45)
<223>  Xaa is F, Y, I, V, L, M or H

<220>
<221>  VARIANT
<222>  (47)..(47)
<223>  Xaa is S, T, H or R

<400>  27

Met Ala Thr Val Lys Phe Xaa Xaa Xaa Gly Glu Glu Lys Glu Val Asp
1               5                   10                  15

Ile Ser Lys Ile Xaa Xaa Val Tyr Arg Xaa Gly Lys Xaa Ile Xaa Phe
            20                  25                  30

Xaa Tyr Asp Glu Gly Gly Gly Lys Xaa Gly Trp Gly Xaa Val Xaa Glu
        35                  40                  45

Lys Asp Ala Pro Lys Glu Leu Leu Gln Leu Leu Glu Lys Gln Lys Lys
    50                  55                  60

<210>  28
<211>  64
<212>  PRT
<213>  Artificial Sequence

<220>
<223>  Complement-binding Variant of Sso7d

<220>
<221>  VARIANT
<222>  (7)..(7)
<223>  Xaa is any amino acid

<220>
<221>  VARIANT
<222>  (21)..(21)
<223>  Xaa is A, S, T, V, I, L, Y, or F

<220>
<221>  VARIANT
<222>  (22)..(22)
<223>  Xaa is any amino acid

<220>
<221>  VARIANT
<222>  (26)..(26)
<223>  Xaa is S, T, V, I, L, Y, or F

<400>  28

Met Ala Thr Val Lys Phe Xaa Ala Thr Gly Glu Glu Lys Glu Val Asp
1               5                   10                  15

Ile Ser Lys Ile Xaa Xaa Val Tyr Arg Xaa Gly Lys Asp Ile Leu Phe
            20                  25                  30

Ser Tyr Asp Glu Gly Gly Gly Lys Ile Gly Trp Gly Tyr Val Ser Glu
        35                  40                  45

Lys Asp Ala Pro Lys Glu Leu Leu Gln Leu Leu Glu Lys Gln Lys Lys
    50                  55                  60

<210>  29
<211>  64
<212>  PRT
<213>  Artificial Sequence

<220>
<223>  Complement-binding Variant of Sso7d

<400>  29

Met Ala Thr Val Lys Phe Asp Ala Thr Gly Glu Glu Lys Glu Val Asp
1               5                   10                  15

Ile Ser Lys Ile Ser Ala Val Tyr Arg Thr Gly Lys Asp Ile Leu Phe
            20                  25                  30

Ser Tyr Asp Glu Gly Gly Gly Lys Ile Gly Trp Gly Tyr Val Ser Glu
        35                  40                  45

Lys Asp Ala Pro Lys Glu Leu Leu Gln Leu Leu Glu Lys Gln Lys Lys
    50                  55                  60

<210>  30
<211>  64
<212>  PRT
<213>  Artificial Sequence

<220>
<223>  Complement-binding Variant of Sso7d

<400>  30

Met Ala Thr Val Lys Phe Ala Ala Thr Gly Glu Glu Lys Glu Val Asp
1               5                   10                  15

Ile Ser Lys Ile Ala Asn Val Tyr Arg Val Gly Lys Asp Ile Leu Phe
            20                  25                  30

Ser Tyr Asp Glu Gly Gly Gly Lys Ile Gly Trp Gly Tyr Val Ser Glu
        35                  40                  45

Lys Asp Ala Pro Lys Glu Leu Leu Gln Leu Leu Glu Lys Gln Lys Lys

50                  55                  60

<210>  31
<211>  68
<212>  PRT
<213>  Artificial Sequence

<220>
<223>  Consensus sequence


<220>
<221>  VARIANT
<222>  (2)..(2)
<223>  Xaa is V, A or T

<220>
<221>  VARIANT
<222>  (3)..(3)
<223>  Xaa is T or K

<220>
<221>  VARIANT
<222>  (4)..(4)
<223>  Xaa is - or K

<220>
<221>  VARIANT
<222>  (6)..(6)
<223>  Xaa is R or K

<220>
<221>  VARIANT
<222>  (8)..(10)
<223>  Xaa is any amino acid

<220>
<221>  VARIANT
<222>  (15)..(15)
<223>  Xaa is E or Q

<220>
<221>  VARIANT
<222>  (18)..(18)
<223>  Xaa is T or I

<220>
<221>  VARIANT
<222>  (22)..(22)
<223>  Xaa is A, S, T, V, I, L, Y, or F

<220>
<221>  VARIANT
<222>  (23)..(23)
<223>  Xaa is any amino acid

<220>
<221>  VARIANT
<222>  (27)..(27)

<220>
<221>  VARIANT

<222>  (30)..(30)
<223>  Xaa is any amino acid

<220>
<221>  VARIANT
<222>  (31)..(31)
<223>  Xaa is V or I

<220>
<221>  VARIANT
<222>  (32)..(32)
<223>  Xaa is L, I, V, Y, F, M or H

<220>
<221>  VARIANT
<222>  (34)..(34)
<223>  Xaa is S, T or A

<220>
<221>  VARIANT
<222>  (37)..(39)
<223>  Xaa Xaa Xaa is EGG or DN-

<220>
<221>  VARIANT
<222>  (42)..(42)
<223>  Xaa is T or I

<220>
<221>  VARIANT
<222>  (46)..(46)
<223>  Xaa is F, Y, I, V, L, M or H

<220>
<221>  VARIANT
<222>  (48)..(48)
<223>  Xaa is S, T, H or R

<220>
<221>  VARIANT
<222>  (57)..(57)
<223>  Xaa is M or L

<220>
<221>  VARIANT
<222>  (58)..(58)
<223>  Xaa is Q, D or E

<220>
<221>  VARIANT
<222>  (59)..(59)
<223>  Xaa is M or K

<220>
<221>  VARIANT
<222>  (61)..(68)
<223>  XaaXaaXaaXaaXaaXaaXaaXaa is -------K, EKS--GKK, EK---QKK,
       ARAEREKK, ARAERE-K, ARAERE--, ARA-EKKK, E-----KK, EKS--GKK,
       ACAEREKK or ACA-EKKK

<400>  31

Met Xaa Xaa Xaa Val Xaa Phe Xaa Xaa Xaa Gly Glu Glu Lys Xaa Val

1               5                   10                  15

Glu Xaa Ser Lys Ile Xaa Xaa Val Tyr Arg Xaa Gly Lys Xaa Xaa Xaa
            20                  25                  30

Phe Xaa Tyr Asp Xaa Xaa Xaa Gly Lys Xaa Gly Trp Gly Xaa Val Xaa
        35                  40                  45

Glu Lys Asp Ala Pro Lys Glu Leu Xaa Xaa Xaa Leu Xaa Xaa Xaa Xaa
    50                  55                  60

Xaa Xaa Xaa Xaa
65

<210>  32
<211>  68
<212>  PRT
<213>  Artificial Sequence

<220>
<223>  Consensus sequence

<220>
<221>  VARIANT
<222>  (2)..(2)
<223>  Xaa is V, A or T

<220>
<221>  VARIANT
<222>  (3)..(3)
<223>  Xaa is T or K

<220>
<221>  VARIANT
<222>  (4)..(4)
<223>  Xaa is - or K

<220>
<221>  VARIANT
<222>  (6)..(6)
<223>  Xaa is R or K

<220>
<221>  VARIANT
<222>  (8)..(9)

<220>
<221>  VARIANT
<222>  (15)..(15)
<223>  Xaa is E or Q

<220>
<221>  VARIANT
<222>  (18)..(18)
<223>  Xaa is T or I

<220>

<221>  VARIANT
<222>  (22)..(22)
<223>  Xaa is A, S, T, V, I, L, Y, or F

<220>
<221>  VARIANT
<222>  (23)..(23)
<223>  Xaa is any amino acid

<220>
<221>  VARIANT
<222>  (27)..(27)
<223>  Xaa is S, T, V, I, L, Y, or F

<220>
<221>  VARIANT
<222>  (30)..(30)

<220>
<221>  VARIANT
<222>  (31)..(31)
<223>  Xaa is V or I

<220>
<221>  VARIANT
<222>  (34)..(34)
<223>  Xaa is S, T or A

<220>
<221>  VARIANT
<222>  (37)..(39)
<223>  Xaa Xaa Xaa is EGG or DN-

<220>
<221>  VARIANT
<222>  (42)..(42)
<223>  Xaa is T or I

<220>
<221>  VARIANT
<222>  (48)..(48)
<223>  Xaa is S, T, H or R

<220>
<221>  VARIANT
<222>  (57)..(57)
<223>  Xaa is M or L

<220>
<221>  VARIANT
<222>  (58)..(58)
<223>  Xaa is Q, D or E

<220>
<221>  VARIANT
<222>  (59)..(59)
<223>  Xaa is M or K

<220>
<221>  VARIANT
<222>  (61)..(68)
<223>  XaaXaaXaaXaaXaaXaaXaaXaa is -------K, EKS--GKK, EK---QKK,
      ARAEREKK, ARAERE-K, ARAERE--, ARA-EKKK, E-----KK, EKS--GKK,

ACAEREKK or ACA-EKKK

<400> 32

Met Xaa Xaa Xaa Val Xaa Phe Xaa Xaa Thr Gly Glu Glu Lys Xaa Val
1               5                   10                  15

Glu Xaa Ser Lys Ile Xaa Xaa Val Tyr Arg Xaa Gly Lys Xaa Xaa Leu
            20                  25                  30

Phe Xaa Tyr Asp Xaa Xaa Xaa Gly Lys Xaa Gly Trp Gly Tyr Val Xaa
        35                  40                  45

Glu Lys Asp Ala Pro Lys Glu Leu Xaa Xaa Xaa Leu Xaa Xaa Xaa Xaa
    50                  55                  60

Xaa Xaa Xaa Xaa
65

<210> 33
<211> 68
<212> PRT
<213> Artificial Sequence

<220>
<223> Consensus sequence

<220>
<221> VARIANT
<222> (2)..(2)
<223> Xaa is V, A or T

<220>
<221> VARIANT
<222> (3)..(3)
<223> Xaa is T or K

<220>
<221> VARIANT
<222> (4)..(4)
<223> Xaa is - or K

<220>
<221> VARIANT
<222> (6)..(6)
<223> Xaa is R or K

<220>
<221> VARIANT
<222> (8)..(8)

<220>
<221> VARIANT
<222> (15)..(15)
<223> Xaa is E or Q

<220>

<221>  VARIANT
<222>  (18)..(18)
<223>  Xaa is T or I

<220>
<221>  VARIANT
<222>  (22)..(22)
<223>  Xaa is A, S, T, V, I, L, Y, or F

<220>
<221>  VARIANT
<222>  (23)..(23)
<223>  Xaa is any amino acid

<220>
<221>  VARIANT
<222>  (27)..(27)
<223>  Xaa is S, T, V, I, L, Y, or F

<220>
<221>  VARIANT
<222>  (31)..(31)
<223>  Xaa is V or I

<220>
<221>  VARIANT
<222>  (37)..(39)
<223>  Xaa Xaa Xaa is EGG or DN-

<220>
<221>  VARIANT
<222>  (57)..(57)
<223>  Xaa is M or L

<220>
<221>  VARIANT
<222>  (58)..(58)
<223>  Xaa is Q, D or E

<220>
<221>  VARIANT
<222>  (59)..(59)
<223>  Xaa is M or K

<220>
<221>  VARIANT
<222>  (61)..(68)
<223>  XaaXaaXaaXaaXaaXaaXaaXaa is -------K, EKS--GKK, EK---QKK,
      ARAEREKK, ARAERE-K, ARAERE--, ARA-EKKK, E-----KK, EKS--GKK,
      ACAEREKK or ACA-EKKK

<400>  33

Met Xaa Xaa Xaa Val Xaa Phe Xaa Ala Thr Gly Glu Glu Lys Xaa Val
1               5                   10                  15

Glu Xaa Ser Lys Ile Xaa Xaa Val Tyr Arg Xaa Gly Lys Asp Xaa Leu
            20                  25                  30

Phe Ser Tyr Asp Xaa Xaa Xaa Gly Lys Ile Gly Trp Gly Tyr Val Ser
        35                  40                  45

Glu Lys Asp Ala Pro Lys Glu Leu Xaa Xaa Xaa Leu Xaa Xaa Xaa Xaa
    50                  55                  60

Xaa Xaa Xaa Xaa
65

<210>  34
<211>  68
<212>  PRT
<213>  Artificial Sequence

<220>
<223>  Consensus sequence

<220>
<221>  VARIANT
<222>  (2)..(2)
<223>  Xaa is V, A or T

<220>
<221>  VARIANT
<222>  (3)..(3)
<223>  Xaa is T or K

<220>
<221>  VARIANT
<222>  (4)..(4)
<223>  Xaa is - or K

<220>
<221>  VARIANT
<222>  (6)..(6)
<223>  Xaa is R or K

<220>
<221>  VARIANT
<222>  (15)..(15)
<223>  Xaa is E or Q

<220>
<221>  VARIANT
<222>  (18)..(18)
<223>  Xaa is T or I

<220>
<221>  VARIANT
<222>  (31)..(31)
<223>  Xaa is V or I

<220>
<221>  VARIANT
<222>  (37)..(39)
<223>  Xaa Xaa Xaa is EGG or DN-

<220>
<221>  VARIANT
<222>  (57)..(57)
<223>  Xaa is M or L

<220>
<221>  VARIANT
<222>  (58)..(58)
<223>  Xaa is Q, D or E

<220>
<221>  VARIANT
<222>  (59)..(59)
<223>  Xaa is M or K

<220>
<221>  VARIANT
<222>  (61)..(68)
<223>  XaaXaaXaaXaaXaaXaaXaaXaa is -------K, EKS--GKK, EK---QKK,
      ARAEREKK, ARAERE-K, ARAERE--, ARA-EKKK, E-----KK, EKS--GKK,
      ACAEREKK or ACA-EKKK

<400>  34

Met Xaa Xaa Xaa Val Xaa Phe Asp Ala Thr Gly Glu Glu Lys Xaa Val
1               5                   10                  15

Glu Xaa Ser Lys Ile Ser Ala Val Tyr Arg Thr Gly Lys Asp Xaa Leu
            20                  25                  30

Phe Ser Tyr Asp Xaa Xaa Xaa Gly Lys Ile Gly Trp Gly Tyr Val Ser
        35                  40                  45

Glu Lys Asp Ala Pro Lys Glu Leu Xaa Xaa Xaa Leu Xaa Xaa Xaa Xaa
    50                  55                  60

Xaa Xaa Xaa Xaa
65

<210>  35
<211>  68
<212>  PRT
<213>  Artificial Sequence

<220>
<223>  Consensus sequence

<220>
<221>  VARIANT
<222>  (2)..(2)
<223>  Xaa is V, A or T

<220>
<221>  VARIANT
<222>  (3)..(3)
<223>  Xaa is T or K

<220>
<221>  VARIANT
<222>  (4)..(4)
<223>  Xaa is - or K

<220>
<221>  VARIANT
<222>  (6)..(6)
<223>  Xaa is R or K

<220>
<221>  VARIANT
<222>  (15)..(15)
<223>  Xaa is E or Q

<220>
<221>  VARIANT
<222>  (18)..(18)
<223>  Xaa is T or I

<220>
<221>  VARIANT
<222>  (31)..(31)
<223>  Xaa is V or I

<220>
<221>  VARIANT
<222>  (37)..(39)
<223>  Xaa Xaa Xaa is EGG or DN-

<220>
<221>  VARIANT
<222>  (57)..(57)
<223>  Xaa is M or L

<220>
<221>  VARIANT
<222>  (58)..(58)
<223>  Xaa is Q, D or E

<220>
<221>  VARIANT
<222>  (59)..(59)
<223>  Xaa is M or K

<220>
<221>  VARIANT
<222>  (61)..(68)
<223>  XaaXaaXaaXaaXaaXaaXaaXaa is -------K, EKS--GKK, EK---QKK,
      ARAEREKK, ARAERE-K, ARAERE--, ARA-EKKK, E-----KK, EKS--GKK,
      ACAEREKK or ACA-EKKK

<400>  35

Met Xaa Xaa Xaa Val Xaa Phe Ala Ala Thr Gly Glu Glu Lys Xaa Val
1               5                   10                  15

Glu Xaa Ser Lys Ile Ala Asn Val Tyr Arg Val Gly Lys Asp Xaa Leu
            20                  25                  30

Phe Ser Tyr Asp Xaa Xaa Xaa Gly Lys Ile Gly Trp Gly Tyr Val Ser
        35                  40                  45

Glu Lys Asp Ala Pro Lys Glu Leu Xaa Xaa Xaa Leu Xaa Xaa Xaa Xaa

50                  55                  60

Xaa Xaa Xaa Xaa
65

<210>  36
<211>  64
<212>  PRT
<213>  Artificial Sequence

<220>
<223>  Complement-binding Variant of Sac7d

<220>
<221>  VARIANT
<222>  (7)..(9)
<223>  Xaa is any amino acid

<220>
<221>  VARIANT
<222>  (21)..(21)
<223>  Xaa is A, S, T, V, I, L, Y, or F

<220>
<221>  VARIANT
<222>  (22)..(22)
<223>  Xaa is any amino acid

<220>
<221>  VARIANT
<222>  (26)..(26)
<223>  Xaa is S, T, V, I, L, Y, or F

<220>
<221>  VARIANT
<222>  (29)..(29)
<223>  Xaa is any amino acid

<220>
<221>  VARIANT
<222>  (31)..(31)
<223>  Xaa is L, I, V, Y, F, M or H

<220>
<221>  VARIANT
<222>  (33)..(33)
<223>  Xaa is S, T or A

<220>
<221>  VARIANT
<222>  (40)..(40)
<223>  Xaa is T or I

<220>
<221>  VARIANT
<222>  (44)..(44)
<223>  Xaa is F, Y, I, V, L, M or H

<220>
<221>  VARIANT

<222>  (46)..(46)
<223>  Xaa is S, T, H or R

<400>  36

Met Val Lys Val Lys Phe Xaa Xaa Xaa Gly Glu Glu Lys Glu Val Glu
1               5                   10                  15

Thr Ser Lys Ile Xaa Xaa Val Tyr Arg Xaa Gly Lys Xaa Val Xaa Phe
            20                  25                  30

Xaa Tyr Asp Asp Gln Gly Lys Xaa Gly Trp Gly Xaa Val Xaa Glu Lys
        35                  40                  45

Asp Ala Pro Lys Glu Leu Leu Asp Met Leu Ala Arg Ala Glu Arg Glu
    50                  55                  60

<210>  37
<211>  64
<212>  PRT
<213>  Artificial Sequence

<220>
<223>  Complement-binding Variant of Sac7d

<220>
<221>  VARIANT
<222>  (7)..(8)
<223>  Xaa is any amino acid

<220>
<221>  VARIANT
<222>  (21)..(21)
<223>  Xaa is A, S, T, V, I, L, Y, or F

<220>
<221>  VARIANT
<222>  (22)..(22)
<223>  Xaa is any amino acid

<220>
<221>  VARIANT
<222>  (26)..(26)
<223>  Xaa is S, T, V, I, L, Y, or F

<220>
<221>  VARIANT
<222>  (29)..(29)
<223>  Xaa is any amino acid

<220>
<221>  VARIANT
<222>  (33)..(33)
<223>  Xaa is S, T or A

<220>
<221>  VARIANT
<222>  (40)..(40)

<223>  Xaa is T or I

<220>
<221>  VARIANT
<222>  (46)..(46)
<223>  Xaa is S, T, H or R

<400>  37

Met Val Lys Val Lys Phe Xaa Xaa Thr Gly Glu Glu Lys Glu Val Glu
1               5                   10                  15

Thr Ser Lys Ile Xaa Xaa Val Tyr Arg Xaa Gly Lys Xaa Val Leu Phe
            20                  25                  30

Xaa Tyr Asp Asp Gln Gly Lys Xaa Gly Trp Gly Tyr Val Xaa Glu Lys
        35                  40                  45

Asp Ala Pro Lys Glu Leu Leu Asp Met Leu Ala Arg Ala Glu Arg Glu
    50                  55                  60

<210>  38
<211>  64
<212>  PRT
<213>  Artificial Sequence

<220>
<223>  Complement-binding Variant of Sac7d

<220>
<221>  VARIANT
<222>  (7)..(7)
<223>  Xaa is any amino acid

<220>
<221>  VARIANT
<222>  (21)..(21)
<223>  Xaa is A, S, T, V, I, L, Y, or F

<220>
<221>  VARIANT
<222>  (22)..(22)
<223>  Xaa is any amino acid

<220>
<221>  VARIANT
<222>  (26)..(26)
<223>  Xaa is S, T, V, I, L, Y, or F

<400>  38

Met Val Lys Val Lys Phe Xaa Ala Thr Gly Glu Glu Lys Glu Val Glu
1               5                   10                  15

Thr Ser Lys Ile Xaa Xaa Val Tyr Arg Xaa Gly Lys Asp Val Leu Phe
            20                  25                  30

Ser Tyr Asp Asp Gln Gly Lys Ile Gly Trp Gly Tyr Val Ser Glu Lys
        35                  40                  45

Asp Ala Pro Lys Glu Leu Leu Asp Met Leu Ala Arg Ala Glu Arg Glu
    50                  55                  60

<210>  39
<211>  64
<212>  PRT
<213>  Artificial Sequence

<220>
<223>  Complement-binding variant of Sac7d

<400>  39

Met Val Lys Val Lys Phe Asp Ala Thr Gly Glu Glu Lys Glu Val Glu
1               5                   10                  15

Thr Ser Lys Ile Ser Ala Val Tyr Arg Thr Gly Lys Asp Val Leu Phe
            20                  25                  30

Ser Tyr Asp Asp Gln Gly Lys Ile Gly Trp Gly Tyr Val Ser Glu Lys
        35                  40                  45

Asp Ala Pro Lys Glu Leu Leu Asp Leu Leu Ala Arg Ala Glu Arg Glu
    50                  55                  60

<210>  40
<211>  64
<212>  PRT
<213>  Artificial Sequence

<220>
<223>  Complement-binding variant of Sac7d

<400>  40

Met Val Lys Val Lys Phe Ala Ala Thr Gly Glu Glu Lys Glu Val Glu
1               5                   10                  15

Thr Ser Lys Ile Ala Asn Val Tyr Arg Val Gly Lys Asp Val Leu Phe
            20                  25                  30

Ser Tyr Asp Asp Gln Gly Lys Ile Gly Trp Gly Tyr Val Ser Glu Lys
        35                  40                  45

Asp Ala Pro Lys Glu Leu Leu Asp Leu Leu Ala Arg Ala Glu Arg Glu
    50                  55                  60

<210>  41
<211>  64

<212>  PRT
<213>  Artificial Sequence

<220>
<223>  Complement-binding Variant of Sso7d


<220>
<221>  VARIANT
<222>  (7)..(9)
<223>  Xaa is any amino acid

<220>
<221>  VARIANT
<222>  (21)..(21)
<223>  Xaa is A, S, T, V, I, L, Y, or F

<220>
<221>  VARIANT
<222>  (22)..(22)
<223>  Xaa is any amino acid

<220>
<221>  VARIANT
<222>  (26)..(26)
<223>  Xaa is S, T, V, I, L, Y, or F

<220>
<221>  VARIANT
<222>  (29)..(29)
<223>  Xaa is any amino acid

<220>
<221>  VARIANT
<222>  (31)..(31)
<223>  Xaa is L, I, V, Y, F, M or H

<220>
<221>  VARIANT
<222>  (33)..(33)
<223>  Xaa is S, T or A

<220>
<221>  VARIANT
<222>  (41)..(41)
<223>  Xaa is T or I

<220>
<221>  VARIANT
<222>  (45)..(45)
<223>  Xaa is F, Y, I, V, L, M or H

<220>
<221>  VARIANT
<222>  (47)..(47)
<223>  Xaa is S, T, H or R

<400>  41

Met Ala Thr Val Lys Phe Xaa Xaa Xaa Gly Glu Glu Lys Glu Val Glu
1               5                   10                  15

Ile Ser Lys Ile Xaa Xaa Val Tyr Arg Xaa Gly Lys Xaa Ile Xaa Phe
            20                  25                  30

Xaa Tyr Asp Glu Gly Gly Gly Lys Xaa Gly Trp Gly Xaa Val Xaa Glu
        35                  40                  45

Lys Asp Ala Pro Lys Glu Leu Leu Gln Leu Leu Glu Lys Gln Lys Lys
    50                  55                  60

<210>  42
<211>  64
<212>  PRT
<213>  Artificial Sequence

<220>
<223>  Complement-binding Variant of Sso7d

<220>
<221>  VARIANT
<222>  (7)..(7)
<223>  Xaa is any amino acid

<220>
<221>  VARIANT
<222>  (21)..(21)
<223>  Xaa is A, S, T, V, I, L, Y, or F

<220>
<221>  VARIANT
<222>  (22)..(22)
<223>  Xaa is any amino acid

<220>
<221>  VARIANT
<222>  (26)..(26)
<223>  Xaa is S, T, V, I, L, Y, or F

<400>  42

Met Ala Thr Val Lys Phe Xaa Ala Thr Gly Glu Glu Lys Glu Val Glu
1               5                   10                  15

Ile Ser Lys Ile Xaa Xaa Val Tyr Arg Xaa Gly Lys Asp Ile Leu Phe
            20                  25                  30

Ser Tyr Asp Glu Gly Gly Gly Lys Ile Gly Trp Gly Tyr Val Ser Glu
        35                  40                  45

Lys Asp Ala Pro Lys Glu Leu Leu Gln Leu Leu Glu Lys Gln Lys Lys
    50                  55                  60

<210>  43
<211>  64
<212>  PRT
<213>  Artificial Sequence

<220>
<223>  Complement-binding Variant of Sso7d

<400>  43

Met Ala Thr Val Lys Phe Asp Ala Thr Gly Glu Glu Lys Glu Val Glu
1               5                   10                  15

Ile Ser Lys Ile Ser Ala Val Tyr Arg Thr Gly Lys Asp Ile Leu Phe
            20                  25                  30

Ser Tyr Asp Glu Gly Gly Gly Lys Ile Gly Trp Gly Tyr Val Ser Glu
        35                  40                  45

Lys Asp Ala Pro Lys Glu Leu Leu Gln Leu Leu Glu Lys Gln Lys Lys
    50                  55                  60

<210>  44
<211>  64
<212>  PRT
<213>  Artificial Sequence

<220>
<223>  Complement-binding Variant of Sso7d

<400>  44

Met Ala Thr Val Lys Phe Ala Ala Thr Gly Glu Glu Lys Glu Val Glu
1               5                   10                  15

Ile Ser Lys Ile Ala Asn Val Tyr Arg Val Gly Lys Asp Ile Leu Phe
            20                  25                  30

Ser Tyr Asp Glu Gly Gly Gly Lys Ile Gly Trp Gly Tyr Val Ser Glu
        35                  40                  45

Lys Asp Ala Pro Lys Glu Leu Leu Gln Leu Leu Glu Lys Gln Lys Lys
    50                  55                  60

<210>  45
<211>  68
<212>  PRT
<213>  Artificial Sequence

<220>
<223>  Consensus sequence

<220>
<221>  VARIANT
<222>  (2)..(2)
<223>  Xaa is V, A or T

<220>

<221>  VARIANT
<222>  (3)..(3)
<223>  Xaa is T or K

<220>
<221>  VARIANT
<222>  (4)..(4)
<223>  Xaa is - or K

<220>
<221>  VARIANT
<222>  (6)..(6)
<223>  Xaa is R or K

<220>
<221>  VARIANT
<222>  (8)..(10)

<220>
<221>  VARIANT
<222>  (15)..(15)
<223>  Xaa is E or Q

<220>
<221>  VARIANT
<222>  (17)..(17)
<223>  Xaa is E or D

<220>
<221>  VARIANT
<222>  (18)..(18)
<223>  Xaa is T or I

<220>
<221>  VARIANT
<222>  (22)..(22)
<223>  Xaa is A, S, T, V, I, L, Y, or F

<220>
<221>  VARIANT
<222>  (23)..(23)
<223>  Xaa is any amino acid

<220>
<221>  VARIANT
<222>  (27)..(27)
<223>  Xaa is S, T, V, I, L, Y, or F

<220>
<221>  VARIANT
<222>  (30)..(30)

<220>
<221>  VARIANT
<222>  (31)..(31)
<223>  Xaa is V or I

<220>
<221>  VARIANT
<222>  (32)..(32)
<223>  Xaa is L, I, V, Y, F, M or H

<220>

<221>  VARIANT
<222>  (34)..(34)
<223>  Xaa is S, T or A

<220>
<221>  VARIANT
<222>  (37)..(39)
<223>  Xaa Xaa Xaa is EGG or DN-

<220>
<221>  VARIANT
<222>  (42)..(42)
<223>  Xaa is T or I

<220>
<221>  VARIANT
<222>  (46)..(46)
<223>  Xaa is F, Y, I, V, L, M or H

<220>
<221>  VARIANT
<222>  (48)..(48)
<223>  Xaa is S, T, H or R

<220>
<221>  VARIANT
<222>  (57)..(57)
<223>  Xaa is M or L

<220>
<221>  VARIANT
<222>  (58)..(58)
<223>  Xaa is Q, D or E

<220>
<221>  VARIANT
<222>  (59)..(59)
<223>  Xaa is M or K

<220>
<221>  VARIANT
<222>  (61)..(68)
<223>  XaaXaaXaaXaaXaaXaaXaaXaa is -------K, EKS--GKK, EK---QKK,
      ARAEREKK, ARAERE-K, ARAERE--, ARA-EKKK, E-----KK, EKS--GKK,
      ACAEREKK or ACA-EKKK

<400>  45

Met Xaa Xaa Xaa Val Xaa Phe Xaa Xaa Xaa Gly Glu Glu Lys Xaa Val
1               5                   10                  15

Xaa Xaa Ser Lys Ile Xaa Xaa Val Tyr Arg Xaa Gly Lys Xaa Xaa Xaa
            20                  25                  30

Phe Xaa Tyr Asp Xaa Xaa Xaa Gly Lys Xaa Gly Trp Gly Xaa Val Xaa
        35                  40                  45

Glu Lys Asp Ala Pro Lys Glu Leu Xaa Xaa Xaa Leu Xaa Xaa Xaa Xaa
    50                  55                  60

Xaa Xaa Xaa Xaa
65

<210> 46
<211> 68
<212> PRT
<213> Artificial Sequence

<220>
<223> Consensus sequence

<220>
<221> VARIANT
<222> (2)..(2)
<223> Xaa is V, A or T

<220>
<221> VARIANT
<222> (3)..(3)
<223> Xaa is T or K

<220>
<221> VARIANT
<222> (4)..(4)
<223> Xaa is - or K

<220>
<221> VARIANT
<222> (6)..(6)
<223> Xaa is R or K

<220>
<221> VARIANT
<222> (8)..(9)

<220>
<221> VARIANT
<222> (15)..(15)
<223> Xaa is E or Q

<220>
<221> VARIANT
<222> (17)..(17)
<223> Xaa is E or D

<220>
<221> VARIANT
<222> (18)..(18)
<223> Xaa is T or I

<220>
<221> VARIANT
<222> (22)..(22)
<223> Xaa is A, S, T, V, I, L, Y, or F

<220>
<221> VARIANT
<222> (23)..(23)
<223> Xaa is any amino acid

<220>
<221>  VARIANT
<222>  (27)..(27)
<223>  Xaa is S, T, V, I, L, Y, or F

<220>
<221>  VARIANT
<222>  (30)..(30)
<223>  Xaa is any amino acid

<220>
<221>  VARIANT
<222>  (31)..(31)
<223>  Xaa is V or I

<220>
<221>  VARIANT
<222>  (34)..(34)
<223>  Xaa is S, T or A

<220>
<221>  VARIANT
<222>  (37)..(39)
<223>  Xaa Xaa Xaa is EGG or DN-

<220>
<221>  VARIANT
<222>  (42)..(42)
<223>  Xaa is T or I

<220>
<221>  VARIANT
<222>  (48)..(48)
<223>  Xaa is S, T, H or R

<220>
<221>  VARIANT
<222>  (57)..(57)
<223>  Xaa is M or L

<220>
<221>  VARIANT
<222>  (58)..(58)
<223>  Xaa is Q, D or E

<220>
<221>  VARIANT
<222>  (59)..(59)
<223>  Xaa is M or K

<220>
<221>  VARIANT
<222>  (61)..(68)
<223>  XaaXaaXaaXaaXaaXaaXaaXaa is -------K, EKS--GKK, EK---QKK,
      ARAEREKK, ARAERE-K, ARAERE--, ARA-EKKK, E-----KK, EKS--GKK,
      ACAEREKK or ACA-EKKK

<400>  46

Met Xaa Xaa Xaa Val Xaa Phe Xaa Xaa Thr Gly Glu Glu Lys Xaa Val
1               5                   10                  15

Xaa Xaa Ser Lys Ile Xaa Xaa Val Tyr Arg Xaa Gly Lys Xaa Xaa Leu
            20                  25                  30

Phe Xaa Tyr Asp Xaa Xaa Xaa Gly Lys Xaa Gly Trp Gly Tyr Val Xaa
        35                  40                  45

Glu Lys Asp Ala Pro Lys Glu Leu Xaa Xaa Xaa Leu Xaa Xaa Xaa Xaa
    50                  55                  60

Xaa Xaa Xaa Xaa
65

<210>  47
<211>  68
<212>  PRT
<213>  Artificial Sequence

<220>
<223>  Consensus sequence

<220>
<221>  VARIANT
<222>  (2)..(2)
<223>  Xaa is V, A or T

<220>
<221>  VARIANT
<222>  (3)..(3)
<223>  Xaa is T or K

<220>
<221>  VARIANT
<222>  (4)..(4)
<223>  Xaa is - or K

<220>
<221>  VARIANT
<222>  (6)..(6)
<223>  Xaa is R or K

<220>
<221>  VARIANT
<222>  (8)..(8)

<220>
<221>  VARIANT
<222>  (15)..(15)
<223>  Xaa is E or Q

<220>
<221>  VARIANT
<222>  (17)..(17)
<223>  Xaa is E or D

<220>
<221>  VARIANT
<222>  (18)..(18)
<223>  Xaa is T or I

<220>
<221>  VARIANT
<222>  (22)..(22)
<223>  Xaa is A, S, T, V, I, L, Y, or F

<220>
<221>  VARIANT
<222>  (23)..(23)
<223>  Xaa is any amino acid

<220>
<221>  VARIANT
<222>  (27)..(27)
<223>  Xaa is S, T, V, I, L, Y, or F

<220>
<221>  VARIANT
<222>  (31)..(31)
<223>  Xaa is V or I

<220>
<221>  VARIANT
<222>  (37)..(39)
<223>  Xaa Xaa Xaa is EGG or DN-

<220>
<221>  VARIANT
<222>  (57)..(57)
<223>  Xaa is M or L

<220>
<221>  VARIANT
<222>  (58)..(58)
<223>  Xaa is Q, D or E

<220>
<221>  VARIANT
<222>  (59)..(59)
<223>  Xaa is M or K

<220>
<221>  VARIANT
<222>  (61)..(68)
<223>  XaaXaaXaaXaaXaaXaaXaaXaa is -------K, EKS--GKK, EK---QKK,
      ARAEREKK, ARAERE-K, ARAERE--, ARA-EKKK, E-----KK, EKS--GKK,
      ACAEREKK or ACA-EKKK

<400>  47

Met Xaa Xaa Xaa Val Xaa Phe Xaa Ala Thr Gly Glu Glu Lys Xaa Val
1               5                   10                  15

Xaa Xaa Ser Lys Ile Xaa Xaa Val Tyr Arg Xaa Gly Lys Asp Xaa Leu
            20                  25                  30

Phe Ser Tyr Asp Xaa Xaa Xaa Gly Lys Ile Gly Trp Gly Tyr Val Ser
        35                  40                  45

Glu Lys Asp Ala Pro Lys Glu Leu Xaa Xaa Xaa Leu Xaa Xaa Xaa Xaa

50                  55                  60

Xaa Xaa Xaa Xaa
65


<210>  48
<211>  68
<212>  PRT
<213>  Artificial Sequence

<220>
<223>  Consensus sequence


<220>
<221>  VARIANT
<222>  (2)..(2)
<223>  Xaa is V, A or T

<220>
<221>  VARIANT
<222>  (3)..(3)
<223>  Xaa is T or K

<220>
<221>  VARIANT
<222>  (4)..(4)
<223>  Xaa is - or K

<220>
<221>  VARIANT
<222>  (6)..(6)
<223>  Xaa is R or K

<220>
<221>  VARIANT
<222>  (15)..(15)
<223>  Xaa is E or Q

<220>
<221>  VARIANT
<222>  (17)..(17)
<223>  Xaa is E or D

<220>
<221>  VARIANT
<222>  (18)..(18)
<223>  Xaa is T or I

<220>
<221>  VARIANT
<222>  (31)..(31)
<223>  Xaa is V or I

<220>
<221>  VARIANT
<222>  (37)..(39)
<223>  Xaa Xaa Xaa is EGG or DN-

<220>
<221>  VARIANT

<222>  (57)..(57)
<223>  Xaa is M or L

<220>
<221>  VARIANT
<222>  (58)..(58)
<223>  Xaa is Q, D or E

<220>
<221>  VARIANT
<222>  (59)..(59)
<223>  Xaa is M or K

<220>
<221>  VARIANT
<222>  (61)..(68)
<223>  XaaXaaXaaXaaXaaXaaXaaXaa is -------K, EKS--GKK, EK---QKK,
       ARAEREKK, ARAERE-K, ARAERE--, ARA-EKKK, E-----KK, EKS--GKK,
       ACAEREKK or ACA-EKKK

<400>  48

Met Xaa Xaa Xaa Val Xaa Phe Asp Ala Thr Gly Glu Glu Lys Xaa Val
1               5                   10                  15

Xaa Xaa Ser Lys Ile Ser Ala Val Tyr Arg Thr Gly Lys Asp Xaa Leu
            20                  25                  30

Phe Ser Tyr Asp Xaa Xaa Xaa Gly Lys Ile Gly Trp Gly Tyr Val Ser
        35                  40                  45

Glu Lys Asp Ala Pro Lys Glu Leu Xaa Xaa Xaa Leu Xaa Xaa Xaa Xaa
    50                  55                  60

Xaa Xaa Xaa Xaa
65

<210>  49
<211>  68
<212>  PRT
<213>  Artificial Sequence

<220>
<223>  Consensus sequence

<220>
<221>  VARIANT
<222>  (2)..(2)
<223>  Xaa is V, A or T

<220>
<221>  VARIANT
<222>  (3)..(3)
<223>  Xaa is T or K

<220>
<221>  VARIANT

<222>  (4)..(4)
<223>  Xaa is - or K

<220>
<221>  VARIANT
<222>  (6)..(6)
<223>  Xaa is R or K

<220>
<221>  VARIANT
<222>  (15)..(15)
<223>  Xaa is E or Q

<220>
<221>  VARIANT
<222>  (17)..(17)
<223>  Xaa is E or D

<220>
<221>  VARIANT
<222>  (18)..(18)
<223>  Xaa is T or I

<220>
<221>  VARIANT
<222>  (31)..(31)
<223>  Xaa is V or I

<220>
<221>  VARIANT
<222>  (37)..(39)
<223>  Xaa Xaa Xaa is EGG or DN-

<220>
<221>  VARIANT
<222>  (57)..(57)
<223>  Xaa is M or L

<220>
<221>  VARIANT
<222>  (58)..(58)
<223>  Xaa is Q, D or E

<220>
<221>  VARIANT
<222>  (59)..(59)
<223>  Xaa is M or K

<220>
<221>  VARIANT
<222>  (61)..(68)
<223>  XaaXaaXaaXaaXaaXaaXaaXaa is -------K, EKS--GKK, EK---QKK,
       ARAEREKK, ARAERE-K, ARAERE--, ARA-EKKK, E-----KK, EKS--GKK,
       ACAEREKK or ACA-EKKK

<400>  49

Met Xaa Xaa Xaa Val Xaa Phe Ala Ala Thr Gly Glu Glu Lys Xaa Val
1               5                   10                  15

Xaa Xaa Ser Lys Ile Ala Asn Val Tyr Arg Val Gly Lys Asp Xaa Leu
            20                  25                  30

Phe Ser Tyr Asp Xaa Xaa Xaa Gly Lys Ile Gly Trp Gly Tyr Val Ser
        35                  40                  45

Glu Lys Asp Ala Pro Lys Glu Leu Xaa Xaa Xaa Leu Xaa Xaa Xaa Xaa
    50                  55                  60

Xaa Xaa Xaa Xaa
65

<210>  50
<211>  64
<212>  PRT
<213>  Artificial Sequence

<220>
<223>  Complement-binding Variant of Sac7d

<220>
<221>  VARIANT
<222>  (7)..(9)
<223>  Xaa is any amino acid

<220>
<221>  VARIANT
<222>  (16)..(16)
<223>  Xaa is E or D

<220>
<221>  VARIANT
<222>  (21)..(21)
<223>  Xaa is A, S, T, V, I, L, Y, or F

<220>
<221>  VARIANT
<222>  (22)..(22)
<223>  Xaa is any amino acid

<220>
<221>  VARIANT
<222>  (26)..(26)
<223>  Xaa is S, T, V, I, L, Y, or F

<220>
<221>  VARIANT
<222>  (29)..(29)
<223>  Xaa is any amino acid

<220>
<221>  VARIANT
<222>  (31)..(31)
<223>  Xaa is L, I, V, Y, F, M or H

<220>
<221>  VARIANT
<222>  (33)..(33)
<223>  Xaa is S, T or A

<220>
<221>  VARIANT
<222>  (37)..(37)
<223>  Xaa is Q or N

<220>
<221>  VARIANT
<222>  (40)..(40)
<223>  Xaa is T or I

<220>
<221>  VARIANT
<222>  (44)..(44)
<223>  Xaa is F, Y, I, V, L, M or H

<220>
<221>  VARIANT
<222>  (46)..(46)
<223>  Xaa is S, T, H or R

<400>  50

Met Val Lys Val Lys Phe Xaa Xaa Xaa Gly Glu Glu Lys Glu Val Xaa
1               5                   10                  15

Thr Ser Lys Ile Xaa Xaa Val Tyr Arg Xaa Gly Lys Xaa Val Xaa Phe
            20                  25                  30

Xaa Tyr Asp Asp Xaa Gly Lys Xaa Gly Trp Gly Xaa Val Xaa Glu Lys
        35                  40                  45

Asp Ala Pro Lys Glu Leu Leu Asp Met Leu Ala Arg Ala Glu Arg Glu
    50                  55                  60

<210>  51
<211>  64
<212>  PRT
<213>  Artificial Sequence

<220>
<223>  Complement-binding Variant of Sac7d

<220>
<221>  VARIANT
<222>  (7)..(8)
<223>  Xaa is any amino acid

<220>
<221>  VARIANT
<222>  (16)..(16)
<223>  Xaa is E or D

<220>
<221>  VARIANT
<222>  (21)..(21)
<223>  Xaa is A, S, T, V, I, L, Y, or F

<220>

<221>  VARIANT
<222>  (22)..(22)
<223>  Xaa is any amino acid

<220>
<221>  VARIANT
<222>  (26)..(26)
<223>  Xaa is S, T, V, I, L, Y, or F

<220>
<221>  VARIANT
<222>  (29)..(29)
<223>  Xaa is any amino acid

<220>
<221>  VARIANT
<222>  (33)..(33)
<223>  Xaa is S, T or A

<220>
<221>  VARIANT
<222>  (37)..(37)
<223>  Xaa is Q or N

<220>
<221>  VARIANT
<222>  (40)..(40)
<223>  Xaa is T or I

<220>
<221>  VARIANT
<222>  (46)..(46)
<223>  Xaa is S, T, H or R

<400>  51

Met Val Lys Val Lys Phe Xaa Xaa Thr Gly Glu Glu Lys Glu Val Xaa
1               5                   10                  15

Thr Ser Lys Ile Xaa Xaa Val Tyr Arg Xaa Gly Lys Xaa Val Leu Phe
            20                  25                  30

Xaa Tyr Asp Asp Xaa Gly Lys Xaa Gly Trp Gly Tyr Val Xaa Glu Lys
        35                  40                  45

Asp Ala Pro Lys Glu Leu Leu Asp Met Leu Ala Arg Ala Glu Arg Glu
    50                  55                  60

<210>  52
<211>  64
<212>  PRT
<213>  Artificial Sequence

<220>
<223>  Complement-binding Variant of Sac7d

<220>
<221>  VARIANT

<222>  (7)..(7)
<223>  Xaa is any amino acid

<220>
<221>  VARIANT
<222>  (16)..(16)
<223>  Xaa is E or D

<220>
<221>  VARIANT
<222>  (21)..(21)
<223>  Xaa is A, S, T, V, I, L, Y, or F

<220>
<221>  VARIANT
<222>  (22)..(22)
<223>  Xaa is any amino acid

<220>
<221>  VARIANT
<222>  (26)..(26)
<223>  Xaa is S, T, V, I, L, Y, or F

<220>
<221>  VARIANT
<222>  (37)..(37)
<223>  Xaa is Q or N

<400>  52

Met Val Lys Val Lys Phe Xaa Ala Thr Gly Glu Glu Lys Glu Val Xaa
1               5                   10                  15

Thr Ser Lys Ile Xaa Xaa Val Tyr Arg Xaa Gly Lys Asp Val Leu Phe
            20                  25                  30

Ser Tyr Asp Asp Xaa Gly Lys Ile Gly Trp Gly Tyr Val Ser Glu Lys
        35                  40                  45

Asp Ala Pro Lys Glu Leu Leu Asp Met Leu Ala Arg Ala Glu Arg Glu
    50                  55                  60

<210>  53
<211>  64
<212>  PRT
<213>  Artificial Sequence

<220>
<223>  Complement-binding variant of Sac7d

<220>
<221>  VARIANT
<222>  (16)..(16)
<223>  Xaa is E or D

<220>
<221>  VARIANT
<222>  (37)..(37)

<223>  Xaa is Q or N

<400>  53

Met Val Lys Val Lys Phe Asp Ala Thr Gly Glu Glu Lys Glu Val Xaa
1               5                   10                  15

Thr Ser Lys Ile Ser Ala Val Tyr Arg Thr Gly Lys Asp Val Leu Phe
            20                  25                  30

Ser Tyr Asp Asp Xaa Gly Lys Ile Gly Trp Gly Tyr Val Ser Glu Lys
        35                  40                  45

Asp Ala Pro Lys Glu Leu Leu Asp Leu Leu Ala Arg Ala Glu Arg Glu
    50                  55                  60

<210>  54
<211>  64
<212>  PRT
<213>  Artificial Sequence

<220>
<223>  Complement-binding variant of Sac7d

<220>
<221>  VARIANT
<222>  (16)..(16)
<223>  Xaa is E or D

<220>
<221>  VARIANT
<222>  (37)..(37)
<223>  Xaa is Q or N

<400>  54

Met Val Lys Val Lys Phe Ala Ala Thr Gly Glu Glu Lys Glu Val Xaa
1               5                   10                  15

Thr Ser Lys Ile Ala Asn Val Tyr Arg Val Gly Lys Asp Val Leu Phe
            20                  25                  30

Ser Tyr Asp Asp Xaa Gly Lys Ile Gly Trp Gly Tyr Val Ser Glu Lys
        35                  40                  45

Asp Ala Pro Lys Glu Leu Leu Asp Leu Leu Ala Arg Ala Glu Arg Glu
    50                  55                  60

<210>  55
<211>  64
<212>  PRT
<213>  Artificial Sequence

<220>

<223>  Complement-binding Variant of Sso7d

<220>
<221>  VARIANT
<222>  (7)..(9)
<223>  Xaa is any amino acid

<220>
<221>  VARIANT
<222>  (16)..(16)
<223>  Xaa is E or D

<220>
<221>  VARIANT
<222>  (21)..(21)
<223>  Xaa is A, S, T, V, I, L, Y, or F

<220>
<221>  VARIANT
<222>  (22)..(22)
<223>  Xaa is any amino acid

<220>
<221>  VARIANT
<222>  (26)..(26)
<223>  Xaa is S, T, V, I, L, Y, or F

<220>
<221>  VARIANT
<222>  (29)..(29)
<223>  Xaa is any amino acid

<220>
<221>  VARIANT
<222>  (31)..(31)
<223>  Xaa is L, I, V, Y, F, M or H

<220>
<221>  VARIANT
<222>  (33)..(33)
<223>  Xaa is S, T or A

<220>
<221>  VARIANT
<222>  (41)..(41)
<223>  Xaa is T or I

<220>
<221>  VARIANT
<222>  (45)..(45)
<223>  Xaa is F, Y, I, V, L, M or H

<220>
<221>  VARIANT
<222>  (47)..(47)
<223>  Xaa is S, T, H or R

<400>  55

Met Ala Thr Val Lys Phe Xaa Xaa Xaa Gly Glu Glu Lys Glu Val Xaa
1               5                   10                  15

Ile Ser Lys Ile Xaa Xaa Val Tyr Arg Xaa Gly Lys Xaa Ile Xaa Phe
            20                  25                  30

Xaa Tyr Asp Glu Gly Gly Gly Lys Xaa Gly Trp Gly Xaa Val Xaa Glu
        35                  40                  45

Lys Asp Ala Pro Lys Glu Leu Leu Gln Leu Leu Glu Lys Gln Lys Lys
    50                  55                  60

<210>  56
<211>  64
<212>  PRT
<213>  Artificial Sequence

<220>
<223>  Complement-binding Variant of Sso7d

<220>
<221>  VARIANT
<222>  (7)..(7)
<223>  Xaa is any amino acid

<220>
<221>  VARIANT
<222>  (16)..(16)
<223>  Xaa is E or D

<220>
<221>  VARIANT
<222>  (21)..(21)
<223>  Xaa is A, S, T, V, I, L, Y, or F

<220>
<221>  VARIANT
<222>  (22)..(22)
<223>  Xaa is any amino acid

<220>
<221>  VARIANT
<222>  (26)..(26)
<223>  Xaa is S, T, V, I, L, Y, or F

<400>  56

Met Ala Thr Val Lys Phe Xaa Ala Thr Gly Glu Glu Lys Glu Val Xaa
1               5                   10                  15

Ile Ser Lys Ile Xaa Xaa Val Tyr Arg Xaa Gly Lys Asp Ile Leu Phe
            20                  25                  30

Ser Tyr Asp Glu Gly Gly Gly Lys Ile Gly Trp Gly Tyr Val Ser Glu
        35                  40                  45

Lys Asp Ala Pro Lys Glu Leu Leu Gln Leu Leu Glu Lys Gln Lys Lys
    50                  55                  60

<210>  57
<211>  64
<212>  PRT
<213>  Artificial Sequence

<220>
<223>  Complement-binding Variant of Sso7d


<220>
<221>  VARIANT
<222>  (16)..(16)
<223>  Xaa is E or D

<400>  57

Met Ala Thr Val Lys Phe Asp Ala Thr Gly Glu Glu Lys Glu Val Xaa
1               5                   10                  15


Ile Ser Lys Ile Ser Ala Val Tyr Arg Thr Gly Lys Asp Ile Leu Phe
            20                  25                  30


Ser Tyr Asp Glu Gly Gly Gly Lys Ile Gly Trp Gly Tyr Val Ser Glu
        35                  40                  45


Lys Asp Ala Pro Lys Glu Leu Leu Gln Leu Leu Glu Lys Gln Lys Lys
    50                  55                  60


<210>  58
<211>  64
<212>  PRT
<213>  Artificial Sequence

<220>
<223>  Complement-binding Variant of Sso7d


<220>
<221>  VARIANT
<222>  (16)..(16)
<223>  Xaa is E or D

<400>  58

Met Ala Thr Val Lys Phe Ala Ala Thr Gly Glu Glu Lys Glu Val Xaa
1               5                   10                  15


Ile Ser Lys Ile Ala Asn Val Tyr Arg Val Gly Lys Asp Ile Leu Phe
            20                  25                  30


Ser Tyr Asp Glu Gly Gly Gly Lys Ile Gly Trp Gly Tyr Val Ser Glu
        35                  40                  45


Lys Asp Ala Pro Lys Glu Leu Leu Gln Leu Leu Glu Lys Gln Lys Lys

50                  55                  60

<210>  59
<211>  64
<212>  PRT
<213>  Artificial Sequence

<220>
<223>  Complement-binding Variant of Sac7d-Aho7c (consensus)


<220>
<221>  VARIANT
<222>  (1)..(1)
<223>  Xaa is V or T

<220>
<221>  VARIANT
<222>  (6)..(8)
<223>  Xaa is any amino acid

<220>
<221>  VARIANT
<222>  (15)..(15)
<223>  Xaa is D or E

<220>
<221>  VARIANT
<222>  (16)..(16)
<223>  Xaa is T or I

<220>
<221>  VARIANT
<222>  (20)..(20)
<223>  Xaa is A, S, T, V, I, L, Y, or F

<220>
<221>  VARIANT
<222>  (21)..(21)
<223>  Xaa is any amino acid

<220>
<221>  VARIANT
<222>  (25)..(25)
<223>  Xaa is S, T, V, I, L, Y, or F

<220>
<221>  VARIANT
<222>  (28)..(28)
<223>  Xaa is any amino acid

<220>
<221>  VARIANT
<222>  (29)..(29)
<223>  Xaa is V or I

<220>
<221>  VARIANT
<222>  (30)..(30)
<223>  Xaa is L, I, V, Y, F, M or H

<220>

<221>  VARIANT
<222>  (32)..(32)
<223>  Xaa is S, T or A

<220>
<221>  VARIANT
<222>  (36)..(36)
<223>  Xaa is N or Q

<220>
<221>  VARIANT
<222>  (39)..(39)
<223>  Xaa is T or I

<220>
<221>  VARIANT
<222>  (43)..(43)
<223>  Xaa is F, Y, I, V, L, M or H

<220>
<221>  VARIANT
<222>  (45)..(45)
<223>  Xaa is S, T, H or R

<220>
<221>  VARIANT
<222>  (55)..(64)
<223>  XaaXaaXaaXaaXaaXaaXaaXaaXaaXaa is DMLARAEREK, DLLARAEREK or
      EKLK------

<400>  59

Xaa Lys Val Lys Phe Xaa Xaa Xaa Gly Glu Glu Lys Glu Val Xaa Xaa
1               5                   10                  15

Ser Lys Ile Xaa Xaa Val Tyr Arg Xaa Gly Lys Xaa Xaa Xaa Phe Xaa
            20                  25                  30

Tyr Asp Asp Xaa Gly Lys Xaa Gly Trp Gly Xaa Val Xaa Glu Lys Asp
        35                  40                  45

Ala Pro Lys Glu Leu Leu Xaa Xaa Xaa Xaa Xaa Xaa Xaa Xaa Xaa Xaa
    50                  55                  60

<210>  60
<211>  64
<212>  PRT
<213>  Artificial Sequence

<220>
<223>  Complement-binding Variant of Sac7d-Aho7c (consensus)

<220>
<221>  VARIANT
<222>  (1)..(1)
<223>  Xaa is V or T

<220>

<221>  VARIANT
<222>  (6)..(7)
<223>  Xaa is any amino acid

<220>
<221>  VARIANT
<222>  (15)..(15)
<223>  Xaa is D or E

<220>
<221>  VARIANT
<222>  (16)..(16)
<223>  Xaa is T or I

<220>
<221>  VARIANT
<222>  (20)..(20)
<223>  Xaa is A, S, T, V, I, L, Y, or F

<220>
<221>  VARIANT
<222>  (21)..(21)
<223>  Xaa is any amino acid

<220>
<221>  VARIANT
<222>  (25)..(25)
<223>  Xaa is S, T, V, I, L, Y, or F

<220>
<221>  VARIANT
<222>  (28)..(28)
<223>  Xaa is any amino acid

<220>
<221>  VARIANT
<222>  (29)..(29)
<223>  Xaa is V or I

<220>
<221>  VARIANT
<222>  (32)..(32)
<223>  Xaa is S, T or A

<220>
<221>  VARIANT
<222>  (36)..(36)
<223>  Xaa is N or Q

<220>
<221>  VARIANT
<222>  (39)..(39)
<223>  Xaa is T or I

<220>
<221>  VARIANT
<222>  (45)..(45)
<223>  Xaa is S, T, H or R

<220>
<221>  VARIANT
<222>  (55)..(64)
<223>  XaaXaaXaaXaaXaaXaaXaaXaaXaaXaa is DMLARAEREK, DLLARAEREK or

EKLK------

<400>  60

Xaa Lys Val Lys Phe Xaa Xaa Thr Gly Glu Glu Lys Glu Val Xaa Xaa
1               5                   10                  15

Ser Lys Ile Xaa Xaa Val Tyr Arg Xaa Gly Lys Xaa Xaa Leu Phe Xaa
            20                  25                  30

Tyr Asp Asp Xaa Gly Lys Xaa Gly Trp Gly Tyr Val Xaa Glu Lys Asp
        35                  40                  45

Ala Pro Lys Glu Leu Leu Xaa Xaa Xaa Xaa Xaa Xaa Xaa Xaa Xaa Xaa
    50                  55                  60

<210>  61
<211>  64
<212>  PRT
<213>  Artificial Sequence

<220>
<223>  Complement-binding Variant of Sac7d-Aho7c (consensus)

<220>
<221>  VARIANT
<222>  (1)..(1)
<223>  Xaa is V or T

<220>
<221>  VARIANT
<222>  (6)..(6)
<223>  Xaa is any amino acid

<220>
<221>  VARIANT
<222>  (15)..(15)
<223>  Xaa is D or E

<220>
<221>  VARIANT
<222>  (16)..(16)
<223>  Xaa is T or I

<220>
<221>  VARIANT
<222>  (20)..(20)
<223>  Xaa is A, S, T, V, I, L, Y, or F

<220>
<221>  VARIANT
<222>  (21)..(21)
<223>  Xaa is any amino acid

<220>
<221>  VARIANT
<222>  (25)..(25)
<223>  Xaa is S, T, V, I, L, Y, or F

<220>
<221>  VARIANT
<222>  (29)..(29)
<223>  Xaa is V or I

<220>
<221>  VARIANT
<222>  (36)..(36)
<223>  Xaa is N or Q

<220>
<221>  VARIANT
<222>  (55)..(64)
<223>  XaaXaaXaaXaaXaaXaaXaaXaaXaaXaa is DMLARAEREK, DLLARAEREK or
       EKLK------

<400>  61

Xaa Lys Val Lys Phe Xaa Ala Thr Gly Glu Glu Lys Glu Val Xaa Xaa
1               5                   10                  15

Ser Lys Ile Xaa Xaa Val Tyr Arg Xaa Gly Lys Asp Xaa Leu Phe Ser
            20                  25                  30

Tyr Asp Asp Xaa Gly Lys Ile Gly Trp Gly Tyr Val Ser Glu Lys Asp
        35                  40                  45

Ala Pro Lys Glu Leu Leu Xaa Xaa Xaa Xaa Xaa Xaa Xaa Xaa Xaa Xaa
    50                  55                  60

<210>  62
<211>  64
<212>  PRT
<213>  Artificial Sequence

<220>
<223>  Complement-binding Variant of Sac7d

<220>
<221>  VARIANT
<222>  (1)..(1)
<223>  Xaa is V or T

<220>
<221>  VARIANT
<222>  (15)..(15)
<223>  Xaa is D or E

<220>
<221>  VARIANT
<222>  (16)..(16)
<223>  Xaa is T or I

<220>
<221>  VARIANT
<222>  (29)..(29)
<223>  Xaa is V or I

<220>
<221>  VARIANT
<222>  (36)..(36)
<223>  Xaa is N or Q

<220>
<221>  VARIANT
<222>  (55)..(64)
<223>  XaaXaaXaaXaaXaaXaaXaaXaaXaaXaa is DMLARAEREK, DLLARAEREK or
       EKLK------

<400>  62

Xaa Lys Val Lys Phe Asp Ala Thr Gly Glu Glu Lys Glu Val Xaa Xaa
1               5                   10                  15

Ser Lys Ile Ser Ala Val Tyr Arg Thr Gly Lys Asp Xaa Leu Phe Ser
            20                  25                  30

Tyr Asp Asp Xaa Gly Lys Ile Gly Trp Gly Tyr Val Ser Glu Lys Asp
        35                  40                  45

Ala Pro Lys Glu Leu Leu Xaa Xaa Xaa Xaa Xaa Xaa Xaa Xaa Xaa Xaa
    50                  55                  60

<210>  63
<211>  64
<212>  PRT
<213>  Artificial Sequence

<220>
<223>  Complement-binding Variant of Sac7d-Aho7c (consensus)

<220>
<221>  VARIANT
<222>  (1)..(1)
<223>  Xaa is V or T

<220>
<221>  VARIANT
<222>  (15)..(15)
<223>  Xaa is D or E

<220>
<221>  VARIANT
<222>  (16)..(16)
<223>  Xaa is T or I

<220>
<221>  VARIANT
<222>  (29)..(29)
<223>  Xaa is V or I

<220>
<221>  VARIANT
<222>  (36)..(36)
<223>  Xaa is N or Q

<220>
<221>  VARIANT
<222>  (55)..(64)
<223>  XaaXaaXaaXaaXaaXaaXaaXaaXaaXaa is DMLARAEREK, DLLARAEREK or
       EKLK------

<400>  63

Xaa Lys Val Lys Phe Ala Ala Thr Gly Glu Glu Lys Glu Val Xaa Xaa
1               5                   10                  15

Ser Lys Ile Ala Asn Val Tyr Arg Val Gly Lys Asp Xaa Leu Phe Ser
            20                  25                  30

Tyr Asp Asp Xaa Gly Lys Ile Gly Trp Gly Tyr Val Ser Glu Lys Asp
        35                  40                  45

Ala Pro Lys Glu Leu Leu Xaa Xaa Xaa Xaa Xaa Xaa Xaa Xaa Xaa Xaa
    50                  55                  60

<210>  64
<211>  64
<212>  PRT
<213>  Artificial Sequence

<220>
<223>  Complement-binding Variant of Sac7d-Aho7c (consensus)

<220>
<221>  VARIANT
<222>  (1)..(1)
<223>  Xaa is V or T

<220>
<221>  VARIANT
<222>  (6)..(8)
<223>  Xaa is any amino acid

<220>
<221>  VARIANT
<222>  (16)..(16)
<223>  Xaa is T or I

<220>
<221>  VARIANT
<222>  (20)..(20)
<223>  Xaa is A, S, T, V, I, L, Y, or F

<220>
<221>  VARIANT
<222>  (21)..(21)
<223>  Xaa is any amino acid

<220>
<221>  VARIANT
<222>  (25)..(25)
<223>  Xaa is S, T, V, I, L, Y, or F

<220>
<221>  VARIANT
<222>  (28)..(28)
<223>  Xaa is any amino acid

<220>
<221>  VARIANT
<222>  (29)..(29)
<223>  Xaa is V or I

<220>
<221>  VARIANT
<222>  (30)..(30)
<223>  Xaa is L, I, V, Y, F, M or H

<220>
<221>  VARIANT
<222>  (32)..(32)
<223>  Xaa is S, T or A

<220>
<221>  VARIANT
<222>  (39)..(39)
<223>  Xaa is T or I

<220>
<221>  VARIANT
<222>  (43)..(43)
<223>  Xaa is F, Y, I, V, L, M or H

<220>
<221>  VARIANT
<222>  (45)..(45)
<223>  Xaa is S, T, H or R

<220>
<221>  VARIANT
<222>  (55)..(64)
<223>  XaaXaaXaaXaaXaaXaaXaaXaaXaaXaa is DMLARAEREK, DLLARAEREK or
      EKLK------

<400>  64

Xaa Lys Val Lys Phe Xaa Xaa Xaa Gly Glu Glu Lys Glu Val Asp Xaa
1               5                   10                  15

Ser Lys Ile Xaa Xaa Val Tyr Arg Xaa Gly Lys Xaa Xaa Xaa Phe Xaa
            20                  25                  30

Tyr Asp Asp Asn Gly Lys Xaa Gly Trp Gly Xaa Val Xaa Glu Lys Asp
        35                  40                  45

Ala Pro Lys Glu Leu Leu Xaa Xaa Xaa Xaa Xaa Xaa Xaa Xaa Xaa Xaa
    50                  55                  60

<210>  65
<211>  64
<212>  PRT

<213>  Artificial Sequence

<220>
<223>  Complement-binding Variant of Sac7d-Aho7c (consensus)


<220>
<221>  VARIANT
<222>  (1)..(1)
<223>  Xaa is V or T

<220>
<221>  VARIANT
<222>  (6)..(7)
<223>  Xaa is any amino acid

<220>
<221>  VARIANT
<222>  (16)..(16)
<223>  Xaa is T or I

<220>
<221>  VARIANT
<222>  (20)..(20)
<223>  Xaa is A, S, T, V, I, L, Y, or F

<220>
<221>  VARIANT
<222>  (21)..(21)
<223>  Xaa is any amino acid

<220>
<221>  VARIANT
<222>  (25)..(25)
<223>  Xaa is S, T, V, I, L, Y, or F

<220>
<221>  VARIANT
<222>  (28)..(28)
<223>  Xaa is any amino acid

<220>
<221>  VARIANT
<222>  (29)..(29)
<223>  Xaa is V or I

<220>
<221>  VARIANT
<222>  (32)..(32)
<223>  Xaa is S, T or A

<220>
<221>  VARIANT
<222>  (39)..(39)
<223>  Xaa is T or I

<220>
<221>  VARIANT
<222>  (45)..(45)
<223>  Xaa is S, T, H or R

<220>
<221>  VARIANT

<222>  (55)..(64)
<223>  XaaXaaXaaXaaXaaXaaXaaXaaXaaXaa is DMLARAEREK, DLLARAEREK or
       EKLK------

<400>  65

Xaa Lys Val Lys Phe Xaa Xaa Thr Gly Glu Glu Lys Glu Val Asp Xaa
1               5                   10                  15

Ser Lys Ile Xaa Xaa Val Tyr Arg Xaa Gly Lys Xaa Xaa Leu Phe Xaa
            20                  25                  30

Tyr Asp Asp Asn Gly Lys Xaa Gly Trp Gly Tyr Val Xaa Glu Lys Asp
        35                  40                  45

Ala Pro Lys Glu Leu Leu Xaa Xaa Xaa Xaa Xaa Xaa Xaa Xaa Xaa Xaa
    50                  55                  60

<210>  66
<211>  64
<212>  PRT
<213>  Artificial Sequence

<220>
<223>  Complement-binding Variant of Sac7d-Aho7c (consensus)

<220>
<221>  VARIANT
<222>  (1)..(1)
<223>  Xaa is V or T

<220>
<221>  VARIANT
<222>  (6)..(6)
<223>  Xaa is any amino acid

<220>
<221>  VARIANT
<222>  (16)..(16)
<223>  Xaa is T or I

<220>
<221>  VARIANT
<222>  (20)..(20)
<223>  Xaa is A, S, T, V, I, L, Y, or F

<220>
<221>  VARIANT
<222>  (21)..(21)
<223>  Xaa is any amino acid

<220>
<221>  VARIANT
<222>  (25)..(25)
<223>  Xaa is S, T, V, I, L, Y, or F

<220>
<221>  VARIANT

<222>  (29)..(29)
<223>  Xaa is V or I

<220>
<221>  VARIANT
<222>  (55)..(64)
<223>  XaaXaaXaaXaaXaaXaaXaaXaaXaaXaa is DMLARAEREK, DLLARAEREK or
       EKLK------

<400>  66

Xaa Lys Val Lys Phe Xaa Ala Thr Gly Glu Glu Lys Glu Val Asp Xaa
1               5                   10                  15

Ser Lys Ile Xaa Xaa Val Tyr Arg Xaa Gly Lys Asp Xaa Leu Phe Ser
            20                  25                  30

Tyr Asp Asp Asn Gly Lys Ile Gly Trp Gly Tyr Val Ser Glu Lys Asp
        35                  40                  45

Ala Pro Lys Glu Leu Leu Xaa Xaa Xaa Xaa Xaa Xaa Xaa Xaa Xaa Xaa
    50                  55                  60

<210>  67
<211>  64
<212>  PRT
<213>  Artificial Sequence

<220>
<223>  Complement-binding Variant of Sac7d-Aho7c (consensus)


<220>
<221>  VARIANT
<222>  (1)..(1)
<223>  Xaa is V or T

<220>
<221>  VARIANT
<222>  (16)..(16)
<223>  Xaa is T or I

<220>
<221>  VARIANT
<222>  (29)..(29)
<223>  Xaa is V or I

<220>
<221>  VARIANT
<222>  (55)..(64)
<223>  XaaXaaXaaXaaXaaXaaXaaXaaXaaXaa is DMLARAEREK, DLLARAEREK or
       EKLK------

<400>  67

Xaa Lys Val Lys Phe Asp Ala Thr Gly Glu Glu Lys Glu Val Asp Xaa
1               5                   10                  15

Ser Lys Ile Ser Ala Val Tyr Arg Thr Gly Lys Asp Xaa Leu Phe Ser
            20                  25                  30

Tyr Asp Asp Asn Gly Lys Ile Gly Trp Gly Tyr Val Ser Glu Lys Asp
        35                  40                  45

Ala Pro Lys Glu Leu Leu Xaa Xaa Xaa Xaa Xaa Xaa Xaa Xaa Xaa Xaa
    50                  55                  60

<210>  68
<211>  64
<212>  PRT
<213>  Artificial Sequence

<220>
<223>  Complement-binding Variant of Sac7d-Aho7c (consensus)

<220>
<221>  VARIANT
<222>  (1)..(1)
<223>  Xaa is V or T

<220>
<221>  VARIANT
<222>  (16)..(16)
<223>  Xaa is T or I

<220>
<221>  VARIANT
<222>  (29)..(29)
<223>  Xaa is V or I

<220>
<221>  VARIANT
<222>  (55)..(64)
<223>  XaaXaaXaaXaaXaaXaaXaaXaaXaaXaa is DMLARAEREK, DLLARAEREK or
       EKLK------

<400>  68

Xaa Lys Val Lys Phe Ala Ala Thr Gly Glu Glu Lys Glu Val Asp Xaa
1               5                   10                  15

Ser Lys Ile Ala Asn Val Tyr Arg Val Gly Lys Asp Xaa Leu Phe Ser
            20                  25                  30

Tyr Asp Asp Asn Gly Lys Ile Gly Trp Gly Tyr Val Ser Glu Lys Asp
        35                  40                  45

Ala Pro Lys Glu Leu Leu Xaa Xaa Xaa Xaa Xaa Xaa Xaa Xaa Xaa Xaa
    50                  55                  60

<210>  69
<211>  64
<212>  PRT

<213>  Artificial Sequence

<220>
<223>  Complement-binding Variant of Sac7d-Aho7c (consensus)


<220>
<221>  VARIANT
<222>  (1)..(1)
<223>  Xaa is V or T

<220>
<221>  VARIANT
<222>  (6)..(8)
<223>  Xaa is any amino acid

<220>
<221>  VARIANT
<222>  (16)..(16)
<223>  Xaa is T or I

<220>
<221>  VARIANT
<222>  (20)..(20)
<223>  Xaa is A, S, T, V, I, L, Y, or F

<220>
<221>  VARIANT
<222>  (21)..(21)
<223>  Xaa is any amino acid

<220>
<221>  VARIANT
<222>  (25)..(25)
<223>  Xaa is S, T, V, I, L, Y, or F

<220>
<221>  VARIANT
<222>  (28)..(28)
<223>  Xaa is any amino acid

<220>
<221>  VARIANT
<222>  (29)..(29)
<223>  Xaa is V or I

<220>
<221>  VARIANT
<222>  (30)..(30)
<223>  Xaa is L, I, V, Y, F, M or H

<220>
<221>  VARIANT
<222>  (32)..(32)
<223>  Xaa is S, T or A

<220>
<221>  VARIANT
<222>  (39)..(39)
<223>  Xaa is T or I

<220>
<221>  VARIANT

<222>  (43)..(43)
<223>  Xaa is F, Y, I, V, L, M or H

<220>
<221>  VARIANT
<222>  (45)..(45)
<223>  Xaa is S, T, H or R

<220>
<221>  VARIANT
<222>  (55)..(64)
<223>  XaaXaaXaaXaaXaaXaaXaaXaaXaaXaa is DMLARAEREK, DLLARAEREK or
       EKLK------

<400>  69

Xaa Lys Val Lys Phe Xaa Xaa Xaa Gly Glu Glu Lys Glu Val Glu Xaa
1               5                   10                  15

Ser Lys Ile Xaa Xaa Val Tyr Arg Xaa Gly Lys Xaa Xaa Xaa Phe Xaa
            20                  25                  30

Tyr Asp Asp Gln Gly Lys Xaa Gly Trp Gly Xaa Val Xaa Glu Lys Asp
        35                  40                  45

Ala Pro Lys Glu Leu Leu Xaa Xaa Xaa Xaa Xaa Xaa Xaa Xaa Xaa Xaa
    50                  55                  60

<210>  70
<211>  64
<212>  PRT
<213>  Artificial Sequence

<220>
<223>  Complement-binding Variant of Sac7d-Aho7c (consensus)

<220>
<221>  VARIANT
<222>  (1)..(1)
<223>  Xaa is V or T

<220>
<221>  VARIANT
<222>  (6)..(7)
<223>  Xaa is any amino acid

<220>
<221>  VARIANT
<222>  (16)..(16)
<223>  Xaa is T or I

<220>
<221>  VARIANT
<222>  (20)..(20)
<223>  Xaa is A, S, T, V, I, L, Y, or F

<220>
<221>  VARIANT

<222>  (21)..(21)
<223>  Xaa is any amino acid

<220>
<221>  VARIANT
<222>  (25)..(25)
<223>  Xaa is S, T, V, I, L, Y, or F

<220>
<221>  VARIANT
<222>  (28)..(28)
<223>  Xaa is any amino acid

<220>
<221>  VARIANT
<222>  (29)..(29)
<223>  Xaa is V or I

<220>
<221>  VARIANT
<222>  (32)..(32)
<223>  Xaa is S, T or A

<220>
<221>  VARIANT
<222>  (39)..(39)
<223>  Xaa is T or I

<220>
<221>  VARIANT
<222>  (45)..(45)
<223>  Xaa is S, T, H or R

<220>
<221>  VARIANT
<222>  (55)..(64)
<223>  XaaXaaXaaXaaXaaXaaXaaXaaXaaXaa is DMLARAEREK, DLLARAEREK or
       EKLK------

<400>  70

Xaa Lys Val Lys Phe Xaa Xaa Thr Gly Glu Glu Lys Glu Val Glu Xaa
1               5                   10                  15


Ser Lys Ile Xaa Xaa Val Tyr Arg Xaa Gly Lys Xaa Xaa Leu Phe Xaa
            20                  25                  30


Tyr Asp Asp Gln Gly Lys Xaa Gly Trp Gly Tyr Val Xaa Glu Lys Asp
        35                  40                  45


Ala Pro Lys Glu Leu Leu Xaa Xaa Xaa Xaa Xaa Xaa Xaa Xaa Xaa Xaa
    50                  55                  60


<210>  71
<211>  64
<212>  PRT
<213>  Artificial Sequence

<220>

<223>  Complement-binding Variant of Sac7d-Aho7c (consensus)

<220>
<221>  VARIANT
<222>  (1)..(1)
<223>  Xaa is V or T

<220>
<221>  VARIANT
<222>  (6)..(6)
<223>  Xaa is any amino acid

<220>
<221>  VARIANT
<222>  (16)..(16)
<223>  Xaa is T or I

<220>
<221>  VARIANT
<222>  (20)..(20)
<223>  Xaa is A, S, T, V, I, L, Y, or F

<220>
<221>  VARIANT
<222>  (21)..(21)
<223>  Xaa is any amino acid

<220>
<221>  VARIANT
<222>  (25)..(25)
<223>  Xaa is S, T, V, I, L, Y, or F

<220>
<221>  VARIANT
<222>  (29)..(29)
<223>  Xaa is V or I

<220>
<221>  VARIANT
<222>  (55)..(64)
<223>  XaaXaaXaaXaaXaaXaaXaaXaaXaaXaa is DMLARAEREK, DLLARAEREK or
      EKLK------

<400>  71

Xaa Lys Val Lys Phe Xaa Ala Thr Gly Glu Glu Lys Glu Val Glu Xaa
1               5                   10                  15

Ser Lys Ile Xaa Xaa Val Tyr Arg Xaa Gly Lys Asp Xaa Leu Phe Ser
            20                  25                  30

Tyr Asp Asp Gln Gly Lys Ile Gly Trp Gly Tyr Val Ser Glu Lys Asp
        35                  40                  45

Ala Pro Lys Glu Leu Leu Xaa Xaa Xaa Xaa Xaa Xaa Xaa Xaa Xaa Xaa
    50                  55                  60

<210>  72

<211>  64
<212>  PRT
<213>  Artificial Sequence

<220>
<223>  Complement-binding Variant of Sac7d-Aho7c (consensus)


<220>
<221>  VARIANT
<222>  (1)..(1)
<223>  Xaa is V or T

<220>
<221>  VARIANT
<222>  (16)..(16)
<223>  Xaa is T or I

<220>
<221>  VARIANT
<222>  (29)..(29)
<223>  Xaa is V or I

<220>
<221>  VARIANT
<222>  (55)..(64)
<223>  XaaXaaXaaXaaXaaXaaXaaXaaXaaXaa is DMLARAEREK, DLLARAEREK or
       EKLK------

<400>  72

Xaa Lys Val Lys Phe Asp Ala Thr Gly Glu Glu Lys Glu Val Glu Xaa
1               5                   10                  15


Ser Lys Ile Ser Ala Val Tyr Arg Thr Gly Lys Asp Xaa Leu Phe Ser
            20                  25                  30


Tyr Asp Asp Gln Gly Lys Ile Gly Trp Gly Tyr Val Ser Glu Lys Asp
        35                  40                  45


Ala Pro Lys Glu Leu Leu Xaa Xaa Xaa Xaa Xaa Xaa Xaa Xaa Xaa Xaa
    50                  55                  60


<210>  73
<211>  64
<212>  PRT
<213>  Artificial Sequence

<220>
<223>  Complement-binding Variant of Sac7d-Aho7c (consensus)


<220>
<221>  VARIANT
<222>  (1)..(1)
<223>  Xaa is V or T

<220>
<221>  VARIANT

<222>  (16)..(16)
<223>  Xaa is T or I

<220>
<221>  VARIANT
<222>  (29)..(29)
<223>  Xaa is V or I

<220>
<221>  VARIANT
<222>  (55)..(64)
<223>  XaaXaaXaaXaaXaaXaaXaaXaaXaaXaa is DMLARAEREK, DLLARAEREK or
       EKLK------

<400>  73

Xaa Lys Val Lys Phe Ala Ala Thr Gly Glu Glu Lys Glu Val Glu Xaa
1               5                   10                  15

Ser Lys Ile Ala Asn Val Tyr Arg Val Gly Lys Asp Xaa Leu Phe Ser
            20                  25                  30

Tyr Asp Asp Gln Gly Lys Ile Gly Trp Gly Tyr Val Ser Glu Lys Asp
        35                  40                  45

Ala Pro Lys Glu Leu Leu Xaa Xaa Xaa Xaa Xaa Xaa Xaa Xaa Xaa Xaa
    50                  55                  60

<210>  74
<211>  64
<212>  PRT
<213>  Artificial Sequence

<220>
<223>  Complement-binding Variant of Sac7d

<220>
<221>  VARIANT
<222>  (6)..(8)
<223>  Xaa is any amino acid

<220>
<221>  VARIANT
<222>  (15)..(15)
<223>  Xaa is D or E

<220>
<221>  VARIANT
<222>  (20)..(20)
<223>  Xaa is A, S, T, V, I, L, Y, or F

<220>
<221>  VARIANT
<222>  (21)..(21)
<223>  Xaa is any amino acid

<220>
<221>  VARIANT

<222>  (25)..(25)
<223>  Xaa is S, T, V, I, L, Y, or F

<220>
<221>  VARIANT
<222>  (28)..(28)
<223>  Xaa is any amino acid

<220>
<221>  VARIANT
<222>  (30)..(30)
<223>  Xaa is L, I, V, Y, F, M or H

<220>
<221>  VARIANT
<222>  (32)..(32)
<223>  Xaa is S, T or A

<220>
<221>  VARIANT
<222>  (36)..(36)
<223>  Xaa is N or Q

<220>
<221>  VARIANT
<222>  (39)..(39)
<223>  Xaa is T or I

<220>
<221>  VARIANT
<222>  (43)..(43)
<223>  Xaa is F, Y, I, V, L, M or H

<220>
<221>  VARIANT
<222>  (45)..(45)
<223>  Xaa is S, T, H or R

<220>
<221>  VARIANT
<222>  (56)..(56)
<223>  Xaa is M or L

<400>  74

Val Lys Val Lys Phe Xaa Xaa Xaa Gly Glu Glu Lys Glu Val Xaa Thr
1               5                   10                  15

Ser Lys Ile Xaa Xaa Val Tyr Arg Xaa Gly Lys Xaa Val Xaa Phe Xaa
            20                  25                  30

Tyr Asp Asp Xaa Gly Lys Xaa Gly Trp Gly Xaa Val Xaa Glu Lys Asp
        35                  40                  45

Ala Pro Lys Glu Leu Leu Asp Xaa Leu Ala Arg Ala Glu Arg Glu Lys
    50                  55                  60

<210>  75
<211>  64

<212>  PRT
<213>  Artificial Sequence

<220>
<223>  Complement-binding Variant of Sac7d


<220>
<221>  VARIANT
<222>  (6)..(7)
<223>  Xaa is any amino acid

<220>
<221>  VARIANT
<222>  (15)..(15)
<223>  Xaa is D or E

<220>
<221>  VARIANT
<222>  (20)..(20)
<223>  Xaa is A, S, T, V, I, L, Y, or F

<220>
<221>  VARIANT
<222>  (21)..(21)
<223>  Xaa is any amino acid

<220>
<221>  VARIANT
<222>  (25)..(25)
<223>  Xaa is S, T, V, I, L, Y, or F

<220>
<221>  VARIANT
<222>  (28)..(28)
<223>  Xaa is any amino acid

<220>
<221>  VARIANT
<222>  (32)..(32)
<223>  Xaa is S, T or A

<220>
<221>  VARIANT
<222>  (36)..(36)
<223>  Xaa is N or Q

<220>
<221>  VARIANT
<222>  (39)..(39)
<223>  Xaa is T or I

<220>
<221>  VARIANT
<222>  (45)..(45)
<223>  Xaa is S, T, H or R

<220>
<221>  VARIANT
<222>  (56)..(56)
<223>  Xaa is M or L

<400>  75

Val Lys Val Lys Phe Xaa Xaa Thr Gly Glu Glu Lys Glu Val Xaa Thr
1               5                   10                  15

Ser Lys Ile Xaa Xaa Val Tyr Arg Xaa Gly Lys Xaa Val Leu Phe Xaa
            20                  25                  30

Tyr Asp Asp Xaa Gly Lys Xaa Gly Trp Gly Tyr Val Xaa Glu Lys Asp
        35                  40                  45

Ala Pro Lys Glu Leu Leu Asp Xaa Leu Ala Arg Ala Glu Arg Glu Lys
    50                  55                  60

<210>  76
<211>  64
<212>  PRT
<213>  Artificial Sequence

<220>
<223>  Complement-binding Variant of Sac7d

<220>
<221>  VARIANT
<222>  (6)..(6)
<223>  Xaa is any amino acid

<220>
<221>  VARIANT
<222>  (15)..(15)
<223>  Xaa is D or E

<220>
<221>  VARIANT
<222>  (20)..(20)
<223>  Xaa is A, S, T, V, I, L, Y, or F

<220>
<221>  VARIANT
<222>  (21)..(21)
<223>  Xaa is any amino acid

<220>
<221>  VARIANT
<222>  (25)..(25)
<223>  Xaa is S, T, V, I, L, Y, or F

<220>
<221>  VARIANT
<222>  (36)..(36)
<223>  Xaa is N or Q

<220>
<221>  VARIANT
<222>  (56)..(56)
<223>  Xaa is M or L

<400>  76

Val Lys Val Lys Phe Xaa Ala Thr Gly Glu Glu Lys Glu Val Xaa Thr
1               5                   10                  15

Ser Lys Ile Xaa Xaa Val Tyr Arg Xaa Gly Lys Asp Val Leu Phe Ser
            20                  25                  30

Tyr Asp Asp Xaa Gly Lys Ile Gly Trp Gly Tyr Val Ser Glu Lys Asp
        35                  40                  45

Ala Pro Lys Glu Leu Leu Asp Xaa Leu Ala Arg Ala Glu Arg Glu Lys
    50                  55                  60

<210>  77
<211>  64
<212>  PRT
<213>  Artificial Sequence

<220>
<223>  Complement-binding Variant of Sac7d

<220>
<221>  VARIANT
<222>  (15)..(15)
<223>  Xaa is D or E

<220>
<221>  VARIANT
<222>  (36)..(36)
<223>  Xaa is N or Q

<220>
<221>  VARIANT
<222>  (56)..(56)
<223>  Xaa is M or L

<400>  77

Val Lys Val Lys Phe Asp Ala Thr Gly Glu Glu Lys Glu Val Xaa Thr
1               5                   10                  15

Ser Lys Ile Ser Ala Val Tyr Arg Thr Gly Lys Asp Val Leu Phe Ser
            20                  25                  30

Tyr Asp Asp Xaa Gly Lys Ile Gly Trp Gly Tyr Val Ser Glu Lys Asp
        35                  40                  45

Ala Pro Lys Glu Leu Leu Asp Xaa Leu Ala Arg Ala Glu Arg Glu Lys
    50                  55                  60

<210>  78
<211>  64
<212>  PRT
<213>  Artificial Sequence

<220>
<223>  Complement-binding Variant of Sac7d

<220>
<221>  VARIANT
<222>  (15)..(15)
<223>  Xaa is D or E

<220>
<221>  VARIANT
<222>  (36)..(36)
<223>  Xaa is N or Q

<220>
<221>  VARIANT
<222>  (56)..(56)
<223>  Xaa is M or L

<400>  78

Val Lys Val Lys Phe Ala Ala Thr Gly Glu Glu Lys Glu Val Xaa Thr
1               5                   10                  15

Ser Lys Ile Ala Asn Val Tyr Arg Val Gly Lys Asp Val Leu Phe Ser
            20                  25                  30

Tyr Asp Asp Xaa Gly Lys Ile Gly Trp Gly Tyr Val Ser Glu Lys Asp
        35                  40                  45

Ala Pro Lys Glu Leu Leu Asp Xaa Leu Ala Arg Ala Glu Arg Glu Lys
    50                  55                  60

<210>  79
<211>  64
<212>  PRT
<213>  Artificial Sequence

<220>
<223>  Complement-binding Variant of Sac7d

<220>
<221>  VARIANT
<222>  (6)..(8)
<223>  Xaa is any amino acid

<220>
<221>  VARIANT
<222>  (20)..(20)
<223>  Xaa is A, S, T, V, I, L, Y, or F

<220>
<221>  VARIANT
<222>  (21)..(21)
<223>  Xaa is any amino acid

<220>
<221>  VARIANT

<222>  (25)..(25)
<223>  Xaa is S, T, V, I, L, Y, or F

<220>
<221>  VARIANT
<222>  (28)..(28)
<223>  Xaa is any amino acid

<220>
<221>  VARIANT
<222>  (30)..(30)
<223>  Xaa is L, I, V, Y, F, M or H

<220>
<221>  VARIANT
<222>  (32)..(32)
<223>  Xaa is S, T or A

<220>
<221>  VARIANT
<222>  (39)..(39)
<223>  Xaa is T or I

<220>
<221>  VARIANT
<222>  (43)..(43)
<223>  Xaa is F, Y, I, V, L, M or H

<220>
<221>  VARIANT
<222>  (45)..(45)
<223>  Xaa is S, T, H or R

<400>  79

Val Lys Val Lys Phe Xaa Xaa Xaa Gly Glu Glu Lys Glu Val Asp Thr
1               5                   10                  15

Ser Lys Ile Xaa Xaa Val Tyr Arg Xaa Gly Lys Xaa Val Xaa Phe Xaa
            20                  25                  30

Tyr Asp Asp Asn Gly Lys Xaa Gly Trp Gly Xaa Val Xaa Glu Lys Asp
        35                  40                  45

Ala Pro Lys Glu Leu Leu Asp Met Leu Ala Arg Ala Glu Arg Glu Lys
    50                  55                  60

<210>  80
<211>  64
<212>  PRT
<213>  Artificial Sequence

<220>
<223>  Complement-binding Variant of Sac7d

<220>
<221>  VARIANT
<222>  (6)..(7)

<223>  Xaa is any amino acid

<220>
<221>  VARIANT
<222>  (20)..(20)
<223>  Xaa is A, S, T, V, I, L, Y, or F

<220>
<221>  VARIANT
<222>  (21)..(21)
<223>  Xaa is any amino acid

<220>
<221>  VARIANT
<222>  (25)..(25)
<223>  Xaa is S, T, V, I, L, Y, or F

<220>
<221>  VARIANT
<222>  (28)..(28)
<223>  Xaa is any amino acid

<220>
<221>  VARIANT
<222>  (32)..(32)
<223>  Xaa is S, T or A

<220>
<221>  VARIANT
<222>  (39)..(39)
<223>  Xaa is T or I

<220>
<221>  VARIANT
<222>  (45)..(45)
<223>  Xaa is S, T, H or R

<400>  80

Val Lys Val Lys Phe Xaa Xaa Thr Gly Glu Glu Lys Glu Val Asp Thr
1               5                   10                  15

Ser Lys Ile Xaa Xaa Val Tyr Arg Xaa Gly Lys Xaa Val Leu Phe Xaa
            20                  25                  30

Tyr Asp Asp Asn Gly Lys Xaa Gly Trp Gly Tyr Val Xaa Glu Lys Asp
        35                  40                  45

Ala Pro Lys Glu Leu Leu Asp Met Leu Ala Arg Ala Glu Arg Glu Lys
    50                  55                  60

<210>  81
<211>  64
<212>  PRT
<213>  Artificial Sequence

<220>
<223>  Complement-binding Variant of Sac7d

<220>
<221>  VARIANT
<222>  (6)..(6)
<223>  Xaa is any amino acid

<220>
<221>  VARIANT
<222>  (20)..(20)
<223>  Xaa is A, S, T, V, I, L, Y, or F

<220>
<221>  VARIANT
<222>  (21)..(21)
<223>  Xaa is any amino acid

<220>
<221>  VARIANT
<222>  (25)..(25)
<223>  Xaa is S, T, V, I, L, Y, or F

<400>  81

Val Lys Val Lys Phe Xaa Ala Thr Gly Glu Glu Lys Glu Val Asp Thr
1               5                   10                  15

Ser Lys Ile Xaa Xaa Val Tyr Arg Xaa Gly Lys Asp Val Leu Phe Ser
            20                  25                  30

Tyr Asp Asp Asn Gly Lys Ile Gly Trp Gly Tyr Val Ser Glu Lys Asp
        35                  40                  45

Ala Pro Lys Glu Leu Leu Asp Met Leu Ala Arg Ala Glu Arg Glu Lys
    50                  55                  60

<210>  82
<211>  64
<212>  PRT
<213>  Artificial Sequence

<220>
<223>  Complement-binding Variant of Sac7d

<400>  82

Val Lys Val Lys Phe Asp Ala Thr Gly Glu Glu Lys Glu Val Asp Thr
1               5                   10                  15

Ser Lys Ile Ser Ala Val Tyr Arg Thr Gly Lys Asp Val Leu Phe Ser
            20                  25                  30

Tyr Asp Asp Asn Gly Lys Ile Gly Trp Gly Tyr Val Ser Glu Lys Asp
        35                  40                  45

Ala Pro Lys Glu Leu Leu Asp Met Leu Ala Arg Ala Glu Arg Glu Lys
    50                  55                  60

<210>  83
<211>  64
<212>  PRT
<213>  Artificial Sequence

<220>
<223>  Complement-binding Variant of Sac7d

<400>  83

Val Lys Val Lys Phe Ala Ala Thr Gly Glu Glu Lys Glu Val Asp Thr
1               5                   10                  15

Ser Lys Ile Ala Asn Val Tyr Arg Val Gly Lys Asp Val Leu Phe Ser
            20                  25                  30

Tyr Asp Asp Asn Gly Lys Ile Gly Trp Gly Tyr Val Ser Glu Lys Asp
        35                  40                  45

Ala Pro Lys Glu Leu Leu Asp Met Leu Ala Arg Ala Glu Arg Glu Lys
    50                  55                  60

<210>  84
<211>  64
<212>  PRT
<213>  Artificial Sequence

<220>
<223>  Complement-binding Variant of Sac7d


<220>
<221>  VARIANT
<222>  (6)..(8)
<223>  Xaa is any amino acid

<220>
<221>  VARIANT
<222>  (20)..(20)
<223>  Xaa is A, S, T, V, I, L, Y, or F

<220>
<221>  VARIANT
<222>  (21)..(21)
<223>  Xaa is any amino acid

<220>
<221>  VARIANT
<222>  (25)..(25)
<223>  Xaa is S, T, V, I, L, Y, or F

<220>
<221>  VARIANT
<222>  (28)..(28)
<223>  Xaa is any amino acid

<220>

<221>  VARIANT
<222>  (30)..(30)
<223>  Xaa is L, I, V, Y, F, M or H

<220>
<221>  VARIANT
<222>  (32)..(32)
<223>  Xaa is S, T or A

<220>
<221>  VARIANT
<222>  (39)..(39)
<223>  Xaa is T or I

<220>
<221>  VARIANT
<222>  (43)..(43)
<223>  Xaa is F, Y, I, V, L, M or H

<220>
<221>  VARIANT
<222>  (45)..(45)
<223>  Xaa is S, T, H or R

<400>  84

Val Lys Val Lys Phe Xaa Xaa Xaa Gly Glu Glu Lys Glu Val Glu Thr
1               5                   10                  15

Ser Lys Ile Xaa Xaa Val Tyr Arg Xaa Gly Lys Xaa Val Xaa Phe Xaa
            20                  25                  30

Tyr Asp Asp Gln Gly Lys Xaa Gly Trp Gly Xaa Val Xaa Glu Lys Asp
        35                  40                  45

Ala Pro Lys Glu Leu Leu Asp Leu Leu Ala Arg Ala Glu Arg Glu Lys
    50                  55                  60

<210>  85
<211>  64
<212>  PRT
<213>  Artificial Sequence

<220>
<223>  Complement-binding Variant of Sac7d

<220>
<221>  VARIANT
<222>  (6)..(7)
<223>  Xaa is any amino acid

<220>
<221>  VARIANT
<222>  (20)..(20)
<223>  Xaa is A, S, T, V, I, L, Y, or F

<220>
<221>  VARIANT

<222>  (21)..(21)
<223>  Xaa is any amino acid

<220>
<221>  VARIANT
<222>  (25)..(25)
<223>  Xaa is S, T, V, I, L, Y, or F

<220>
<221>  VARIANT
<222>  (28)..(28)
<223>  Xaa is any amino acid

<220>
<221>  VARIANT
<222>  (32)..(32)
<223>  Xaa is S, T or A

<220>
<221>  VARIANT
<222>  (39)..(39)
<223>  Xaa is T or I

<220>
<221>  VARIANT
<222>  (45)..(45)
<223>  Xaa is S, T, H or R

<400>  85

Val Lys Val Lys Phe Xaa Xaa Thr Gly Glu Glu Lys Glu Val Glu Thr
1               5                   10                  15

Ser Lys Ile Xaa Xaa Val Tyr Arg Xaa Gly Lys Xaa Val Leu Phe Xaa
            20                  25                  30

Tyr Asp Asp Gln Gly Lys Xaa Gly Trp Gly Tyr Val Xaa Glu Lys Asp
        35                  40                  45

Ala Pro Lys Glu Leu Leu Asp Leu Leu Ala Arg Ala Glu Arg Glu Lys
    50                  55                  60

<210>  86
<211>  64
<212>  PRT
<213>  Artificial Sequence

<220>
<223>  Complement-binding Variant of Sac7d

<220>
<221>  VARIANT
<222>  (6)..(6)
<223>  Xaa is any amino acid

<220>
<221>  VARIANT
<222>  (20)..(20)

<223>  Xaa is A, S, T, V, I, L, Y, or F

<220>
<221>  VARIANT
<222>  (21)..(21)
<223>  Xaa is any amino acid

<220>
<221>  VARIANT
<222>  (25)..(25)
<223>  Xaa is S, T, V, I, L, Y, or F

<400>  86

Val Lys Val Lys Phe Xaa Ala Thr Gly Glu Glu Lys Glu Val Glu Thr
1               5                   10                  15

Ser Lys Ile Xaa Xaa Val Tyr Arg Xaa Gly Lys Asp Val Leu Phe Ser
            20                  25                  30

Tyr Asp Asp Gln Gly Lys Ile Gly Trp Gly Tyr Val Ser Glu Lys Asp
        35                  40                  45

Ala Pro Lys Glu Leu Leu Asp Leu Leu Ala Arg Ala Glu Arg Glu Lys
    50                  55                  60

<210>  87
<211>  64
<212>  PRT
<213>  Artificial Sequence

<220>
<223>  Complement-binding Variant of Sac7d

<400>  87

Val Lys Val Lys Phe Asp Ala Thr Gly Glu Glu Lys Glu Val Glu Thr
1               5                   10                  15

Ser Lys Ile Ser Ala Val Tyr Arg Thr Gly Lys Asp Val Leu Phe Ser
            20                  25                  30

Tyr Asp Asp Gln Gly Lys Ile Gly Trp Gly Tyr Val Ser Glu Lys Asp
        35                  40                  45

Ala Pro Lys Glu Leu Leu Asp Leu Leu Ala Arg Ala Glu Arg Glu Lys
    50                  55                  60

<210>  88
<211>  64
<212>  PRT
<213>  Artificial Sequence

<220>
<223>  Complement-binding Variant of Sac7d

<400>  88

Val Lys Val Lys Phe Ala Ala Thr Gly Glu Glu Lys Glu Val Glu Thr
1               5                   10                  15

Ser Lys Ile Ala Asn Val Tyr Arg Val Gly Lys Asp Val Leu Phe Ser
            20                  25                  30

Tyr Asp Asp Gln Gly Lys Ile Gly Trp Gly Tyr Val Ser Glu Lys Asp
        35                  40                  45

Ala Pro Lys Glu Leu Leu Asp Leu Leu Ala Arg Ala Glu Arg Glu Lys
    50                  55                  60

<210>  89
<211>  58
<212>  PRT
<213>  Artificial Sequence

<220>
<223>  Complement-binding Variant of Aho7c

<220>
<221>  VARIANT
<222>  (6)..(8)
<223>  Xaa is any amino acid

<220>
<221>  VARIANT
<222>  (15)..(15)
<223>  Xaa is D or E

<220>
<221>  VARIANT
<222>  (20)..(20)
<223>  Xaa is A, S, T, V, I, L, Y, or F

<220>
<221>  VARIANT
<222>  (21)..(21)
<223>  Xaa is any amino acid

<220>
<221>  VARIANT
<222>  (25)..(25)
<223>  Xaa is S, T, V, I, L, Y, or F

<220>
<221>  VARIANT
<222>  (28)..(28)
<223>  Xaa is any amino acid

<220>
<221>  VARIANT
<222>  (30)..(30)
<223>  Xaa is L, I, V, Y, F, M or H

<220>
<221>  VARIANT
<222>  (32)..(32)
<223>  Xaa is S, T or A

<220>
<221>  VARIANT
<222>  (36)..(36)
<223>  Xaa is N or Q

<220>
<221>  VARIANT
<222>  (39)..(39)
<223>  Xaa is T or I

<220>
<221>  VARIANT
<222>  (43)..(43)
<223>  Xaa is F, Y, I, V, L, M or H

<220>
<221>  VARIANT
<222>  (45)..(45)
<223>  Xaa is S, T, H or R

<400>  89

Thr Lys Val Lys Phe Xaa Xaa Xaa Gly Glu Glu Lys Glu Val Xaa Ile
1               5                   10                  15

Ser Lys Ile Xaa Xaa Val Tyr Arg Xaa Gly Lys Xaa Ile Xaa Phe Xaa
            20                  25                  30

Tyr Asp Asp Xaa Gly Lys Xaa Gly Trp Gly Xaa Val Xaa Glu Lys Asp
        35                  40                  45

Ala Pro Lys Glu Leu Leu Glu Lys Leu Lys
    50                  55

<210>  90
<211>  58
<212>  PRT
<213>  Artificial Sequence

<220>
<223>  Complement-binding Variant of Aho7c

<220>
<221>  VARIANT
<222>  (6)..(7)
<223>  Xaa is any amino acid

<220>
<221>  VARIANT
<222>  (15)..(15)
<223>  Xaa is D or E

<220>

<221>  VARIANT
<222>  (20)..(20)
<223>  Xaa is A, S, T, V, I, L, Y, or F

<220>
<221>  VARIANT
<222>  (21)..(21)
<223>  Xaa is any amino acid

<220>
<221>  VARIANT
<222>  (25)..(25)
<223>  Xaa is S, T, V, I, L, Y, or F

<220>
<221>  VARIANT
<222>  (28)..(28)
<223>  Xaa is any amino acid

<220>
<221>  VARIANT
<222>  (32)..(32)
<223>  Xaa is S, T or A

<220>
<221>  VARIANT
<222>  (36)..(36)
<223>  Xaa is N or Q

<220>
<221>  VARIANT
<222>  (39)..(39)
<223>  Xaa is T or I

<220>
<221>  VARIANT
<222>  (45)..(45)
<223>  Xaa is S, T, H or R

<400>  90

Thr Lys Val Lys Phe Xaa Xaa Thr Gly Glu Glu Lys Glu Val Xaa Ile
1               5                   10                  15

Ser Lys Ile Xaa Xaa Val Tyr Arg Xaa Gly Lys Xaa Ile Leu Phe Xaa
            20                  25                  30

Tyr Asp Asp Xaa Gly Lys Xaa Gly Trp Gly Tyr Val Xaa Glu Lys Asp
        35                  40                  45

Ala Pro Lys Glu Leu Leu Glu Lys Leu Lys
    50                  55

<210>  91
<211>  58
<212>  PRT
<213>  Artificial Sequence

<220>

<223>  Complement-binding Variant of Aho7c

<220>
<221>  VARIANT
<222>  (6)..(6)
<223>  Xaa is any amino acid

<220>
<221>  VARIANT
<222>  (15)..(15)
<223>  Xaa is D or E

<220>
<221>  VARIANT
<222>  (20)..(20)
<223>  Xaa is A, S, T, V, I, L, Y, or F

<220>
<221>  VARIANT
<222>  (21)..(21)
<223>  Xaa is any amino acid

<220>
<221>  VARIANT
<222>  (25)..(25)
<223>  Xaa is S, T, V, I, L, Y, or F

<220>
<221>  VARIANT
<222>  (36)..(36)
<223>  Xaa is N or Q

<400>  91

Thr Lys Val Lys Phe Xaa Ala Thr Gly Glu Glu Lys Glu Val Xaa Ile
1               5                   10                  15

Ser Lys Ile Xaa Xaa Val Tyr Arg Xaa Gly Lys Asp Ile Leu Phe Ser
            20                  25                  30

Tyr Asp Asp Xaa Gly Lys Ile Gly Trp Gly Tyr Val Ser Glu Lys Asp
        35                  40                  45

Ala Pro Lys Glu Leu Leu Glu Lys Leu Lys
    50                  55

<210>  92
<211>  58
<212>  PRT
<213>  Artificial Sequence

<220>
<223>  Complement-binding Variant of Aho7c

<220>
<221>  VARIANT
<222>  (15)..(15)

<223>  Xaa is D or E

<220>
<221>  VARIANT
<222>  (36)..(36)
<223>  Xaa is N or Q

<400>  92

Thr Lys Val Lys Phe Asp Ala Thr Gly Glu Glu Lys Glu Val Xaa Ile
1               5                   10                  15

Ser Lys Ile Ser Ala Val Tyr Arg Thr Gly Lys Asp Ile Leu Phe Ser
            20                  25                  30

Tyr Asp Asp Xaa Gly Lys Ile Gly Trp Gly Tyr Val Ser Glu Lys Asp
        35                  40                  45

Ala Pro Lys Glu Leu Leu Glu Lys Leu Lys
    50                  55

<210>  93
<211>  58
<212>  PRT
<213>  Artificial Sequence

<220>
<223>  Complement-binding Variant of Aho7c

<220>
<221>  VARIANT
<222>  (15)..(15)
<223>  Xaa is D or E

<220>
<221>  VARIANT
<222>  (36)..(36)
<223>  Xaa is N or Q

<400>  93

Thr Lys Val Lys Phe Ala Ala Thr Gly Glu Glu Lys Glu Val Xaa Ile
1               5                   10                  15

Ser Lys Ile Ala Asn Val Tyr Arg Val Gly Lys Asp Ile Leu Phe Ser
            20                  25                  30

Tyr Asp Asp Xaa Gly Lys Ile Gly Trp Gly Tyr Val Ser Glu Lys Asp
        35                  40                  45

Ala Pro Lys Glu Leu Leu Glu Lys Leu Lys
    50                  55

<210>  94

<211>  58
<212>  PRT
<213>  Artificial Sequence

<220>
<223>  Complement-binding Variant of Aho7c



<220>
<221>  VARIANT
<222>  (6)..(8)
<223>  Xaa is any amino acid

<220>
<221>  VARIANT
<222>  (20)..(20)
<223>  Xaa is A, S, T, V, I, L, Y, or F

<220>
<221>  VARIANT
<222>  (21)..(21)
<223>  Xaa is any amino acid

<220>
<221>  VARIANT
<222>  (25)..(25)
<223>  Xaa is S, T, V, I, L, Y, or F

<220>
<221>  VARIANT
<222>  (28)..(28)
<223>  Xaa is any amino acid

<220>
<221>  VARIANT
<222>  (30)..(30)
<223>  Xaa is L, I, V, Y, F, M or H

<220>
<221>  VARIANT
<222>  (32)..(32)
<223>  Xaa is S, T or A

<220>
<221>  VARIANT
<222>  (39)..(39)
<223>  Xaa is T or I

<220>
<221>  VARIANT
<222>  (43)..(43)
<223>  Xaa is F, Y, I, V, L, M or H

<220>
<221>  VARIANT
<222>  (45)..(45)
<223>  Xaa is S, T, H or R

<400>  94

Thr Lys Val Lys Phe Xaa Xaa Xaa Gly Glu Glu Lys Glu Val Asp Ile
1               5                   10                  15

Ser Lys Ile Xaa Xaa Val Tyr Arg Xaa Gly Lys Xaa Ile Xaa Phe Xaa
            20                  25                  30

Tyr Asp Asp Asn Gly Lys Xaa Gly Trp Gly Xaa Val Xaa Glu Lys Asp
        35                  40                  45

Ala Pro Lys Glu Leu Leu Glu Lys Leu Lys
    50                  55

<210>  95
<211>  58
<212>  PRT
<213>  Artificial Sequence

<220>
<223>  Complement-binding Variant of Aho7c

<220>
<221>  VARIANT
<222>  (6)..(7)
<223>  Xaa is any amino acid

<220>
<221>  VARIANT
<222>  (20)..(20)
<223>  Xaa is A, S, T, V, I, L, Y, or F

<220>
<221>  VARIANT
<222>  (21)..(21)
<223>  Xaa is any amino acid

<220>
<221>  VARIANT
<222>  (25)..(25)
<223>  Xaa is S, T, V, I, L, Y, or F

<220>
<221>  VARIANT
<222>  (28)..(28)
<223>  Xaa is any amino acid

<220>
<221>  VARIANT
<222>  (32)..(32)
<223>  Xaa is S, T or A

<220>
<221>  VARIANT
<222>  (39)..(39)
<223>  Xaa is T or I

<220>
<221>  VARIANT
<222>  (45)..(45)
<223>  Xaa is S, T, H or R

<400>  95

Thr Lys Val Lys Phe Xaa Xaa Thr Gly Glu Glu Lys Glu Val Asp Ile
1               5                   10                  15

Ser Lys Ile Xaa Xaa Val Tyr Arg Xaa Gly Lys Xaa Ile Leu Phe Xaa
            20                  25                  30

Tyr Asp Asp Asn Gly Lys Xaa Gly Trp Gly Tyr Val Xaa Glu Lys Asp
        35                  40                  45

Ala Pro Lys Glu Leu Leu Glu Lys Leu Lys
    50                  55

<210>  96
<211>  58
<212>  PRT
<213>  Artificial Sequence

<220>
<223>  Complement-binding Variant of Aho7c

<220>
<221>  VARIANT
<222>  (6)..(6)
<223>  Xaa is any amino acid

<220>
<221>  VARIANT
<222>  (20)..(20)
<223>  Xaa is A, S, T, V, I, L, Y, or F

<220>
<221>  VARIANT
<222>  (21)..(21)
<223>  Xaa is any amino acid

<220>
<221>  VARIANT
<222>  (25)..(25)
<223>  Xaa is S, T, V, I, L, Y, or F

<400>  96

Thr Lys Val Lys Phe Xaa Ala Thr Gly Glu Glu Lys Glu Val Asp Ile
1               5                   10                  15

Ser Lys Ile Xaa Xaa Val Tyr Arg Xaa Gly Lys Asp Ile Leu Phe Ser
            20                  25                  30

Tyr Asp Asp Asn Gly Lys Ile Gly Trp Gly Tyr Val Ser Glu Lys Asp
        35                  40                  45

Ala Pro Lys Glu Leu Leu Glu Lys Leu Lys
    50                  55

<210>  97
<211>  58
<212>  PRT
<213>  Artificial Sequence

<220>
<223>  Complement-binding Variant of Aho7c

<400>  97

Thr Lys Val Lys Phe Asp Ala Thr Gly Glu Glu Lys Glu Val Asp Ile
1               5                   10                  15

Ser Lys Ile Ser Ala Val Tyr Arg Thr Gly Lys Asp Ile Leu Phe Ser
            20                  25                  30

Tyr Asp Asp Asn Gly Lys Ile Gly Trp Gly Tyr Val Ser Glu Lys Asp
        35                  40                  45

Ala Pro Lys Glu Leu Leu Glu Lys Leu Lys
    50                  55

<210>  98
<211>  58
<212>  PRT
<213>  Artificial Sequence

<220>
<223>  Complement-binding Variant of Aho7c

<400>  98

Thr Lys Val Lys Phe Ala Ala Thr Gly Glu Glu Lys Glu Val Asp Ile
1               5                   10                  15

Ser Lys Ile Ala Asn Val Tyr Arg Val Gly Lys Asp Ile Leu Phe Ser
            20                  25                  30

Tyr Asp Asp Asn Gly Lys Ile Gly Trp Gly Tyr Val Ser Glu Lys Asp
        35                  40                  45

Ala Pro Lys Glu Leu Leu Glu Lys Leu Lys
    50                  55

<210>  99
<211>  58
<212>  PRT
<213>  Artificial Sequence

<220>
<223>  Complement-binding Variant of Aho7c

<220>

<221>  VARIANT
<222>  (6)..(8)
<223>  Xaa is any amino acid

<220>
<221>  VARIANT
<222>  (20)..(20)
<223>  Xaa is A, S, T, V, I, L, Y, or F

<220>
<221>  VARIANT
<222>  (21)..(21)
<223>  Xaa is any amino acid

<220>
<221>  VARIANT
<222>  (25)..(25)
<223>  Xaa is S, T, V, I, L, Y, or F

<220>
<221>  VARIANT
<222>  (28)..(28)
<223>  Xaa is any amino acid

<220>
<221>  VARIANT
<222>  (30)..(30)
<223>  Xaa is L, I, V, Y, F, M or H

<220>
<221>  VARIANT
<222>  (32)..(32)
<223>  Xaa is S, T or A

<220>
<221>  VARIANT
<222>  (39)..(39)
<223>  Xaa is T or I

<220>
<221>  VARIANT
<222>  (43)..(43)
<223>  Xaa is F, Y, I, V, L, M or H

<220>
<221>  VARIANT
<222>  (45)..(45)
<223>  Xaa is S, T, H or R

<400>  99

Thr Lys Val Lys Phe Xaa Xaa Xaa Gly Glu Glu Lys Glu Val Glu Ile
1               5                   10                  15

Ser Lys Ile Xaa Xaa Val Tyr Arg Xaa Gly Lys Xaa Ile Xaa Phe Xaa
            20                  25                  30

Tyr Asp Asp Gln Gly Lys Xaa Gly Trp Gly Xaa Val Xaa Glu Lys Asp
        35                  40                  45

Ala Pro Lys Glu Leu Leu Glu Lys Leu Lys
    50                  55

<210>  100
<211>  58
<212>  PRT
<213>  Artificial Sequence

<220>
<223>  Complement-binding Variant of Aho7c

<220>
<221>  VARIANT
<222>  (6)..(7)
<223>  Xaa is any amino acid

<220>
<221>  VARIANT
<222>  (20)..(20)
<223>  Xaa is A, S, T, V, I, L, Y, or F

<220>
<221>  VARIANT
<222>  (21)..(21)
<223>  Xaa is any amino acid

<220>
<221>  VARIANT
<222>  (25)..(25)
<223>  Xaa is S, T, V, I, L, Y, or F

<220>
<221>  VARIANT
<222>  (28)..(28)
<223>  Xaa is any amino acid

<220>
<221>  VARIANT
<222>  (32)..(32)
<223>  Xaa is S, T or A

<220>
<221>  VARIANT
<222>  (39)..(39)
<223>  Xaa is T or I

<220>
<221>  VARIANT
<222>  (45)..(45)
<223>  Xaa is S, T, H or R

<400>  100

Thr Lys Val Lys Phe Xaa Xaa Thr Gly Glu Glu Lys Glu Val Glu Ile
1               5                   10                  15

Ser Lys Ile Xaa Xaa Val Tyr Arg Xaa Gly Lys Xaa Ile Leu Phe Xaa
            20                  25                  30

Tyr Asp Asp Gln Gly Lys Xaa Gly Trp Gly Tyr Val Xaa Glu Lys Asp
        35                  40                  45

Ala Pro Lys Glu Leu Leu Glu Lys Leu Lys
    50                  55

<210>  101
<211>  58
<212>  PRT
<213>  Artificial Sequence

<220>
<223>  Complement-binding Variant of Aho7c

<220>
<221>  VARIANT
<222>  (6)..(6)
<223>  Xaa is any amino acid

<220>
<221>  VARIANT
<222>  (20)..(20)
<223>  Xaa is A, S, T, V, I, L, Y, or F

<220>
<221>  VARIANT
<222>  (21)..(21)
<223>  Xaa is any amino acid

<220>
<221>  VARIANT
<222>  (25)..(25)
<223>  Xaa is S, T, V, I, L, Y, or F

<400>  101

Thr Lys Val Lys Phe Xaa Ala Thr Gly Glu Glu Lys Glu Val Glu Ile
1               5                   10                  15

Ser Lys Ile Xaa Xaa Val Tyr Arg Xaa Gly Lys Asp Ile Leu Phe Ser
            20                  25                  30

Tyr Asp Asp Gln Gly Lys Ile Gly Trp Gly Tyr Val Ser Glu Lys Asp
        35                  40                  45

Ala Pro Lys Glu Leu Leu Glu Lys Leu Lys
    50                  55

<210>  102
<211>  58
<212>  PRT
<213>  Artificial Sequence

<220>
<223>  Complement-binding Variant of Aho7c

<400>  102

Thr Lys Val Lys Phe Asp Ala Thr Gly Glu Glu Lys Glu Val Glu Ile
1               5                   10                  15

Ser Lys Ile Ser Ala Val Tyr Arg Thr Gly Lys Asp Ile Leu Phe Ser
            20                  25                  30

Tyr Asp Asp Gln Gly Lys Ile Gly Trp Gly Tyr Val Ser Glu Lys Asp
        35                  40                  45

Ala Pro Lys Glu Leu Leu Glu Lys Leu Lys
    50                  55

<210>  103
<211>  58
<212>  PRT
<213>  Artificial Sequence

<220>
<223>  Complement-binding Variant of Aho7c

<400>  103

Thr Lys Val Lys Phe Ala Ala Thr Gly Glu Glu Lys Glu Val Glu Ile
1               5                   10                  15

Ser Lys Ile Ala Asn Val Tyr Arg Val Gly Lys Asp Ile Leu Phe Ser
            20                  25                  30

Tyr Asp Asp Gln Gly Lys Ile Gly Trp Gly Tyr Val Ser Glu Lys Asp
        35                  40                  45

Ala Pro Lys Glu Leu Leu Glu Lys Leu Lys
    50                  55

<210>  104
<211>  64
<212>  PRT
<213>  Artificial

<220>
<223>  Cosensus sequence Sac7d-Aho7c

<220>
<221>  VARIANT
<222>  (1)..(1)
<223>  Xaa is V or T

<220>
<221>  VARIANT
<222>  (16)..(16)
<223>  Xaa is T or I

<220>
<221>  VARIANT
<222>  (29)..(29)
<223>  Xaa is V or I

<220>
<221>  VARIANT
<222>  (55)..(64)
<223>  XaaXaaXaaXaaXaaXaaXaaXaaXaaXaa is DMLARAEREK, DLLARAEREK or
       EKLK------

<400>  104

Xaa Lys Val Lys Phe Lys Tyr Lys Gly Glu Glu Lys Glu Val Asp Xaa
1               5                   10                  15

Ser Lys Ile Lys Lys Val Trp Arg Val Gly Lys Met Xaa Ser Phe Thr
            20                  25                  30

Tyr Asp Asp Asn Gly Lys Thr Gly Arg Gly Ala Val Ser Glu Lys Asp
        35                  40                  45

Ala Pro Lys Glu Leu Leu Xaa Xaa Xaa Xaa Xaa Xaa Xaa Xaa Xaa Xaa
    50                  55                  60